(19) Europäisches Patentamt
European Patent Office
Office européen des brevets

(11) EP 3 760 628 A1

(12) EUROPEAN PATENT APPLICATION
published in accordance with Art. 153(4) EPC

(43) Date of publication:
**06.01.2021 Bulletin 2021/01**

(21) Application number: **19757537.6**

(22) Date of filing: **26.02.2019**

(51) Int Cl.:
***C07D 471/04*** (2006.01) ***C07D 491/048*** (2006.01)
***C07D 495/04*** (2006.01) ***C07D 487/04*** (2006.01)
***A61K 31/4184*** (2006.01) ***A61K 31/4545*** (2006.01)
***A61K 31/4985*** (2006.01) ***A61K 31/454*** (2006.01)
***A61K 31/4709*** (2006.01) ***A61K 31/438*** (2006.01)
***A61K 31/55*** (2006.01) ***A61P 35/00*** (2006.01)
***A61P 29/00*** (2006.01) ***A61P 27/00*** (2006.01)
***A61P 37/00*** (2006.01) ***A61P 17/06*** (2006.01)
***A61P 19/02*** (2006.01) ***A61P 25/28*** (2006.01)
***A61P 11/00*** (2006.01)

(86) International application number:
**PCT/CN2019/076129**

(87) International publication number:
**WO 2019/161803 (29.08.2019 Gazette 2019/35)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **26.02.2018 CN 201810158900**
**11.12.2018 CN 201811512419**

(71) Applicant: **Nanjing Transthera Biosciences Co., Ltd.**
**Nanjing, Jiangsu 210032 (CN)**

(72) Inventors:
- **WU, Frank**
**Nanjing, Jiangsu 210032 (CN)**
- **LI, Lin**
**Nanjing, Jiangsu 210032 (CN)**

(74) Representative: **Grünecker Patent- und Rechtsanwälte PartG mbB**
**Leopoldstraße 4**
**80802 München (DE)**

# (54) PEPTIDYLARGININE DEIMINASE INHIBITOR AND USE THEREOF

(57) The present invention relates to the technical field of pharmaceuticals, and particularly to a peptidylarginine deiminase (PAD4) inhibitor compound of formula (I) or a pharmaceutically acceptable salt, stereoisomer and tautomer thereof, and pharmaceutical composition, pharmaceutical formulation and use thereof. X, Y, $R_1$, $R_2$, $R_3$, $R_4$, $R_5$, $R_7$, $R_8$, $R_9$, ring B and m are defined in the specification. The compound disclosed herein has an inhibitory effect on peptidylarginine deiminase (PAD4), and can be used to treat a variety of diseases, such as rheumatoid arthritis, vasculitis, systemic lupus erythematosus, ulcerative colitis, multiple sclerosis, cystic fibrosis, cancer, cutaneous lupus erythematosus, asthma and psoriasis.

EP 3 760 628 A1

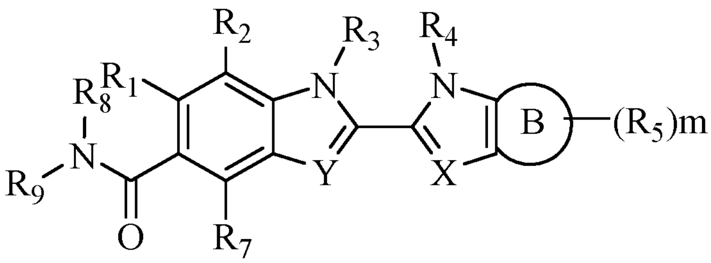

(I)

## Description

### TECHNICAL FIELD

The present invention relates to the technical field of pharmaceuticals, and particularly to a peptidylarginine deiminase (PAD4) inhibitor compound of general formula (I), a pharmaceutically acceptable salt, stereoisomer and tautomer thereof, and pharmaceutical composition, pharmaceutical formulation and use thereof. The compound disclosed herein has an inhibitory effect on PAD4, and can be used to treat a variety of diseases, such as rheumatoid arthritis, vasculitis, systemic lupus erythematosus, ulcerative colitis, multiple sclerosis, cystic fibrosis, cancer, cutaneous lupus erythematosus, asthma and psoriasis.

### BACKGROUND

PAD4, which is a member of the peptidylarginine deiminase (PAD) family, has a homodimer structure, and is a $Ca^{2+}$-dependent enzyme, with each monomer containing five binding sites for calcium ions. PAD4 consists of 663 amino acid residues with a molecular weight of 74 kDa. In the presence of $Ca^{2+}$, PAD4 can catalyze arginine residues in the protein polypeptide to produce citrulline. The original molecular conformation of the citrullinated protein is often changed, leading to a change in the biochemical activity of the protein, and the citrullinated protein participates in a variety of physiological and pathological responses, such as rheumatoid arthritis, systemic lupus erythematosus, ulcerative colitis, vasculitis and cancer.

Rheumatoid arthritis (RA) is a chronic, inflammatory and systematic autoimmune disease, the main clinical manifestations of which are joint swelling and pain caused by synovial membranes of facet joints followed by functional disorders such as cartilage destruction, joint space narrowing, joint stiffness and deformity. The worldwide incidence of the disease is 0.4% to 1%, among which there are about five million patients with rheumatoid arthritis in China. This disease occurs more frequently among women, with a female-to-male incidence ratio of 3:1, and the age of onset mainly ranges from 25 to 55 years old. It has been discovered in a study that the content of PAD4 in the serum of patients with RA significantly increases, the body could produce autoantibodies against PAD4, and PAD4 citrullinates a variety of proteins to cause the autoimmune response of the body and participate in the occurrence and development of RA. For example, after a vimentin is citrullinated, the citrullinated peptide fragments were identified by HLA-DR molecules, causing immune response of T cells.

In addition, the expression of PAD4 in the brain of patients with multiple sclerosis (MS) increases, so the PAD4 inhibitor can also be used to treat multiple sclerosis. Moreover, histone citrullination is associated with the formation of neutrophil extracellular traps (NETs) (an innate immune response mechanism), while the PAD4 inhibitor can reduce the activity of neutrophils under a variety of pathologies. Therefore, the PAD4 inhibitor can be used for tissue damage diseases caused by the formation of NETs. These diseases include, but are not limited to, systemic lupus erythematosus, ulcerative colitis, vasculitis, cystic fibrosis, and asthma. In addition, the formation of NETs is associated with the pathologies of skin diseases, such as psoriasis and cutaneous lupus erythematosus. Therefore, the PAD4 inhibitor can also be used to treat skin diseases caused by the formation of NETs. Besides, the PAD4 inhibitor can also be used to treat cancer, and it has been proved at present that PAD4 is overexpressed in many cancers.

At present, inhibitors targeting PAD are all at the preclinical study stage, aiming at catalytic active sites thereof, and are mainly divided into two major categories, *i.e.*, irreversible inhibitors and reversible inhibitors: the first category mainly includes PAD enzyme substrate analogs (*i.e.*, mimetic peptides), which are non-selective inhibitors, including haloamidines, O-haloamidines and tripeptides; and the second category mainly includes PAD4 selective reversible inhibitors. WO2014/015905A1 discloses a compound (GSK199) with the following structure. For GSK199, which is currently at the preclinical study stage, the main indication is rheumatoid arthritis (RA), but the *in-vitro* enzymological activity has not yet reached an ideal level.

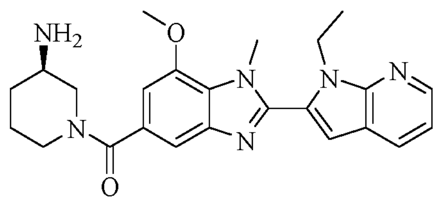

(GSK199)

At present, there are few PAD4 inhibitor varieties under development. In order to better satisfy the tremendous clinical demand, we aim to develop a PAD4 inhibitor with higher activity and better druggability.

## SUMMARY OF THE INVENTION

The objective of the present invention is to provide a peptidylarginine deiminase (PAD4) inhibitor compound and a pharmaceutically acceptable salt, stereoisomer and tautomer thereof. With good kinase-inhibiting activity on PAD4, the compound disclosed herein can be used to treat or prevent diseases mediated by PAD4.

The technical solution adopted by the present invention is as follows:

**A compound of general formula (I) or a pharmaceutically acceptable salt, stereoisomer and tautomer thereof:**

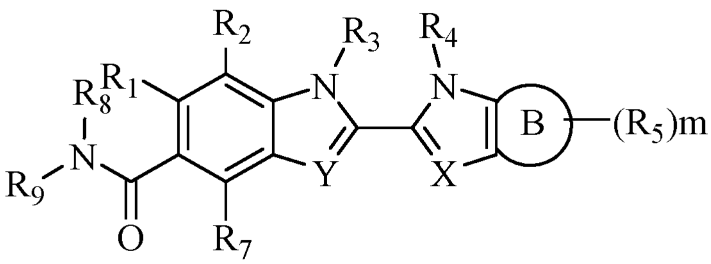

(I)

wherein X and Y are each independently selected from $CR_6$ or N;

$R_1$ is hydrogen or $C_{1-6}$ alkyl;
$R_2$ is hydrogen, $C_{1-6}$ alkyl, 3-6 membered cycloalkyl, $C_{1-6}$ alkoxy, $C_{1-6}$ haloalkyl or $C_{1-6}$ haloalkoxy;
$R_3$ is hydrogen, cyano, $C_{1-6}$ alkyl, $C_{1-6}$ alkoxy, $C_{1-6}$ alkoxy $C_{1-6}$ alkyl, $-L_1-Cy_1$, or $C_{1-6}$ alkyl, $C_{1-6}$ alkylcarbonyl, $C_{1-6}$ alkoxycarbonyl, $C_{1-6}$ alkylsulfonyl, $C_{1-6}$ alkoxy $C_{1-6}$ alkyl, $C_{1-6}$ alkylamino or $(C_{1-6}$ alkyl$)_2$ amino unsubstituted or substituted by halogen, cyano, amino or hydroxyl, $L_1$ is absent or is $C_{1-6}$ alkylene, $Cy_1$ is 3-12 membered cycloalkyl, 3-12 membered cycloalkenyl, 3-12 membered heterocyclyl, aryl or 5-10 membered heteroaryl, and $Cy_1$ may be optionally substituted by hydrogen, halogen, cyano, amino, hydroxyl, $C_{1-6}$ alkyl or $C_{1-6}$ alkoxy;
$R_4$ is hydrogen, cyano, 3-6 membered cycloalkyl, or $C_{1-6}$ alkyl, $C_{1-6}$ alkylcarbonyl, $C_{1-6}$ alkoxycarbonyl, $C_{1-6}$ alkylsulfonyl, $C_{1-6}$ alkoxy $C_{1-6}$ alkyl, $C_{1-6}$ alkylamino or $(C_{1-6}$ alkyl$)_2$ amino unsubstituted or substituted by halogen, cyano, amino, hydroxyl or 3-6 membered cycloalkyl;
$R_5$ is hydrogen, halogen, cyano, amino, hydroxyl, $-L_2-Cy_2$, or $C_{1-6}$ alkyl, $C_{2-8}$ alkenyl, $C_{1-6}$ alkylcarbonyl, $C_{1-6}$ alkylcarbonylamino, $C_{1-6}$ alkoxy, $C_{1-6}$ alkoxycarbonyl, $C_{1-6}$ alkylsulfonyl, $C_{1-6}$ alkylthio, $C_{1-6}$ alkylsulfonylamino, $C_{1-6}$ alkylamino or $(C_{1-6}$ alkyl$)_2$ amino unsubstituted or substituted by halogen, cyano, amino or hydroxyl, $L_2$ is absent or is $C_{1-6}$ alkylene, $C_{1-6}$ alkyleneoxy, $C_{2-6}$ alkenylene or $C_{1-6}$ alkyleneamino, $Cy_2$ is 3-12 membered cycloalkyl, 3-12 membered cycloalkenyl, 3-12 membered heterocyclyl, aryl or 5-10 membered heteroaryl, and $Cy_2$ may be optionally substituted by hydrogen, halogen, cyano, amino, hydroxyl, $C_{1-6}$ alkyl or $C_{1-6}$ alkoxy;
$R_6$ is hydrogen, $C_{1-6}$ alkyl or $C_{3-6}$ cycloalkyl;
$R_7$ is hydrogen or $C_{1-6}$ alkyl;
$R_8$ is hydrogen, halogen, cyano, amino, hydroxyl, $-L_3-Cy_3$, or $C_{1-6}$ alkyl, $C_{2-8}$ alkenyl, $C_{1-6}$ alkylcarbonyl, $C_{1-6}$ alkylcarbonylamino, $C_{1-6}$ alkoxy or $C_{1-6}$ alkoxycarbonyl unsubstituted or substituted by halogen, cyano, amino or hydroxyl, $L_3$ is absent or is $C_{1-6}$ alkylene or $C_{2-6}$ alkenylene, $C_{y3}$ is 3-12 membered cycloalkyl, 3-12 membered cycloalkenyl, 3-12 membered heterocyclyl, aryl or 5-10 membered heteroaryl, and $C_{y3}$ may be optionally substituted by hydrogen, halogen, cyano, amino, hydroxyl, $C_{1-6}$ alkyl or $C_{1-6}$ alkoxy;
$R_9$ is hydrogen or $C_{1-6}$ alkyl;
alternatively, $R_8$ and $R_9$, along with N connected thereto, form 4-7 membered heterocyclyl, 6-11 membered ortho-heterocyclyl, 7-12 membered spiro-heterocyclyl or 6-10 membered bridged heterocyclyl unsubstituted or substituted by a substituent selected from hydrogen, halogen, cyano, amino, hydroxyl, $C_{1-6}$ alkyl, $C_{1-6}$ alkoxy, $C_{1-6}$ alkylamino, $(C_{1-6}$ alkyl$)_2$ amino and $-L_4-Cy_4$, $L_4$ is absent or is $C_{1-6}$ alkylene, and $Cy_4$ is 3-12 membered cycloalkyl, 3-12 membered cycloalkenyl, 3-12 membered heterocyclyl, aryl or 5-10 membered heteroaryl;
ring B is

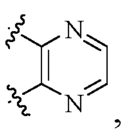 , 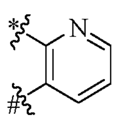 , 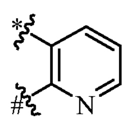 , 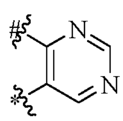

or 5 membered heteroaryl;
wherein ring B is fused to

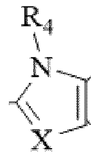

with the * terminus upward and the # terminus downward, and if no * terminus or # terminus is specified, ring B can be fused in any direction;
with the *proviso* that
when ring B is

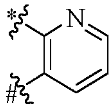

and $R_8$ and $R_9$ along with N connected thereto form

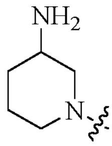

,

X is N;
when ring B is

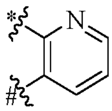

and X is $CR_6$, $R_8$ and $R_9$ along with N connected thereto form

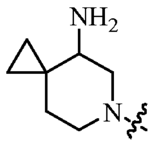

;

and
m is an integer of 0 to 4.

In one embodiment, X is $CR_6$, and Y is N.
In one embodiment, Y is $CR_6$, and X is N.
In one embodiment, X and Y are each independently selected from $CR_6$.
In one embodiment, X and Y are each independently selected from N.
In one embodiment, $R_1$ is hydrogen.
In one embodiment, $R_2$ is hydrogen, $C_{1-6}$ alkyl, $C_{1-6}$ alkoxy, $C_{1-6}$ haloalkyl or $C_{1-6}$ haloalkoxy. In another embodiment, $R_2$ is hydrogen, $C_{1-6}$ alkyl or $C_{1-6}$ alkoxy. In another embodiment, $R_2$ is $C_{1-6}$ alkoxy. In another embodiment, $R_2$ is methoxy, ethoxy, *n*-propoxy, isopropoxy, *n*-butoxy or *tert*-butoxy. In another embodiment, $R_2$ is methoxy.
In one embodiment, $R_3$ is hydrogen, cyano, $C_{1-6}$ alkyl, $C_{1-6}$ alkoxy, $C_{1-6}$ alkoxy $C_{1-6}$ alkyl, or $C_{1-6}$ alkyl unsubstituted or substituted by halogen, cyano, amino or hydroxyl. In another embodiment, $R_3$ is hydrogen or $C_{1-6}$ alkyl. In another embodiment, $R_3$ is $C_{1-6}$ alkyl. In another embodiment, $R_3$ is methyl, ethyl, *n*-propyl, isopropyl, *n*-butyl, isobutyl,

*sec*-butyl or *tert*-butyl. In another embodiment, $R_3$ is methyl.

In one embodiment, $R_4$ is hydrogen, cyano, 3-6 membered cycloalkyl, or $C_{1-6}$ alkyl unsubstituted or substituted by halogen, cyano, amino, hydroxyl or 3-6 membered cycloalkyl. In another embodiment, $R_4$ is hydrogen, or $C_{1-6}$ alkyl unsubstituted or substituted by halogen or 3-6 membered cycloalkyl. In another embodiment, $R_4$ is hydrogen, or $C_{1-6}$ alkyl unsubstituted or substituted by halogen, cyclopropyl or cyclobutyl. In another embodiment, $R_4$ is $C_{1-6}$ alkyl unsubstituted or substituted by halogen, cyclopropyl or cyclobutyl. In another embodiment, $R_4$ is methyl, ethyl, *n*-propyl, isopropyl, *n*-butyl, isobutyl, *sec*-butyl or *tert-butyl* unsubstituted or substituted by halogen, cyclopropyl or cyclobutyl. In another embodiment, $R_4$ is methyl or ethyl unsubstituted or substituted by halogen, cyclopropyl or cyclobutyl. In another embodiment, $R_4$ is ethyl, isobutyl, trifluoroethyl, cyclopropylmethyl or cyclobutylmethyl. In another embodiment, $R_4$ is cyclobutylmethyl or cyclopropylmethyl.

In one embodiment, $R_5$ is hydrogen, halogen, cyano, amino, hydroxyl, or $C_{1-6}$ alkyl or $C_{1-6}$ alkoxy unsubstituted or substituted by halogen, cyano, amino or hydroxyl. In another embodiment, $R_5$ is hydrogen, halogen, cyano, amino, hydroxyl, or $C_{1-6}$ alkyl unsubstituted or substituted by halogen, cyano, amino or hydroxyl. In another embodiment, $R_5$ is hydrogen, halogen, or $C_{1-6}$ alkyl or $C_{1-6}$ alkoxy unsubstituted or substituted by halogen. In another embodiment, $R_5$ is hydrogen, halogen, $C_{1-6}$ alkyl or $C_{1-6}$ alkoxy. In another embodiment, $R_5$ is hydrogen, fluorine, chlorine, methyl, ethyl, *n*-propyl, isopropyl, *n*-butyl, isobutyl, *sec*-butyl, *tert*-butyl, methoxy, ethoxy, *n*-propoxy, isopropoxy, *n*-butoxy or tert-butoxy. In another embodiment, $R_5$ is hydrogen, fluorine, methyl or methoxy. In another embodiment, $R_5$ is hydrogen, fluorine, methyl or methoxy. In another embodiment, $R_5$ is hydrogen.

In one embodiment, $R_6$ is hydrogen or $C_{1-6}$ alkyl. In another embodiment, $R_6$ is hydrogen.

In one embodiment, $R_7$ is hydrogen.

In one embodiment, $R_8$ is hydrogen, halogen, cyano, amino, hydroxyl, $-L_3-Cy_3$, or $C_{1-6}$ alkyl unsubstituted or substituted by halogen, cyano, amino or hydroxyl, $L_3$ is absent or is $C_{1-6}$ alkylene, $Cy_3$ is 3-6 membered cycloalkyl, 3-12 membered heterocyclyl, aryl or 5-10 membered heteroaryl, and $Cy_3$ may be optionally substituted by hydrogen, halogen, cyano, amino, hydroxyl, $C_{1-6}$ alkyl or $C_{1-6}$ alkoxy. In another embodiment, $R_8$ is hydrogen, or $C_{1-6}$ alkyl unsubstituted or substituted by amino. In another embodiment, $R_8$ is $-L_3-Cy_3$, $L_3$ is absent, $Cy_3$ is 3-6 membered cycloalkyl, and $Cy_3$ is substituted by amino. In another embodiment, $R_8$ is $-L_3-Cy_3$, $L_3$ is absent, $Cy_3$ is cyclobutyl, and $Cy_3$ is substituted by amino.

In one embodiment, $R_9$ is $C_{1-6}$ alkyl. In another embodiment, $R_9$ is methyl, ethyl, *n*-propyl, isopropyl, *n*-butyl, isobutyl, *sec*-butyl or *tert*-butyl. In another embodiment, $R_9$ is methyl.

In one embodiment, $R_8$ and $R_9$, along with N connected thereto, form 5-6 membered heterocyclyl, 7-10 membered ortho-heterocyclyl, 7-11 membered spiro-heterocyclyl or 7-10 membered bridged heterocyclyl unsubstituted or substituted by a substituent selected from hydrogen, halogen, cyano, amino, hydroxyl, $C_{1-6}$ alkyl and $C_{1-6}$ alkoxy. In one embodiment, $R_8$ and $R_9$ along with N connected thereto form 5-6 membered heterocyclyl unsubstituted or substituted by a substituent selected from amino. In one embodiment, $R_8$ and $R_9$ along with N connected thereto form 5-6 membered saturated nitrogen containing heterocyclyl unsubstituted or substituted by a substituent selected from amino. In another embodiment, $R_8$ and $R_9$ along with N connected thereto form azacyclobutyl, azacycloheptyl, pyrrolidinyl, piperidinyl, piperazinyl or morpholinyl unsubstituted or substituted by a substituent selected from hydrogen, halogen, cyano, amino, hydroxyl, $C_{1-6}$ alkyl and $C_{1-6}$ alkoxy. In another embodiment, $R_8$ and $R_9$ along with N connected thereto form pyrrolidinyl, piperidinyl, piperazinyl or morpholinyl unsubstituted or substituted by a substituent selected from hydrogen, halogen, cyano, amino, hydroxyl, $C_{1-6}$ alkyl and $C_{1-6}$ alkoxy. In another embodiment, $R_8$ and $R_9$ along with N connected thereto form pyrrolidinyl, piperidinyl or azacycloheptyl substituted by amino. In another embodiment, $R_8$ and $R_9$ along with N connected thereto form piperidinyl substituted by amino. In another embodiment, $R_8$ and $R_9$ along with N connected thereto form

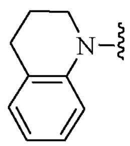, 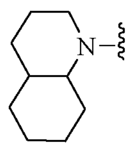, 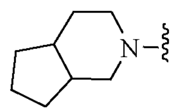,

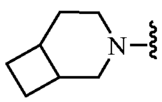, 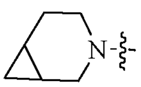, 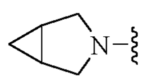, 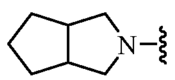, 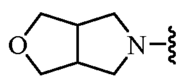, 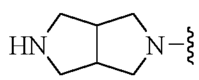,

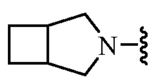 or 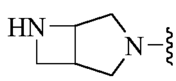

unsubstituted or substituted by a substituent selected from hydrogen, halogen, cyano, amino, hydroxyl, $C_{1-6}$ alkyl and $C_{1-6}$ alkoxy. In another embodiment, $R_8$ and $R_9$ along with N connected thereto form

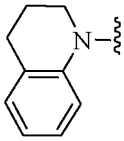

substituted by amino. In another embodiment, $R_8$ and $R_9$ along with N connected

thereto form 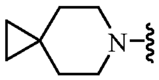, 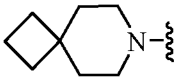, 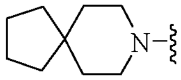, 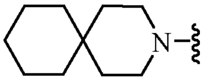, 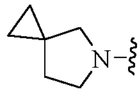,

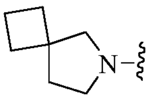, 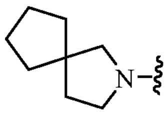, 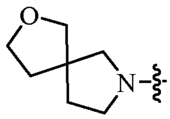, 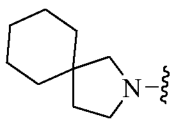, 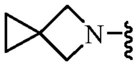, 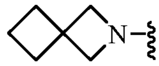,

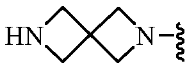, 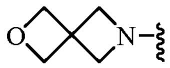, 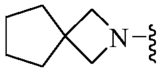, 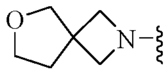, 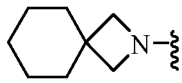

or

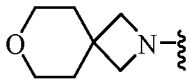

unsubstituted or substituted by a substituent selected from hydrogen, halogen, cyano, amino, hydroxyl, $C_{1-6}$ alkyl and $C_{1-6}$ alkoxy. In another embodiment, $R_8$ and $R_9$ along with N connected thereto form

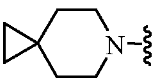, 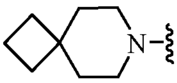, 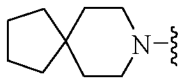, 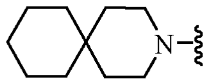, 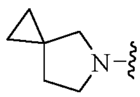, 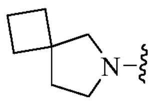,

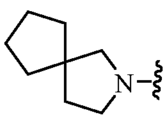, 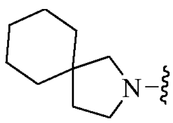, 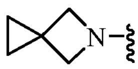, 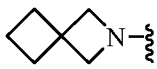, 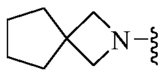 or 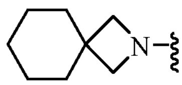

substituted by amino. In another embodiment, $R_8$ and $R_9$ along with N connected thereto form

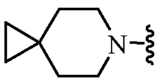

substituted by amino. In another embodiment, $R_8$ and $R_9$ along with N connected thereto form

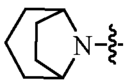, 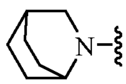, 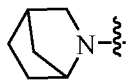

or

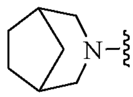

unsubstituted or substituted by a substituent selected from hydrogen, halogen, cyano, amino, hydroxyl, $C_{1-6}$ alkyl and $C_{1-6}$ alkoxy. In another embodiment, $R_8$ and $R_9$ along with N connected thereto form

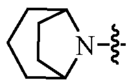

substituted by amino.

In one embodiment, ring B is

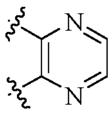, 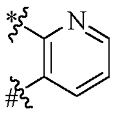, 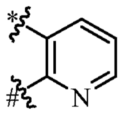, 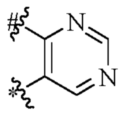

or 5 membered heteroaryl. In another embodiment, ring B is 5 membered heteroaryl. In another embodiment, ring B is

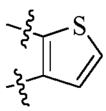, 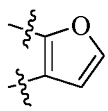 or 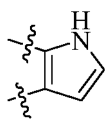.

In another embodiment, ring B is

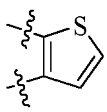 or 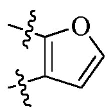.

In another embodiment, ring B is

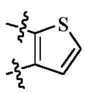.

When ring B is 5 membered heteroaryl, ring B is preferably fused to

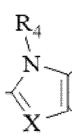

with the heteroatom upward.

In one embodiment, m is 0, 1, 2, 3 or 4. In another embodiment, m is 0, 1, 2 or 3. In another embodiment, m is 0, 1 or 2. In another embodiment, m is 0 or 1. In another embodiment, m is 0.

**In another preferred embodiment, the compound of general formula (I) or the pharmaceutically acceptable salt, stereoisomer and tautomer thereof,**

wherein X and Y are each independently selected from $CR_6$ or N;

$R_1$ is hydrogen or $C_{1-6}$ alkyl;

$R_2$ is hydrogen, $C_{1-6}$ alkyl, 3-6 membered cycloalkyl, $C_{1-6}$ alkoxy, $C_{1-6}$ haloalkyl or $C_{1-6}$ haloalkoxy;

$R_3$ is hydrogen, cyano, $C_{1-6}$ alkyl, $C_{1-6}$ alkoxy, $C_{1-6}$ alkoxy $C_{1-6}$ alkyl, $-L_1-C_{y1}$, or $C_{1-6}$ alkyl, $C_{1-6}$ alkylcarbonyl, $C_{1-6}$ alkoxycarbonyl, $C_{1-6}$ alkylsulfonyl, $C_{1-6}$ alkoxy $C_{1-6}$ alkyl, $C_{1-6}$ alkylamino or $(C_{1-6}$ alkyl$)_2$ amino unsubstituted or substituted by halogen, cyano, amino or hydroxyl, $L_1$ is absent or is $C_{1-6}$ alkylene, $Cy_1$ is 3-12 membered cycloalkyl, 3-12 membered cycloalkenyl, 3-12 membered heterocyclyl, aryl or 5-10 membered heteroaryl, and $Cy_1$ may be optionally substituted by hydrogen, halogen, cyano, amino, hydroxyl, $C_{1-6}$ alkyl or $C_{1-6}$ alkoxy;

$R_4$ is hydrogen, cyano, 3-6 membered cycloalkyl, or $C_{1-6}$ alkyl, $C_{1-6}$ alkylcarbonyl, $C_{1-6}$ alkoxycarbonyl, $C_{1-6}$ alkylsulfonyl, $C_{1-6}$ alkoxy $C_{1-6}$ alkyl, $C_{1-6}$ alkylamino or $(C_{1-6}$ alkyl$)_2$ amino unsubstituted or substituted by halogen, cyano, amino, hydroxyl or 3-6 membered cycloalkyl;

$R_5$ is hydrogen, halogen, cyano, amino, hydroxyl, $-L_2-Cy_2$, or $C_{1-6}$ alkyl, $C_{2-8}$ alkenyl, $C_{1-6}$ alkylcarbonyl, $C_{1-6}$ alkylcarbonylamino, $C_{1-6}$ alkoxy, $C_{1-6}$ alkoxycarbonyl, $C_{1-6}$ alkylsulfonyl, $C_{1-6}$ alkylthio, $C_{1-6}$ alkylsulfonylamino, $C_{1-6}$ alkylamino or $(C_{1-6}$ alkyl$)_2$ amino unsubstituted or substituted by halogen, cyano, amino or hydroxyl, $L_2$ is absent or is $C_{1-6}$ alkylene, $C_{1-6}$ alkyleneoxy, $C_{2-6}$ alkenylene or $C_{1-6}$ alkyleneamino, $Cy_2$ is 3-12 membered cycloalkyl, 3-12 membered cycloalkenyl, 3-12 membered heterocyclyl, aryl or 5-10 membered heteroaryl, and $Cy_2$ may be optionally substituted by hydrogen, halogen, cyano, amino, hydroxyl, $C_{1-6}$ alkyl or $C_{1-6}$ alkoxy;

$R_6$ is hydrogen, $C_{1-6}$ alkyl or $C_{3-6}$ cycloalkyl;

$R_7$ is hydrogen or $C_{1-6}$ alkyl;

$R_8$ is hydrogen, halogen, cyano, amino, hydroxyl, $-L_3-Cy_3$, or $C_{1-6}$ alkyl, $C_{2-8}$ alkenyl, $C_{1-6}$ alkylcarbonyl, $C_{1-6}$ alkylcarbonylamino, $C_{1-6}$ alkoxy or $C_{1-6}$ alkoxycarbonyl unsubstituted or substituted by halogen, cyano, amino or hydroxyl, $L_3$ is absent or is $C_{1-6}$ alkylene or $C_{2-6}$ alkenylene, $Cy_3$ is 3-12 membered cycloalkyl, 3-12 membered cycloalkenyl, 3-12 membered heterocyclyl, aryl or 5-10 membered heteroaryl, and $Cy_3$ may be optionally substituted by hydrogen, halogen, cyano, amino, hydroxyl, $C_{1-6}$ alkyl or $C_{1-6}$ alkoxy;

$R_9$ is hydrogen or $C_{1-6}$ alkyl;

alternatively, $R_8$ and $R_9$, along with N connected thereto, form 4-7 membered heterocyclyl, 6-11 membered ortho-heterocyclyl, 7-12 membered spiro-heterocyclyl or 6-10 membered bridged heterocyclyl unsubstituted or substituted by a substituent selected from hydrogen, halogen, cyano, amino, hydroxyl, $C_{1-6}$ alkyl, $C_{1-6}$ alkoxy, $C_{1-6}$ alkylamino, $(C_{1-6}$ alkyl$)_2$ amino and $-L_4-Cy_4$, $L_4$ is absent or is $C_{1-6}$ alkylene, and $Cy_4$ is 3-12 membered cycloalkyl, 3-12 membered cycloalkenyl, 3-12 membered heterocyclyl, aryl or 5-10 membered heteroaryl;

ring B is 5 membered heteroaryl; preferably, ring B is

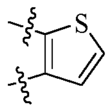,
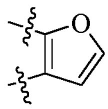

or

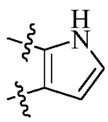;

more preferably, ring B is

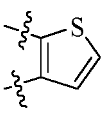 or 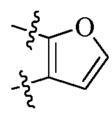;

and further preferably, ring B is

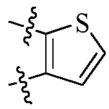
.

In one embodiment, X is $CR_6$, and Y is N.

In one embodiment, Y is $CR_6$, and X is N.

In one embodiment, X and Y are each independently selected from $CR_6$.

In one embodiment, X and Y are each independently selected from N.

**In another preferred embodiment, the compound of general formula (I) or the pharmaceutically acceptable salt, stereoisomer and tautomer thereof,**

wherein X and Y are each independently selected from $CR_6$ or N;

$R_1$ is hydrogen or $C_{1-6}$ alkyl;

$R_2$ is hydrogen, $C_{1-6}$ alkyl, $C_{1-6}$ alkoxy, $C_{1-6}$ haloalkyl or $C_{1-6}$ haloalkoxy;

$R_3$ is hydrogen, cyano, $C_{1-6}$ alkyl, $C_{1-6}$ alkoxy, $C_{1-6}$ alkoxy $C_{1-6}$ alkyl, or $C_{1-6}$ alkyl unsubstituted or substituted by halogen, cyano, amino or hydroxyl;

$R_4$ is hydrogen, cyano, 3-6 membered cycloalkyl, or $C_{1-6}$ alkyl unsubstituted or substituted by halogen, cyano, amino, hydroxyl or 3-6 membered cycloalkyl;

$R_5$ is hydrogen, halogen, cyano, amino, hydroxyl, or $C_{1-6}$ alkyl or $C_{1-6}$ alkoxy unsubstituted or substituted by halogen, cyano, amino or hydroxyl;

$R_6$ is hydrogen or $C_{1-6}$ alkyl;

$R_7$ is hydrogen or $C_{1-6}$ alkyl;

$R_8$ is hydrogen, halogen, cyano, amino, hydroxyl, $-L_3-Cy_3$, or $C_{1-6}$ alkyl unsubstituted or substituted by halogen, cyano, amino or hydroxyl, $L_3$ is absent or is $C_{1-6}$ alkylene, $Cy_3$ is 3-6 membered cycloalkyl, 3-12 membered heterocyclyl, aryl or 5-10 membered heteroaryl, and $Cy_3$ may be optionally substituted by hydrogen, halogen, cyano, amino, hydroxyl, $C_{1-6}$ alkyl or $C_{1-6}$ alkoxy;

$R_9$ is hydrogen or $C_{1-6}$ alkyl;

alternatively, $R_8$ and $R_9$ along with N connected thereto form 5-6 membered heterocyclyl, 7-10 membered ortho-heterocyclyl, 7-11 membered spiro-heterocyclyl or 7-10 membered bridged heterocyclyl unsubstituted or substituted by a substituent selected from hydrogen, halogen, cyano, amino, hydroxyl, $C_{1-6}$ alkyl and $C_{1-6}$ alkoxy;

ring B is 5 membered heteroaryl; preferably, ring B is

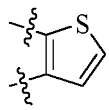
,
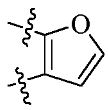

or

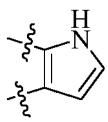
;

more preferably, ring B is

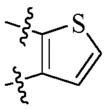
or
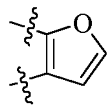
;

and further preferably, ring B is

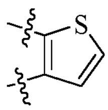
;

m is an integer of 0 to 4.

In one embodiment, X is $CR_6$, and Y is N.

In one embodiment, Y is $CR_6$, and X is N.

In one embodiment, X and Y are each independently selected from $CR_6$.

In one embodiment, X and Y are each independently selected from N.

**In another preferred embodiment, the compound of general formula (I) or the pharmaceutically acceptable salt, stereoisomer and tautomer thereof,**

wherein X and Y are each independently selected from $CR_6$ or N;
$R_1$ is hydrogen or $C_{1-6}$ alkyl;
$R_2$ is hydrogen, $C_{1-6}$ alkyl or $C_{1-6}$ alkoxy;
$R_3$ is hydrogen or $C_{1-6}$ alkyl;
$R_4$ is hydrogen, or $C_{1-6}$ alkyl unsubstituted or substituted by halogen or 3-6 membered cycloalkyl;
$R_5$ is hydrogen, halogen, $C_{1-6}$ alkyl or $C_{1-6}$ alkoxy;
$R_6$ is hydrogen or $C_{1-6}$ alkyl;
$R_7$ is hydrogen or $C_{1-6}$ alkyl;
$R_8$ is hydrogen, $-L_3-Cy_3$, or $C_{1-6}$ alkyl unsubstituted or substituted by amino, $L_3$ is absent, and $Cy_3$ is 3-6 membered cycloalkyl optionally substituted by amino;
$R_9$ is hydrogen or $C_{1-6}$ alkyl;
alternatively, $R_8$ and $R_9$ along with N connected thereto form 4-7 membered heterocyclyl or 7-12 membered spiro-heterocyclyl unsubstituted or substituted by a substituent selected from amino;
ring B is

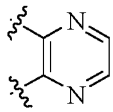 , 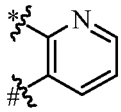 , 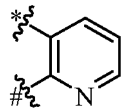

or 5 membered heteroaryl; wherein ring B is fused to

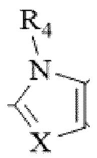

with the * terminus upward and the # terminus downward, and if no * terminus or # terminus is specified, ring B can be fused in any direction;
with the proviso that
when ring B is

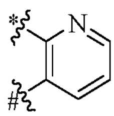

and $R_8$ and $R_9$ along with N connected thereto form

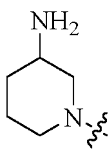 ,

X is N; when ring B is

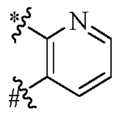

and X is $CR_6$, $R_8$ and $R_9$ along with N connected thereto form

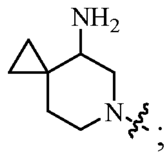

and
m is 0 or 1.

Preferably, $R_8$ and $R_9$ along with N connected thereto form piperidinyl substituted by amino.

Preferably, ring B is 5 membered heteroaryl; more preferably, ring B is

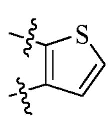 , 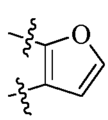 or 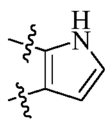 ;

further preferably, ring B is

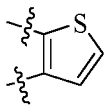 or 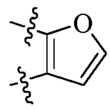 ;

and more further preferably, ring B is

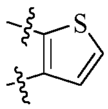 .

In one embodiment, X is $CR_6$, and Y is N.

In one embodiment, Y is $CR_6$, and X is N.

In one embodiment, X and Y are each independently selected from $CR_6$.

In one embodiment, X and Y are each independently selected from N.

**In another preferred embodiment, the compound of general formula (I) or the pharmaceutically acceptable salt, stereoisomer and tautomer thereof,**
wherein X and Y are each independently selected from $CR_6$ or N;
$R_1$ is hydrogen or $C_{1-6}$ alkyl;
$R_2$ is hydrogen, $C_{1-6}$ alkyl or $C_{1-6}$ alkoxy;
$R_3$ is hydrogen or $C_{1-6}$ alkyl;
$R_4$ is hydrogen, or $C_{1-6}$ alkyl unsubstituted or substituted by halogen or 3-6 membered cycloalkyl;
$R_5$ is hydrogen, halogen, $C_{1-6}$ alkyl or $C_{1-6}$ alkoxy;
$R_6$ is hydrogen or $C_{1-6}$ alkyl;
$R_7$ is hydrogen or $C_{1-6}$ alkyl;
$R_8$ and $R_9$ along with N connected thereto form 5-6 membered heterocyclyl unsubstituted or substituted by a substituent selected from amino;
ring B is 5 membered heteroaryl; and
m is an integer of 0 to 4.

Preferably, $R_8$ and $R_9$ along with N connected thereto form piperidinyl substituted by amino.

Preferably, ring B is

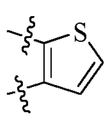, 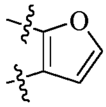 or 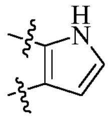;

more preferably, ring B is

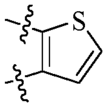 or 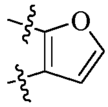;

and further preferably, ring B is

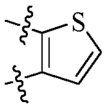.

In one embodiment, X is $CR_6$, and Y is N.

In one embodiment, X and Y are each independently selected from N.

**In another preferred embodiment, the compound of general formula (I) or the pharmaceutically acceptable salt, stereoisomer and tautomer thereof,**

wherein X and Y are each independently selected from $CR_6$ or N;
$R_1$ is hydrogen or $C_{1-6}$ alkyl;
$R_2$ is hydrogen, $C_{1-6}$ alkyl or $C_{1-6}$ alkoxy;
$R_3$ is hydrogen or $C_{1-6}$ alkyl;
$R_4$ is hydrogen, or $C_{1-6}$ alkyl unsubstituted or substituted by halogen, cyclopropyl or cyclobutyl;
$R_5$ is hydrogen or $C_{1-6}$ alkyl;
$R_6$ is hydrogen or $C_{1-6}$ alkyl;
$R_7$ is hydrogen or $C_{1-6}$ alkyl;
$R_8$ and $R_9$ along with N connected thereto form 5-6 membered heterocyclyl unsubstituted or substituted by a substituent selected from amino;
ring B is

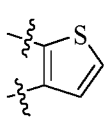, 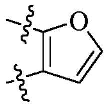 or 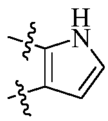;

and
m is an integer of 0 to 4.

Preferably, $R_8$ and $R_9$ along with N connected thereto form piperidinyl substituted by amino.

Preferably, ring B is

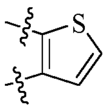 or 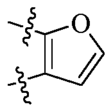;

and more preferably, ring B is

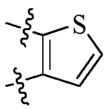.

In one embodiment, X is $CR_6$, and Y is N.

In one embodiment, X and Y are each independently selected from N.

**In another preferred embodiment, the compound of general formula (I) or the pharmaceutically acceptable salt, stereoisomer and tautomer thereof,**

wherein $R_8$ and $R_9$ along with N connected thereto form pyrrolidinyl, piperidinyl, piperazinyl or morpholinyl unsubstituted or substituted by a substituent selected from amino.

In one embodiment, X is $CR_6$, and Y is N.

In one embodiment, X and Y are each independently selected from N.

**In another preferred embodiment, the compound of general formula (I) or the pharmaceutically acceptable salt, stereoisomer and tautomer thereof,**

wherein $R_8$ and $R_9$ along with N connected thereto form

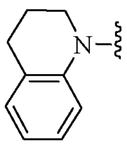,

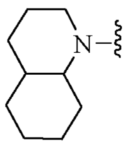, 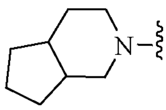, 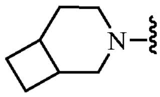, 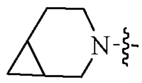, 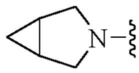, 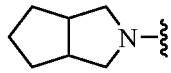,

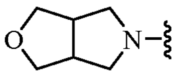, 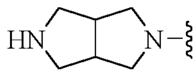, 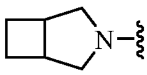 or 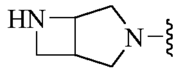

unsubstituted or substituted by a substituent selected from hydrogen, halogen, cyano, amino, hydroxyl, $C_{1-6}$ alkyl and $C_{1-6}$ alkoxy.

In one embodiment, X is $CR_6$, and Y is N.

In one embodiment, X and Y are each independently selected from N.

**In another preferred embodiment, the compound of general formula (I) or the pharmaceutically acceptable salt, stereoisomer and tautomer thereof,**

wherein $R_8$ and $R_9$ along with N connected thereto form

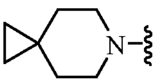,

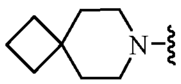, 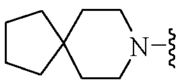, 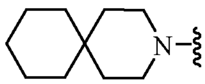, 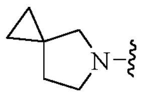, 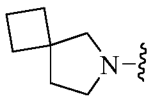, 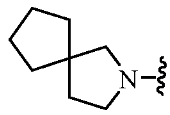,

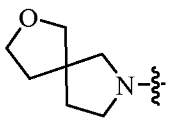, 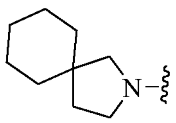, 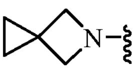, 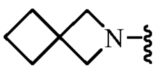, 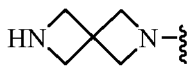, 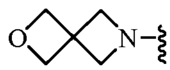,

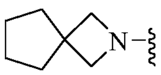, 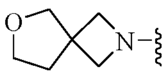, 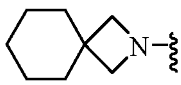 or 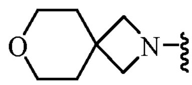

unsubstituted or substituted by a substituent selected from hydrogen, halogen, cyano, amino, hydroxyl, $C_{1-6}$ alkyl and

$C_{1-6}$ alkoxy.

In one embodiment, X is $CR_6$, and Y is N.

In one embodiment, X and Y are each independently selected from N.

**In another preferred embodiment, the compound of general formula (I) or the pharmaceutically acceptable salt, stereoisomer and tautomer thereof,**

wherein $R_8$ and $R_9$ along with N connected thereto form

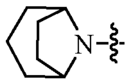

,

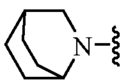

,

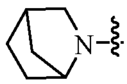

or

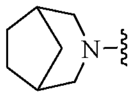

unsubstituted or substituted by a substituent selected from hydrogen, halogen, cyano, amino, hydroxyl, $C_{1-6}$ alkyl and $C_{1-6}$ alkoxy.

In one embodiment, X is $CR_6$, and Y is N.

In one embodiment, X and Y are each independently selected from N.

**In another preferred embodiment, the compound of general formula (I) or the pharmaceutically acceptable salt, stereoisomer and tautomer thereof,**

wherein $R_8$ is hydrogen, halogen, cyano, amino, hydroxyl, $-L_3-Cy_3$, or $C_{1-6}$ alkyl unsubstituted or substituted by halogen, cyano, amino or hydroxyl, $L_3$ is absent or is $C_{1-6}$ alkylene, $Cy_3$ is 3-6 membered cycloalkyl or 3-8 membered heterocyclyl, and $Cy_3$ may be optionally substituted by halogen, cyano, amino, hydroxyl, $C_{1-6}$ alkyl or $C_{1-6}$ alkoxy; and

$R_9$ is hydrogen or $C_{1-6}$ alkyl.

In one embodiment, X is $CR_6$, and Y is N.

In one embodiment, X and Y are each independently selected from N.

**In another preferred embodiment, the compound of general formula (I) or the pharmaceutically acceptable salt, stereoisomer and tautomer thereof,**

wherein X and Y are each independently selected from $CR_6$ or N;

$R_1$ is hydrogen or $C_{1-6}$ alkyl;

$R_2$ is hydrogen, $C_{1-6}$ alkyl or $C_{1-6}$ alkoxy;

$R_3$ is hydrogen or $C_{1-6}$ alkyl;

$R_4$ is hydrogen, or $C_{1-6}$ alkyl unsubstituted or substituted by halogen or 3-6 membered cycloalkyl;

$R_5$ is hydrogen, halogen, $C_{1-6}$ alkyl or $C_{1-6}$ alkoxy;

$R_6$ is hydrogen or $C_{1-6}$ alkyl;

$R_7$ is hydrogen or $C_{1-6}$ alkyl;

$R_8$ is hydrogen, or $C_{1-6}$ alkyl unsubstituted or substituted by amino;

$R_9$ is hydrogen or $C_{1-6}$ alkyl;

alternatively, $R_8$ and $R_9$ along with N connected thereto form 5-6 membered saturated nitrogen containing heterocyclyl unsubstituted or substituted by a substituent selected from hydrogen, amino and $C_{1-6}$ alkyl; preferably, the 5-6 membered saturated nitrogen containing heterocyclyl is piperidinyl;

ring B is

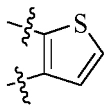

or

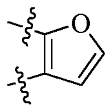

;

preferably, ring B is

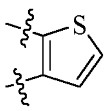

;

and
m is an integer of 0 to 4.

In one embodiment, X is $CR_6$, and Y is N.

In one embodiment, X and Y are each independently selected from N.

**In another preferred embodiment, the compound of general formula (I) or the pharmaceutically acceptable salt, stereoisomer and tautomer thereof,**
wherein X and Y are each independently selected from $CR_6$ or N;
$R_1$ is hydrogen or $C_{1-6}$ alkyl;
$R_2$ is hydrogen, $C_{1-6}$ alkyl or $C_{1-6}$ alkoxy;
$R_3$ is hydrogen or $C_{1-6}$ alkyl;
$R_4$ is hydrogen, or $C_{1-6}$ alkyl unsubstituted or substituted by halogen or 3-6 membered cycloalkyl;
$R_5$ is hydrogen, halogen, $C_{1-6}$ alkyl or $C_{1-6}$ alkoxy;
$R_6$ is hydrogen or $C_{1-6}$ alkyl;
$R_7$ is hydrogen or $C_{1-6}$ alkyl;
$R_8$ and $R_9$ along with N connected thereto form 5-6 membered saturated nitrogen containing heterocyclyl unsubstituted or substituted by a substituent selected from hydrogen, amino and $C_{1-6}$ alkyl; preferably, the 5-6 membered saturated nitrogen containing heterocyclyl is piperidinyl;
ring B is

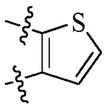
or
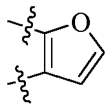
;

preferably, ring B is

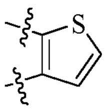
;

and
m is an integer of 0 to 4.

In one embodiment, X is $CR_6$, and Y is N.

In one embodiment, X and Y are each independently selected from N.

**In another preferred embodiment, the compound of general formula (I) or the pharmaceutically acceptable salt, stereoisomer and tautomer thereof,**
wherein X and Y are each independently selected from $CR_6$ or N;
$R_1$ is hydrogen or $C_{1-6}$ alkyl;
$R_2$ is hydrogen, $C_{1-6}$ alkyl or $C_{1-6}$ alkoxy;
$R_3$ is hydrogen or $C_{1-6}$ alkyl;
$R_4$ is hydrogen, or $C_{1-6}$ alkyl unsubstituted or substituted by halogen, cyclopropyl or cyclobutyl;
$R_5$ is hydrogen, halogen, $C_{1-6}$ alkyl or $C_{1-6}$ alkoxy;
$R_6$ is hydrogen or $C_{1-6}$ alkyl;
$R_7$ is hydrogen or $C_{1-6}$ alkyl;
$R_8$ and $R_9$ along with N connected thereto form 5-6 membered saturated nitrogen containing heterocyclyl unsubstituted or substituted by a substituent selected from amino; preferably, the 5-6 membered saturated nitrogen containing heterocyclyl is piperidinyl;
ring B is

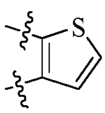
or
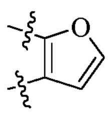
;

preferably, ring B is

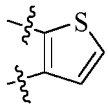
;

and

m is 0 or 1, and preferably, m is 0.

In one embodiment, X is $CR_6$, and Y is N.

In one embodiment, X and Y are each independently selected from N.

**In another preferred embodiment, the compound of general formula (I) or the pharmaceutically acceptable salt, stereoisomer and tautomer thereof,**

wherein X and Y are each independently selected from $CR_6$ or N;

$R_1$ is hydrogen or $C_{1-6}$ alkyl;

$R_2$ is hydrogen, $C_{1-6}$ alkyl or $C_{1-6}$ alkoxy;

$R_3$ is hydrogen or $C_{1-6}$ alkyl;

$R_4$ is butyl, isobutyl, trifluoroethyl, cyclopropylmethyl or cyclobutylmethyl;

$R_5$ is hydrogen, halogen, $C_{1-6}$ alkyl or $C_{1-6}$ alkoxy;

$R_6$ is hydrogen or $C_{1-6}$ alkyl;

$R_7$ is hydrogen or $C_{1-6}$ alkyl;

$R_8$ and $R_9$ along with N connected thereto form 5-6 membered saturated nitrogen containing heterocyclyl unsubstituted or substituted by a substituent selected from amino; preferably, the 5-6 membered saturated nitrogen containing heterocyclyl is piperidinyl;

ring B is

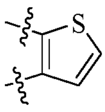
or
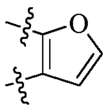
;

preferably, ring B is

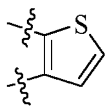
;

and

m is 0 or 1, and preferably, m is 0.

In one embodiment, X is $CR_6$, and Y is N.

In one embodiment, X and Y are each independently selected from N.

**In another preferred embodiment, the compound of general formula (I) or the pharmaceutically acceptable salt, stereoisomer and tautomer thereof,**

wherein X and Y are each independently selected from $CR_6$ or N;

$R_1$ is hydrogen;

$R_2$ is methoxy;

$R_3$ is methyl;

$R_4$ is ethyl, isobutyl, trifluoroethyl, cyclopropylmethyl or cyclobutylmethyl;

$R_5$ is hydrogen, halogen, $C_{1-6}$ alkyl or $C_{1-6}$ alkoxy;

$R_6$ is hydrogen;

$R_7$ is hydrogen;

$R_8$ is hydrogen, $-L_3-Cy_3$, or $C_{1-6}$ alkyl unsubstituted or substituted by amino;

$L_3$ is absent, $Cy_3$ is cyclobutyl, and $Cy_3$ is substituted by amino;

$R_9$ is methyl;

alternatively, $R_8$ and $R_9$ along with N connected thereto form pyrrolidinyl, piperidinyl or azacycloheptyl substituted by amino,

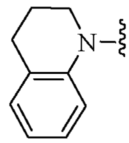

substituted by amino or

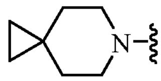

substituted by amino; ring B is

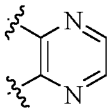, 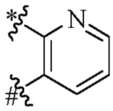, 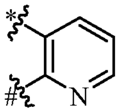, 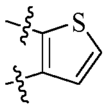 or 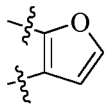;

and
m is 0 or 1.

In one embodiment, X is $CR_6$, and Y is N.

In one embodiment, X and Y are each independently selected from N.

**In another preferred embodiment, the compound of general formula (I) or the pharmaceutically acceptable salt, stereoisomer and tautomer thereof,**
wherein X and Y are each independently selected from $CR_6$ or N;
$R_1$ is hydrogen;
$R_2$ is methoxy;
$R_3$ is methyl;
$R_4$ is ethyl, isobutyl, trifluoroethyl, cyclopropylmethyl or cyclobutylmethyl, and preferably, $R_4$ is cyclopropylmethyl or cyclobutylmethyl;
$R_6$ is hydrogen;
$R_7$ is hydrogen;
$R_8$ and $R_9$ along with N connected thereto form piperidinyl substituted by amino;
ring B is

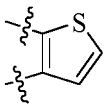;

and
m is 0.

In one embodiment, X is $CR_6$, and Y is N.

In one embodiment, X and Y are each independently selected from N.

Part of the compounds disclosed herein are shown in the following table:

| Serial number | Structure | Serial number | Structure |
|---|---|---|---|
| 1 | 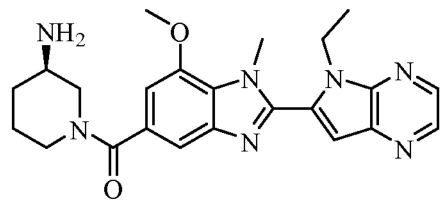 | 2 | 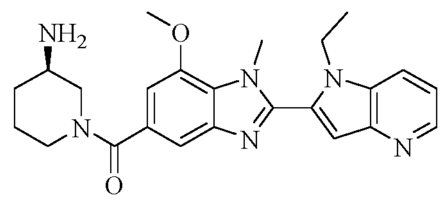 |
| 3 | 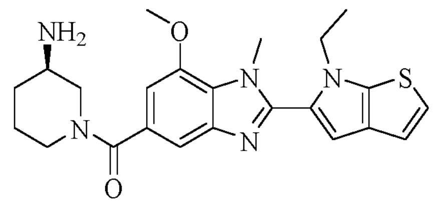 | 4 | 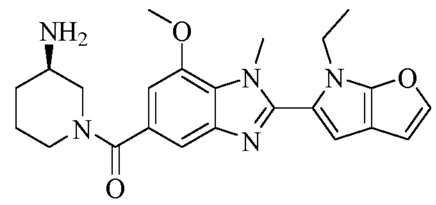 |
| 5 | 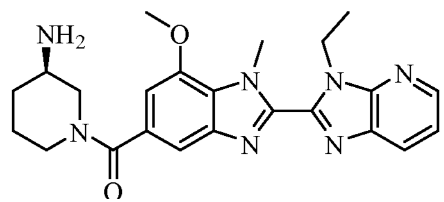 | | |
| | | 8 | 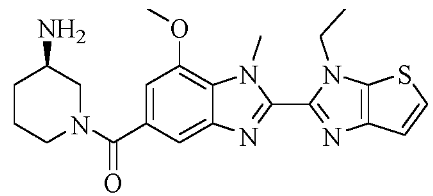 |
| 9 | 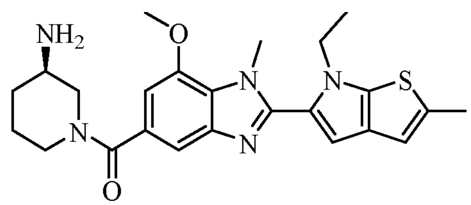 | 10 | 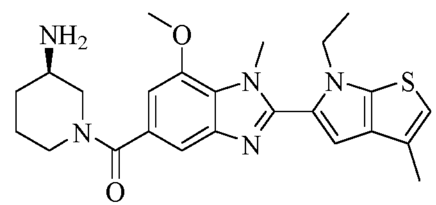 |

(continued)

| Serial number | Structure | Serial number | Structure |
|---|---|---|---|
| 11 | 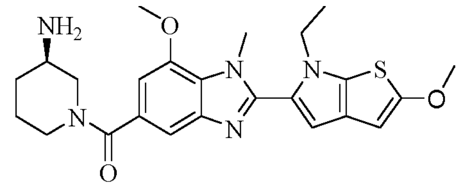 | 12 | 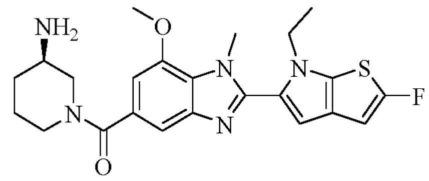 |
| 13 | 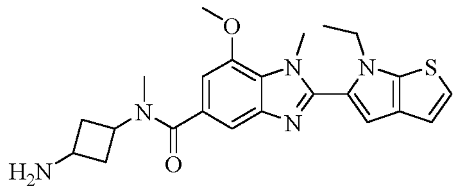 | 14 | 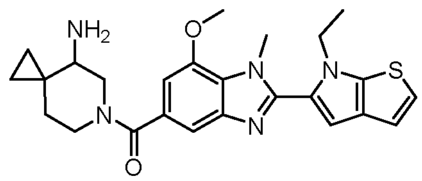 |
| 15 | 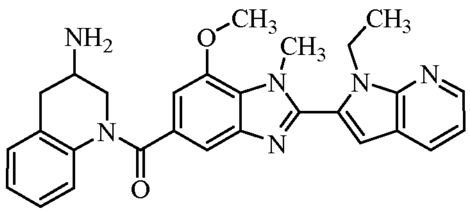 | 16 | 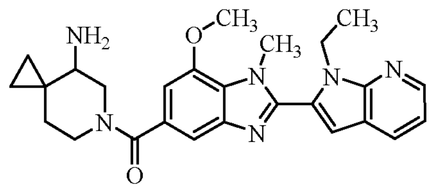 |
| 17 | 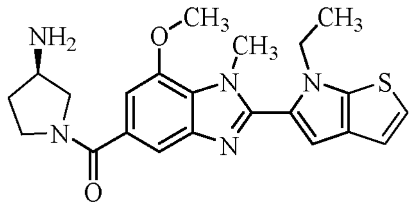 | 18 | 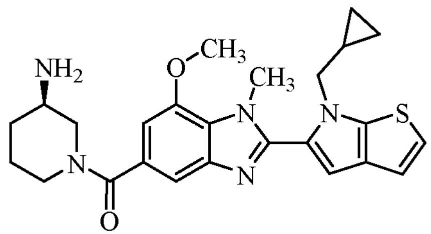 |

(continued)

| Serial number | Structure | Serial number | Structure |
|---|---|---|---|
| 19 | 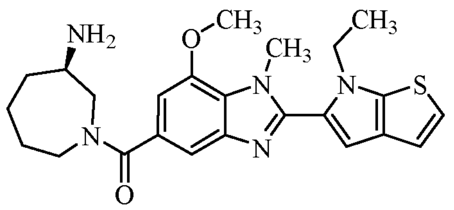 | 20 | 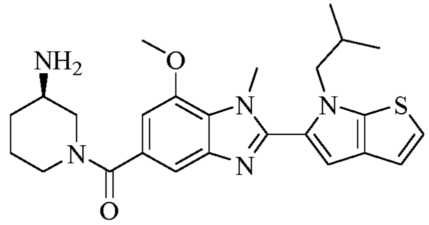 |
| 21 | 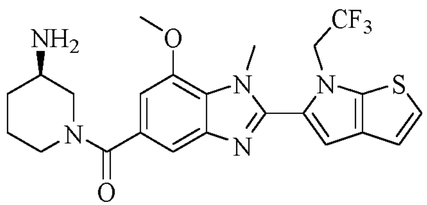 | 22 | 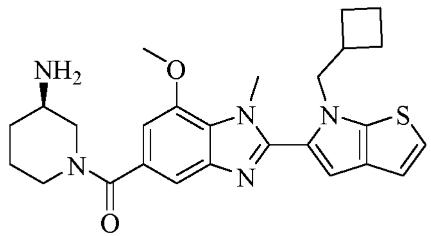 |
| 23 | 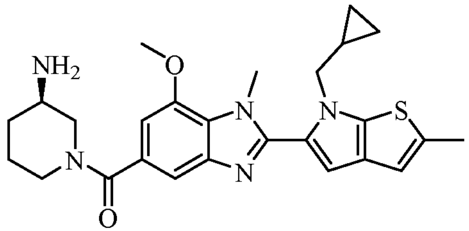 | 24 | 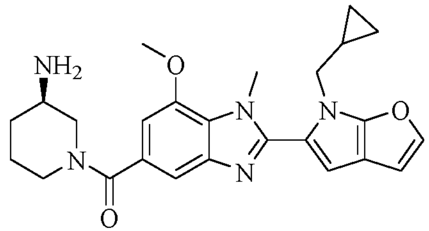 |
| 25 | 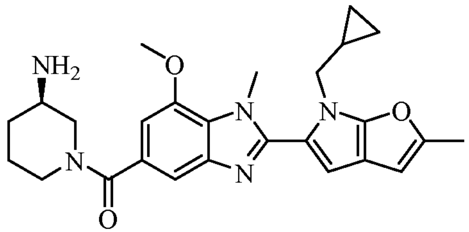 | | |

The present invention also claims a pharmaceutical composition that comprises one or more of the compounds or the pharmaceutically acceptable salts, stereoisomers and tautomers thereof disclosed herein, and optionally one or more pharmaceutical carriers, and can be optionally formulated into any pharmaceutically acceptable dosage form.

The pharmaceutical carrier described herein may be one or more solid or liquid carriers suitable for administration in human. Preferably, the pharmaceutical carrier has sufficient purity and low toxicity, is compatible with the active ingredients described herein and does not significantly decrease the efficacy of the active ingredients. For example, the pharmaceutical carrier may be selected from filler, binder, disintegrant, lubricant, aqueous solvent, nonaqueous solvent, *etc.*

The pharmaceutical composition disclosed herein may be optionally formulated into any pharmaceutically acceptable dosage form, and can be administered by any suitable route of administration, such as oral, parenteral, rectal or pulmonary administrations, to patients or subjects in need of such treatment. For oral administration, the pharmaceutical composition can be formulated into a tablet, capsule, pill, granule, *etc.* For parenteral administration, the pharmaceutical composition can be formulated into injection solution, sterile powder for injection, *etc.* For rectal administration, the pharmaceutical composition can be formulated into a suppository, *etc.* For pulmonary administration, the pharmaceutical composition can be formulated into aerosol, spray, dry powder inhalation, *etc.*

The present invention relates to use of the compound, the pharmaceutically acceptable salt, stereoisomer and tautomer thereof or the pharmaceutical composition thereof disclosed herein in preparing a medicament for treating or preventing diseases mediated by PAD4 (including diseases caused by abnormal expression of PAD4), such as rheumatoid arthritis, vasculitis, systemic lupus erythematosus, ulcerative colitis, multiple sclerosis, cystic fibrosis, cancer, cutaneous lupus erythematosus, asthma and psoriasis.

The present invention further relates to use of the compound, the pharmaceutically acceptable salt, stereoisomer and tautomer thereof or the pharmaceutical composition thereof disclosed herein in treating or preventing diseases mediated by PAD4 (including diseases caused by abnormal expression of PAD4), such as rheumatoid arthritis, vasculitis, systemic lupus erythematosus, ulcerative colitis, multiple sclerosis, cystic fibrosis, cancer, cutaneous lupus erythematosus, asthma and psoriasis.

The present invention further relates to the compound, the pharmaceutically acceptable salt, stereoisomer and tautomer thereof or the pharmaceutical composition thereof disclosed herein used for treating or preventing diseases. In one embodiment, the disease is a condition mediated by PAD4 (including diseases caused by abnormal expression of PAD4), such as rheumatoid arthritis, vasculitis, systemic lupus erythematosus, ulcerative colitis, multiple sclerosis, cystic fibrosis, cancer, cutaneous lupus erythematosus, asthma and psoriasis.

The present invention further relates to a method for treating or preventing diseases, comprising administering the compound, the pharmaceutically acceptable salt, stereoisomer and tautomer thereof or the pharmaceutical composition thereof disclosed herein to a patient in need. In one embodiment, the disease is a condition mediated by PAD4 (including diseases caused by abnormal expression of PAD4), such as rheumatoid arthritis, vasculitis, systemic lupus erythematosus, ulcerative colitis, multiple sclerosis, cystic fibrosis, cancer, cutaneous lupus erythematosus, asthma and psoriasis. In one embodiment, the patient is a mammal, preferably a human.

**Other embodiments of the present invention**

The present invention also relates to the following embodiments:

1. A compound of general formula (I) or a pharmaceutically acceptable salt, stereoisomer and tautomer thereof:

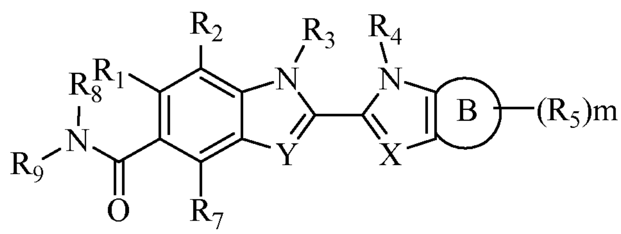

(I)

wherein X and Y are each independently selected from $CR_6$ or N;
$R_1$ is hydrogen or $C_{1-6}$ alkyl;
$R_2$ is hydrogen, $C_{1-6}$ alkyl, 3-6 membered cycloalkyl, $C_{1-6}$ alkoxy, $C_{1-6}$ haloalkyl or $C_{1-6}$ haloalkoxy;
$R_3$ is hydrogen, cyano, $C_{1-6}$ alkyl, $C_{1-6}$ alkoxy, $C_{1-6}$ alkoxy $C_{1-6}$ alkyl, $-L_1-Cy_1$, or $C_{1-6}$ alkyl, $C_{1-6}$ alkylcarbonyl,

$C_{1-6}$ alkoxycarbonyl, $C_{1-6}$ alkylsulfonyl, $C_{1-6}$ alkoxy $C_{1-6}$ alkyl, $C_{1-6}$ alkylamino or $(C_{1-6}$ alkyl$)_2$ amino unsubstituted or substituted by halogen, cyano, amino or hydroxyl, $L_1$ is absent or is $C_{1-6}$ alkylene, $C_{y1}$ is 3-12 membered cycloalkyl, 3-12 membered cycloalkenyl, 3-12 membered heterocyclyl, aryl or 5-10 membered heteroaryl, and $Cy_1$ may be optionally substituted by hydrogen, halogen, cyano, amino, hydroxyl, $C_{1-6}$ alkyl or $C_{1-6}$ alkoxy;
$R_4$ is hydrogen, cyano, 3-6 membered cycloalkyl, or $C_{1-6}$ alkyl, $C_{1-6}$ alkylcarbonyl, $C_{1-6}$ alkoxycarbonyl, $C_{1-6}$ alkylsulfonyl, $C_{1-6}$ alkoxy $C_{1-6}$ alkyl, $C_{1-6}$ alkylamino or $(C_{1-6}$ alkyl$)_2$ amino unsubstituted or substituted by halogen, cyano, amino, hydroxyl or 3-6 membered cycloalkyl;
$R_8$ is hydrogen, halogen, cyano, amino, hydroxyl, $-L_2-Cy_2$, or $C_{1-6}$ alkyl, $C_{2-8}$ alkenyl, $C_{1-6}$ alkylcarbonyl, $C_{1-6}$ alkylcarbonylamino, $C_{1-6}$ alkoxy, $C_{1-6}$ alkoxycarbonyl, $C_{1-6}$ alkylsulfonyl, $C_{1-6}$ alkylthio, $C_{1-6}$ alkylsulfonylamino, $C_{1-6}$ alkylamino or $(C_{1-6}$ alkyl$)_2$ amino unsubstituted or substituted by halogen, cyano, amino or hydroxyl, $L_2$ is absent or is $C_{1-6}$ alkylene, $C_{1-6}$ alkyleneoxy, $C_{2-6}$ alkenylene or $C_{1-6}$ alkyleneamino, $C_{y2}$ is 3-12 membered cycloalkyl, 3-12 membered cycloalkenyl, 3-12 membered heterocyclyl, aryl or 5-10 membered heteroaryl, and $C_{y2}$ may be optionally substituted by hydrogen, halogen, cyano, amino, hydroxyl, $C_{1-6}$ alkyl or $C_{1-6}$ alkoxy;
$R_6$ is hydrogen, $C_{1-6}$ alkyl or $C_{3-6}$ cycloalkyl;
$R_7$ is hydrogen or $C_{1-6}$ alkyl;
$R_8$ is hydrogen, halogen, cyano, amino, hydroxyl, $-L_3-Cy_3$, or $C_{1-6}$ alkyl, $C_{2-8}$ alkenyl, $C_{1-6}$ alkylcarbonyl, $C_{1-6}$ alkylcarbonylamino, $C_{1-6}$ alkoxy or $C_{1-6}$ alkoxycarbonyl unsubstituted or substituted by halogen, cyano, amino or hydroxyl, $L_3$ is absent or is $C_{1-6}$ alkylene or $C_{2-6}$ alkenylene, $C_{y3}$ is 3-12 membered cycloalkyl, 3-12 membered cycloalkenyl, 3-12 membered heterocyclyl, aryl or 5-10 membered heteroaryl, and $Cy_3$ may be optionally substituted by hydrogen, halogen, cyano, amino, hydroxyl, $C_{1-6}$ alkyl or $C_{1-6}$ alkoxy;
$R_9$ is hydrogen or $C_{1-6}$ alkyl;
alternatively, $R_8$ and $R_9$, along with N connected thereto, form 4-7 membered heterocyclyl, 6-11 membered ortho-heterocyclyl, 7-12 membered spiro-heterocyclyl or 6-10 membered bridged heterocyclyl unsubstituted or substituted by a substituent selected from hydrogen, halogen, cyano, amino, hydroxyl, $C_{1-6}$ alkyl, $C_{1-6}$ alkoxy, $C_{1-6}$ alkylamino, $(C_{1-6}$ alkyl$)_2$ amino and $-L_4-Cy_4$, $L_4$ is absent or is $C_{1-6}$ alkylene, and $Cy_4$ is 3-12 membered cycloalkyl, 3-12 membered cycloalkenyl, 3-12 membered heterocyclyl, aryl or 5-10 membered heteroaryl;
ring B is

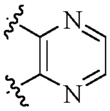 , 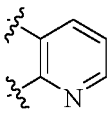 , 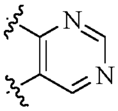 , 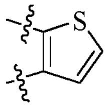 , 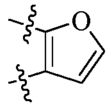 or 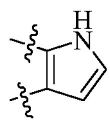 ;

and
m is an integer of 0 to 4.

2. The compound or the pharmaceutically acceptable salt, stereoisomer and tautomer thereof according to Embodiment 1,
wherein X and Y are each independently selected from $CR_6$ or N;
$R_1$ is hydrogen or $C_{1-6}$ alkyl;
$R_2$ is hydrogen, $C_{1-6}$ alkyl, $C_{1-6}$ alkoxy, $C_{1-6}$ haloalkyl or $C_{1-6}$ haloalkoxy;
$R_3$ is hydrogen, cyano, $C_{1-6}$ alkyl, $C_{1-6}$ alkoxy, $C_{1-6}$ alkoxy $C_{1-6}$ alkyl, or $C_{1-6}$ alkyl unsubstituted or substituted by halogen, cyano, amino or hydroxyl;
$R_4$ is hydrogen, cyano, 3-6 membered cycloalkyl, or $C_{1-6}$ alkyl unsubstituted or substituted by halogen, cyano, amino, hydroxyl or 3-6 membered cycloalkyl;
$R_8$ is hydrogen, halogen, cyano, amino, hydroxyl, or $C_{1-6}$ alkyl unsubstituted or substituted by halogen, cyano, amino or hydroxyl;
$R_6$ is hydrogen or $C_{1-6}$ alkyl;
$R_7$ is hydrogen or $C_{1-6}$ alkyl;
$R_8$ is hydrogen, halogen, cyano, amino, hydroxyl, $-L_3-Cy_3$, or $C_{1-6}$ alkyl unsubstituted or substituted by halogen, cyano, amino or hydroxyl, $L_3$ is absent or is $C_{1-6}$ alkylene, $Cy_3$ is 3-6 membered cycloalkyl, 3-12 membered heterocyclyl, aryl or 5-10 membered heteroaryl, and $Cy_3$ may be optionally substituted by hydrogen, halogen, cyano, amino, hydroxyl, $C_{1-6}$ alkyl or $C_{1-6}$ alkoxy;
$R_9$ is hydrogen or $C_{1-6}$ alkyl;
alternatively, $R_8$ and $R_9$ along with N connected thereto form 5-6 membered heterocyclyl, 7-10 membered ortho-heterocyclyl, 7-11 membered spiro-heterocyclyl or 7-10 membered bridged heterocyclyl unsubstituted or substituted by a substituent selected from hydrogen, halogen, cyano, amino, hydroxyl, $C_{1-6}$ alkyl and $C_{1-6}$ alkoxy;

ring B is

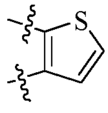, 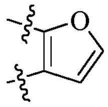 or 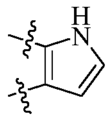;

and

m is an integer of 0 to 4.

3. The compound or the pharmaceutically acceptable salt, stereoisomer and tautomer thereof according to Embodiment 2,

wherein $R_8$ and $R_9$ along with N connected thereto form pyrrolidinyl, piperidinyl, piperazinyl or morpholinyl unsubstituted or substituted by a substituent selected from hydrogen, halogen, cyano, amino, hydroxyl, $C_{1-6}$ alkyl and $C_{1-6}$ alkoxy.

4. The compound or the pharmaceutically acceptable salt, stereoisomer and tautomer thereof according to Embodiment 2,

wherein $R_8$ and $R_9$ along with N connected thereto form

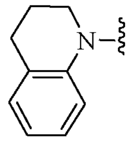,

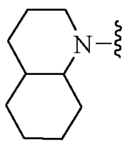, 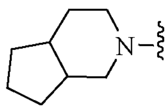, 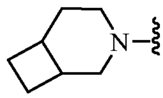, 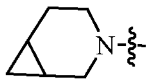, 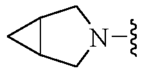, 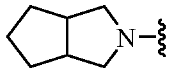,

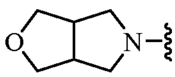, 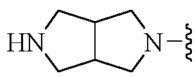, 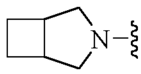 or 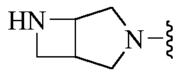

unsubstituted or substituted by a substituent selected from hydrogen, halogen, cyano, amino, hydroxyl, $C_{1-6}$ alkyl and $C_{1-6}$ alkoxy.

5. The compound or the pharmaceutically acceptable salt, stereoisomer and tautomer thereof according to Embodiment 2,

wherein $R_8$ and $R_9$ along with N connected thereto form

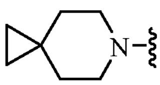,

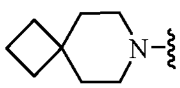, 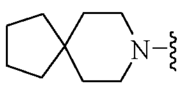, 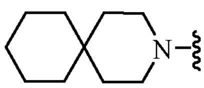, 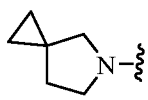, 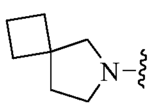, 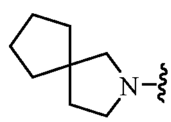,

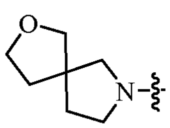, 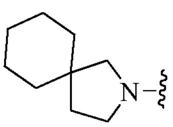, 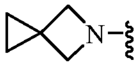, 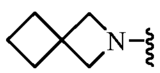, 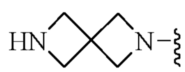, 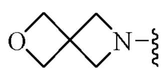,

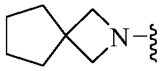, 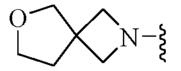, 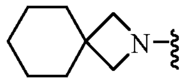 or 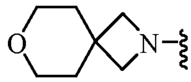

unsubstituted or substituted by a substituent selected from hydrogen, halogen, cyano, amino, hydroxyl, $C_{1-6}$ alkyl and $C_{1-6}$ alkoxy.

6. The compound or the pharmaceutically acceptable salt, stereoisomer and tautomer thereof according to Embodiment 2,

wherein $R_8$ and $R_9$ along with N connected thereto form

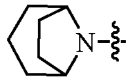,

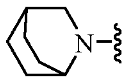, 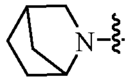 or 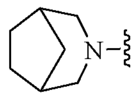

unsubstituted or substituted by a substituent selected from hydrogen, halogen, cyano, amino, hydroxyl, $C_{1-6}$ alkyl and $C_{1-6}$ alkoxy.

7. The compound or the pharmaceutically acceptable salt, stereoisomer and tautomer thereof according to Embodiment 2,

wherein $R_8$ is hydrogen, halogen, cyano, amino, hydroxyl, $-L_3-Cy_3$, or $C_{1-6}$ alkyl unsubstituted or substituted by halogen, cyano, amino or hydroxyl, $L_3$ is absent or is $C_{1-6}$ alkylene, $C_{y3}$ is 3-6 membered cycloalkyl or 3-8 membered heterocyclyl, and $Cy_3$ may be optionally substituted by halogen, cyano, amino, hydroxyl, $C_{1-6}$ alkyl or $C_{1-6}$ alkoxy; and $R_9$ is hydrogen or $C_{1-6}$ alkyl.

8. The compound or the pharmaceutically acceptable salt, stereoisomer and tautomer thereof according to Embodiment 1, which are selected from compounds with the following structures:

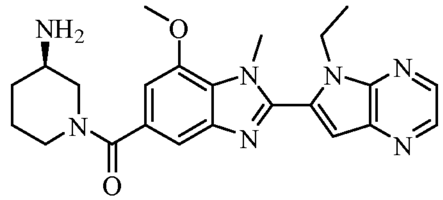, 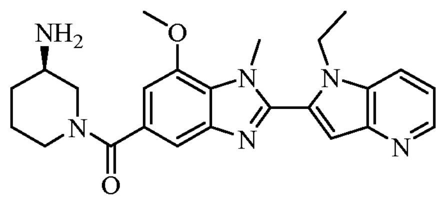,

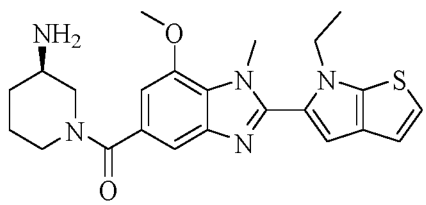, 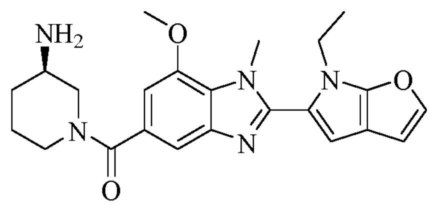,

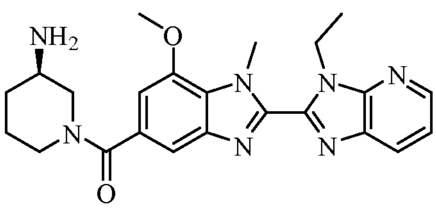, 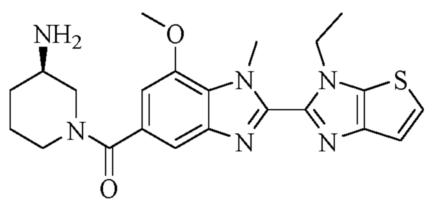,

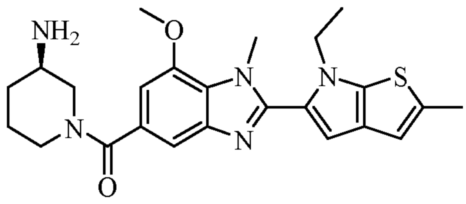,
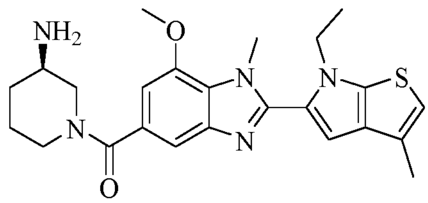,

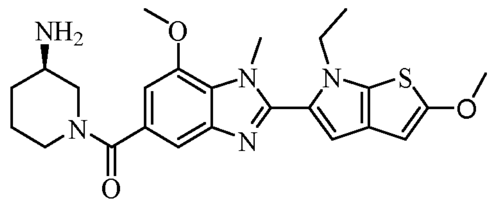,
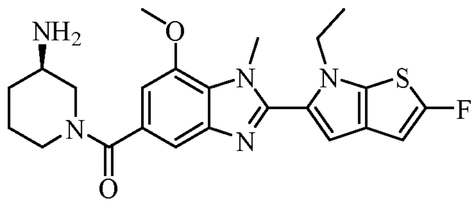,

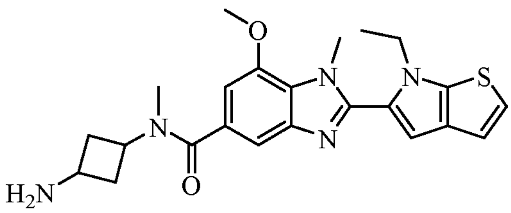.

9. A pharmaceutical composition containing the compound or the pharmaceutically acceptable salt, stereoisomer and tautomer thereof according to any one of Embodiments 1-8, which may optionally contain one or more pharmaceutical carriers and can be formulated into any pharmaceutically acceptable dosage form.

10. The pharmaceutical composition according to Embodiment 9, which may further comprise one or more second therapeutically active agents, wherein the second therapeutically active agents are antimetabolites, growth factor inhibitors, mitosis inhibitors, anti-tumor hormones, alkylating agents, metals, topoisomerase inhibitors, hormone drugs, immunomodulators, tumor suppressor genes, cancer vaccines, immune checkpoints or antibodies or small molecule drugs related to tumor immunotherapy.

11. Use of the compound or the pharmaceutically acceptable salt, stereoisomer and tautomer thereof according to any one of Embodiments 1-8 and the pharmaceutical composition according to Embodiment 9 in preparing a medicament for treating or preventing diseases mediated by peptidylarginine deiminase (PAD4).

12. The use according to Embodiment 11, wherein the diseases mediated by PAD4 are rheumatoid arthritis, vasculitis, systemic lupus erythematosus, ulcerative colitis, multiple sclerosis, cystic fibrosis, cancer, cutaneous lupus erythematosus, asthma and psoriasis.

13. The compound or the pharmaceutically acceptable salt, stereoisomer and tautomer thereof according to Embodiment 2,
wherein X and Y are each independently selected from $CR_6$ or N;
$R_1$ is hydrogen or $C_{1-6}$ alkyl;
$R_2$ is hydrogen, $C_{1-6}$ alkyl or $C_{1-6}$ alkoxy;
$R_3$ is hydrogen or $C_{1-6}$ alkyl;
$R_4$ is hydrogen, or $C_{1-6}$ alkyl unsubstituted or substituted by halogen or 3-6 membered cycloalkyl;
$R_8$ is hydrogen, halogen, or $C_{1-6}$ alkyl or $C_{1-6}$ alkoxy unsubstituted or substituted by halogen;
$R_6$ is hydrogen or $C_{1-6}$ alkyl;
$R_7$ is hydrogen or $C_{1-6}$ alkyl;
$R_8$ is hydrogen, or $C_{1-6}$ alkyl unsubstituted or substituted by amino;
$R_9$ is hydrogen or $C_{1-6}$ alkyl;
alternatively, $R_8$ and $R_9$ along with N connected thereto form 5-6 membered heterocyclyl unsubstituted or substituted by a substituent selected from amino;
ring B is

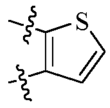, 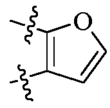 or 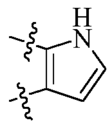;

and

m is an integer of 0 to 4.

14. The compound or the pharmaceutically acceptable salt, stereoisomer and tautomer thereof according to Embodiment 2,

wherein $R_8$ and $R_9$ along with N connected thereto form azacyclobutyl, azacycloheptyl, pyrrolidinyl, piperidinyl, piperazinyl or morpholinyl unsubstituted or substituted by a substituent selected from hydrogen, halogen, cyano, amino, hydroxyl, $C_{1-6}$ alkyl and $C_{1-6}$ alkoxy.

## DETAILED DESCRIPTION OF THE INVENTION

"Halogen" described herein refers to fluorine, chlorine, bromine, iodine, *etc.,* preferably fluorine atom and chlorine atom.

"Halo" described herein means that one or more hydrogen atoms in a substituent are substituted by one or more identical or different halogen atoms. "Halogen" is as defined above.

"$C_{1-6}$ alkyl" described herein refers to linear or branched alkyl derived by removing one hydrogen atom from alkane containing 1 to 6 carbon atoms, such as methyl, ethyl, *n*-propyl, isopropyl, *n*-butyl, isobutyl, *sec*-butyl, *tert*-butyl, *n*-pentyl, isopentyl, 2-methylbutyl, neopentyl, 1-ethylpropyl, *n*-hexyl, isohexyl, 4-methylpentyl, 3-methylpentyl, 2-methylpentyl, 1-methylpentyl, 3,3-dimethylbutyl, 2,2-dimethylbutyl, 1,1-dimethylbutyl, 1,2-dimethylbutyl, 1,3-dimethylbutyl, 2,3-dimethylbutyl, 2-ethylbutyl and 1-methyl-2-methylpropyl. "$C_{1-4}$ alkyl" refers to the aforementioned examples containing 1 to 4 carbon atoms, such as methyl, ethyl, *n*-propyl, isopropyl, *n*-butyl, isobutyl, *sec*-butyl and *tert-butyl.*

"$C_{2-8}$ alkenyl" described herein refers to linear or branched alkenyl derived by removing one hydrogen atom from alkene containing 2 to 8 carbon atoms and at least one carbon-carbon double bond, such as vinyl, 1-propenyl, 2-propenyl, 1-butenyl, 2-butenyl, 1,3-butadien-1-yl, 1-penten-3-yl, 2-penten-1-yl, 3-penten-1-yl, 3-penten-2-yl, 1,3-pentadien-1-yl, 1,4-pentadien-3-yl, 1-hexen -3-yl and 1,4-hexadien-1-yl. Preferably, "$C_{2-8}$ alkenyl" contains one carbon- carbon double bond.

"$C_{1-6}$ alkoxy" described herein refers to a group obtained by linking an oxygen atom to the parent molecule of "$C_{1-6}$ alkyl" defined above, *i.e.*, a "$C_{1-6}$ alkyl-O-" group, such as methoxy, ethoxy, *n*-propoxy, isopropoxy, *n*-butoxy, tert-butoxy, *n*-pentyloxy, neopentyloxy and *n*-hexyloxy. "$C_{1-4}$ alkoxy" refers to the aforementioned examples containing 1 to 4 carbon atoms, *i.e.*, a "$C_{1-4}$ alkyl-O-" group, such as methoxy, ethoxy, *n*-propoxy, isopropoxy, *n*-butoxy and *tert*-butoxy.

"$C_{1-6}$ alkoxy $C_{1-6}$ alkyl" described herein refers to a group formed by substituting $C_{1-6}$ alkyl with $C_{1-6}$ alkoxy.

"$C_{1-6}$ alkoxycarbonyl" described herein refers to $C_{1-6}$ alkoxy-C(O)- group.

"$C_{1-6}$ alkylamino", "($C_{1-6}$ alkyl)$_2$ amino", "$C_{1-6}$ alkylcarbonylamino", "$C_{1-6}$ alkylsulfonylamino", "$C_{1-6}$ alkylsulfonyl", "$C_{1-6}$ alkylthio" and "$C_{1-6}$ alkylcarbonyl" described herein refer to $C_{1-6}$ alkyl-NH- group, ($C_{1-6}$ alkyl)($C_{1-6}$ alkyl)N- group, $C_{1-6}$ alkyl-C(O)-NH- group, $C_{1-6}$ alkyl-S(O)$_2$-NH$_2$- group, $C_{1-6}$ alkyl-S(O)$_2$- group, $C_{1-6}$ alkyl-S- group and $C_{1-6}$ alkyl-C(O)- group, respectively.

"Ene" of "$C_{1-6}$ alkylene, $C_{2-6}$ alkenylene, $C_{1-6}$ alkyleneoxy and $C_{1-6}$ alkyleneamino" described herein respectively refers to divalent groups derived by removing two hydrogen atoms from $C_{1-6}$ alkyl, $C_{2-6}$ alkenyl, $C_{1-6}$ alkoxy and $C_{1-6}$ alkylamino groups.

The "fused ring" described herein refers to a multi-ring system structure formed by two or more ring structures connected by an ortho-, spiro-or bridged linkage. The ortho-fused ring refers to a fused ring structure formed by two or more ring structures sharing two adjacent ring atoms (*i.e.,* sharing a bond) with each other. The bridged ring refers to a fused ring structure formed by two or more ring structures sharing two non-adjacent ring atoms with each other. The spiro-ring refers to a fused ring structure formed by two or more ring structures sharing a ring atom with each other.

"3-12 membered cycloalkyl" described herein refers to a monovalent group or (as required) divalent group derived from 3-12 membered cycloalkane, which may be a monocyclic, bicyclic or polycyclic cycloalkyl system. Unless otherwise specified, all possibly formed monocyclic and polycyclic (including fused in the form of ortho-, spiro- or bridged) cases are included. A monocyclic system is typically a cyclic hydrocarbon group containing 3 to 12 carbon atoms (such as 3 to 8 or 3 to 6 carbon atoms). Examples of cycloalkyl include, but are not limited to: cyclopropyl, cyclobutyl, cyclopentyl, cyclohexyl, cycloheptyl, cyclooctyl, cyclopentyl-1, 3-diyl, cyclohexyl-1,4-diyl, cycloheptyl-1,4-diyl, *etc.* Fused cycloalkyl includes ortho-cycloalkyl, bridged cycloalkyl and spiro-cycloalkyl. Ortho-cycloalkyl may be 6-11 membered ortho-cycloalkyl (such as 7-10 membered ortho-cycloalkyl), and the representative examples include, but are not limited to, bicyclo[3.1.1]heptane, bicyclo[2.2.1]heptane, bicyclo[2.2.2] octane, bicyclo[3.2.2]nonane, bicyclo[3.3.1]nonane and bicyclo[4.2.1]nonyl. Spiro-cycloalkyl may be 7-12 membered spiro-cycloalkyl (such as 7-11 membered spiro-cycloalkyl), and the examples include, but are not limited to:

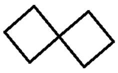, 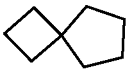, 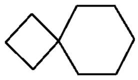, 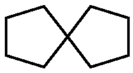, 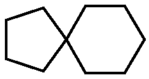 and 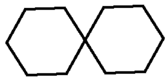

groups. Bridged cycloalkyl may be 6-10 membered bridged cycloalkyl (such as 7-10 membered bridged cycloalkyl), and the examples include, but are not limited to:

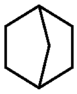, 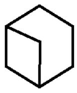, 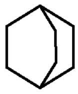, 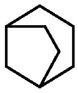, 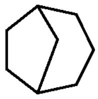 and 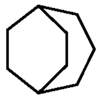

groups.

"3-12 membered cycloalkenyl" described herein refers to a group obtained by having at least one double bond in the aforementioned 3-12 membered cycloalkyl group, such as cyclobutenyl, cyclopentenyl, cyclohexenyl, cycloheptenyl and cyclooctenyl. Therefore, unless otherwise specified, "3-12 membered cycloalkenyl" described herein includes all possibly formed monocyclic and fused ring (including fused in the form of ortho-, spiro- or bridged) cases, such as 3-8 membered cycloalkenyl, 7-11 membered spiro-cycloalkenyl, 7-11 membered ortho-cycloalkenyl and 6-11 membered bridged cycloalkenyl. Spiro-cycloalkenyl may be 7-12 membered spiro-cycloalkenyl (such as 7-11 membered spiro-cycloalkenyl), and the examples include, but are not limited to:

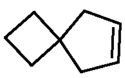 and 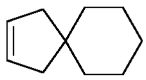

groups. Bridged cycloalkenyl may be 6-10 membered bridged cycloalkenyl (such as 7-10 membered bridged cycloalkenyl), and the examples include, but are not limited to:

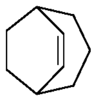

group.

"3-12 membered heterocyclyl" described herein refers to a monovalent group or (as required) divalent group derived from 3-12 membered heterocycloalkane, *i.e.*, a non-aromatic cyclic group obtained by substituting at least one ring carbon atom of 3-12 membered heterocycloalkane with a heteroatom selected from O, S, S(O), $S(O)_2$, C(O) and N, which preferably contains 1 to 3 heteroatoms. "3-12 membered heterocyclyl" includes monocyclic heterocyclyl system, bicyclic heterocyclyl system or polycyclic heterocyclyl system, wherein one or more rings may be saturated or partially saturated, but do not include aromatic ring. Unless otherwise specified, all possibly formed monocyclic, fused ring (including fused in the form of ortho-, spiro- or bridged), saturated and partially saturated cases are included.

Monocyclic heterocyclyl may be 3-8 membered heterocyclyl (such as 3-6 membered heterocyclyl, 4-7 membered heterocyclyl or 5-6 membered heterocyclyl), 3-8 membered nitrogen containing heterocyclyl (such as 4-7 membered nitrogen containing heterocyclyl or 5-6 membered nitrogen containing heterocyclyl) and 3-8 membered saturated heterocyclyl (such as 5-6 membered saturated heterocyclyl). The examples include, but are not limited to, azacyclopropyl, ozacyclopropyl, thiocyclopropyl, azacyclobutyl, oxacyclobutyl, thiocyclobutyl, tetrahydrofuranyl, tetrahydropyrrolyl, tetrahydrothienyl, imidazolidinyl, pyrazolidinyl, 1,2-oxazolidinyl, 1,3-oxazolidinyl, 1,2-thiazolidinyl, 1,3-thiazolidinyl, tetrahydro-2H-pyranyl, tetrahydro-2*H*- thiopyranyl, piperidinyl, piperazinyl, morpholinyl, 1,4-dioxanyl, 1,4-oxathianyl, 4,5-dihydroisoxazolyl, 4,5-dihydrooxazolyl, 2,5-dihydrooxazolyl, 2,3-dihydrooxazolyl, 3,4-dihydro-2*H*-pyrrolyl, 2,3-dihydro-1*H*-pyrrolyl, 2,5-dihydro-1*H*-imidazolyl, 4,5-dihydro-1*H*-imidazolyl, 4,5-dihydro-1*H*- pyrazolyl, 4,5-dihydro-3*H*-pyrazolyl, 4,5-dihydrothiazolyl, 2,5-dihydrothiazolyl, 2*H*-pyranyl, 4*H*-pyranyl, 2*H*-thiopyranyl, 4*H*-thiopyranyl, 2,3,4,5-tetrahydropyridyl, 1,2-isoxazinyl, 1,4-isoxazinyl, 6*H*-1,3-oxazinyl, *etc.*

Fused heterocyclyl includes ortho-heterocyclyl, spiro-heterocyclyl and bridged heterocyclyl, which may be saturated, partially saturated or unsaturated, but non-aromatic. Fused heterocyclyl may be a 5-6 membered monocyclic

heterocyclyl ring which is fused to a benzene ring, 5-6 membered monocyclic cycloalkyl, 5-6 membered monocyclic cycloalkenyl, 5-6 membered monocyclic heterocyclyl or 5-6 membered monocyclic heteroaryl.

The ortho-heterocyclyl may be 6-12 membered ortho-heterocyclyl (such as 6-11 membered ortho-heterocyclyl or 7-10 membered ortho-heterocyclyl), 6-11 membered saturated ortho-heterocyclyl or 6-11 membered nitrogen containing ortho-heterocyclyl, and the representative examples include, but are not limited to: 3-azabicyclo[3.1.0]hexyl, 3,6-diazabicyclo[3.2.0]heptyl, 3,8-diazabicyclo[4.2.0]octyl, 3,7-diazabicyclo[4.2.0]octyl, octahydropyrrolo [3,4-*c*]pyrrolyl, octahydropyrrolo[3,4-*b*]pyrrolyl, octahydropyrrolo[3,4-*b*][1,4] oxazinyl, octahydro-1*H*-pyrrolo[3,4-*c*]pyridyl, 2,3-dihydrobenzofuran-2-yl, 2,3-dihydrobenzofuran-3-yl, indolin-1-yl, indolin-2-yl, indolin-3-yl, 2,3-dihydrobenzothiophen-2-yl, octahydro-1*H*-indolyl and octahydrobenzofuranyl.

The spiro-heterocyclyl may be 6-12 membered spiro-heterocyclyl (such as 7-12 membered spiro-heterocyclyl), 7-12 membered saturated spiro-heterocyclyl or 7-12 membered nitrogen containing spiro-heterocyclyl, and the examples include, but are not limited to:

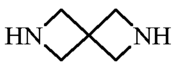, 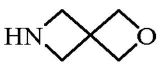, 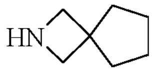,

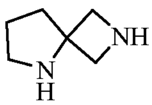, 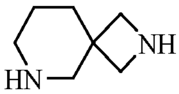, 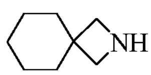, 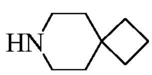, 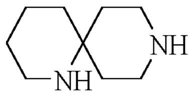, 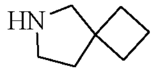,

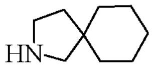 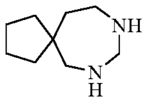, 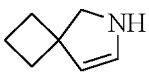, 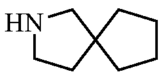 and 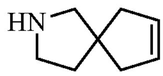.

The bridged heterocyclyl may be 6-12 membered bridged heterocyclyl (such as 6-10 membered bridged heterocyclyl or 7-10 membered bridged heterocyclyl), and the examples include, but are not limited to:

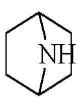, 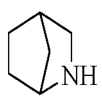,

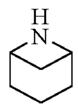, 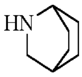, 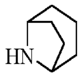, 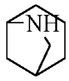, 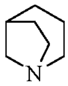, 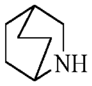, 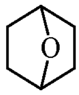, 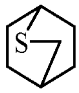, 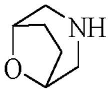 and 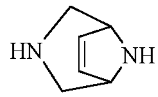.

"Aryl" described herein refers to a monovalent or (as required) divalent cyclic aromatic group containing 6 to 14 carbon atoms derived from aromatic carbocyclic hydrocarbon, including phenyl, naphthyl, phenanthryl, *etc.*

"5-10 membered heteroaryl" described herein refers to an aromatic 5-10 membered cyclic group in which at least one ring carbon atom is substituted by a heteroatom selected from O, S and N, which preferably contains 1 to 3 heteroatoms, and moreover, the case where carbon atoms or sulfur atoms are oxidized or nitridized is included. For example, carbon atoms are substituted by C(O) and sulfur atoms are substituted by S(O) or $S(O)_2$. Heteroaryl includes monocyclic heteroaryl and fused heteroaryl. Unless otherwise specified, a certain membered heteroaryl includes all possibly formed monocyclic, fused ring, fully aromatic and partially aromatic cases. Monocyclic heteroaryl may be 5-7 membered heteroaryl (such as 5-6 membered heteroaryl), and the examples include, but are not limited to, furanyl, imidazolyl, isoxazolyl, thiazolyl, isothiazolyl, oxadiazolyl, oxazolyl, pyridyl, pyridazinyl, pyrimidinyl, pyrazinyl, pyrazolyl, pyrrolyl, tetrazolyl, thiadiazolyl, thienyl, triazolyl and triazinyl. In some embodiments, fused heteroaryl refers to a group which is formed by fusing a monocyclic heteroaromatic ring to phenyl, cycloalkenyl, heteroaryl, cycloalkyl or heterocyclyl. In some embodiments, fused heteroaryl may be 8-12 membered ortho-heteroaryl (such as 9-10 membered ortho-heteroaryl), and the examples include, but are not limited to, benzimidazolyl, benzofuranyl, benzothienyl, benzoxadiazolyl, benzothiadiazolyl, benzothiazolyl, cinnolinyl, 5,6-dihydroquinolin-2-yl, 5,6-dihydroisoquinolin-1-yl, furopyridinyl, indazolyl, indolyl, isoindolyl, isoquinolinyl, naphthyridinyl, purinyl, quinolinyl, 5,6,7,8-tetrahydroquinolin-2-yl, 5,6,7,8-tetrahydroquinolinyl, 5,6,7,8-tetrahydroquinolin-4-yl, 5,6,7,8-tetrahydroisoquinolin-1-yl, thienopyridyl, 4,5,6,7-tetrahydro[*c*][1,2,5]oxadiazolyl and 6,7-d$_i$hydro[*c*][1,2,5]oxadiazol-4 (5*H*)keto.

The "pharmaceutically acceptable salt" described herein refers to a pharmaceutically acceptable addition salt of acid or base or a solvate thereof. Such pharmaceutically acceptable salts include addition salts of acids such as hydrochloric acid, phosphoric acid, hydrobromic acid, sulfuric acid, sulfurous acid, formic acid, toluenesulfonic acid, methanesulfonic acid, nitric acid, benzoic acid, citric acid, tartaric acid, maleic acid, hydroiodic acid and alkanoic acid (such as acetic acid and $HOOC-(CH_2)n-COOH$ (n is 0 to 4)). Such pharmaceutically acceptable salts also include addition salts of bases such as sodium salt, potassium salt, calcium salt, and ammonium salt. A variety of nontoxic pharmaceutically acceptable addition salts are known to those skilled in the art.

Stereoisomers refer to isomers which are produced by different spatial arrangements of atoms in molecules. When an asymmetric carbon atom exists in a compound, an enantiomer will be produced. When a carbon-carbon double bond or a cyclic structure exists in a compound, a cis-trans isomer will be produced. All enantiomers, diastereomers, racemes, cis-trans isomers, geometric isomers, epimers and mixtures thereof of the compound of formula (I) are included in the scope of the present invention.

"Tautomer" refers to a special functional group isomer produced by the rapid shifting of a certain atom between two positions in a molecule. The examples include a tautomer of a carbonyl compound containing α-H, such as

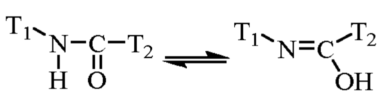 and 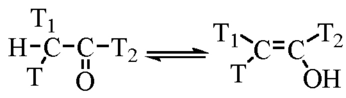

(T, $T_1$ and $T_2$ are each independently selected from any group meeting the bonding rule of compounds), and other prototropic tautomers, such as a phenol-keto tautomer, a nitroso-oximino tautomer and an imine-enamine tautomer.

All numerical ranges mentioned in the present invention include both endpoints of the ranges, all integers within the range and subranges formed by these integers. For example, "3-12 membered" includes 3, 4, 5, 6, 7, 8, 9, 10, 11 or 12 membered, "5-10 membered", "10 membered" and so on.

All variables involved in the compounds of the general formulas described herein, which are presented in the specification, including X, Y, $R_1$, $R_2$, $R_3$, $R_4$, $R_5$, $R_7$, $R_8$, $R_9$, ring B and m, are independently selected. Various levels of definitions of these variables listed in the specification can be combined with one another, and moreover, the present invention comprises the definitions of the compounds of the general formulas composed of any one of these combinations.

**Compound preparation**

The compound disclosed herein can be prepared by a variety of methods including standard chemical methods. Unless otherwise stated, any variable defined above will continue to have the meaning defined above. Exemplary general synthesis methods are elaborated in the following schemes, and can be easily modified to prepare other compounds disclosed herein. The specific compounds disclosed herein were prepared in examples.

The compound of formula (I) can be prepared by deprotecting the compound of formula (II). The method for preparing the compound of formula (I) by deprotecting the compound of formula (II) is as follows:

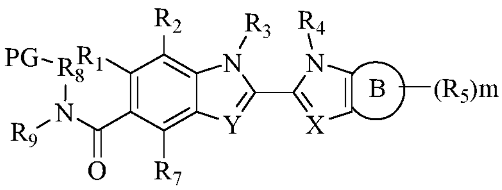

(II)

wherein $R_1$, $R_2$, $R_3$, $R_4$, $R_5$, $R_7$, $R_8$, $R_9$, ring B, X, Y and m are as defined above, and PG is a suitable protecting group for amine, such as *tert*-butyloxycarbonyl (Boc) or benzyloxycarbonyl (Cbz). Thereafter, if necessary, a salt of the compound formed in this way can be prepared.

For example, a suitable reaction reagent (such as trifluoroacetic acid, a solution of hydrogen chloride in ethanol, trimethyliodosilane or trimethylsilyl trifluoromethanesulfonate, and phenol in some embodiments) is added into a solution of the compound of formula (II) in a suitable solvent (such as dichloromethane, ethanol, methanol or acetonitrile), and the resulting mixture is stirred at an appropriate temperature (0 °C or ambient temperature) for an appropriate period of time (such as 10 min to 10 h). After the end of reaction, a suitable base (such as saturated aqueous sodium bicarbonate solution or ammonia solution) is added into the reaction solution, so that the pH of the reaction mixture is adjusted to be neutral, or the reaction mixture is concentrated under reduced pressure. After the crude product is dissolved in water,

a suitable base (such as saturated aqueous sodium bicarbonate solution or ammonia solution) is added, so that the pH value is adjusted to be neutral. Extraction is carried out with a suitable extractant (such as dichloromethane or ethyl acetate). After concentration under reduced pressure, the compound of formula (I) is separated by a suitable purification method (such as silica gel column chromatography, preparative thin-layer chromatography or reversed-phase column chromatography).

When Y is N, the compound of formula (II) can be prepared through the reaction between the compound of formula (III) and the compound of formula (IV).

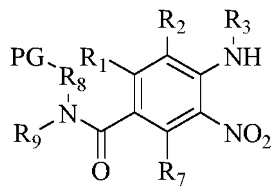

(III)

wherein $R_1$, $R_2$, $R_3$, $R_7$, $R_8$ and $R_9$ are as defined above, and PG is a suitable protecting group for amine, such as *tert*-butyloxycarbonyl (Boc) or benzyloxycarbonyl (Cbz).

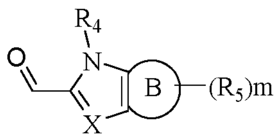

(IV)

wherein $R_4$, ring B, $R_5$ and m are as defined above.

The method for preparing the compound of formula (II) through the reaction between the compound of formula (III) and the compound of formula (IV) is as follows:

For example, the compound of formula (III) and the compound of formula (IV) are added into a suitable solvent (such as ethanol and water), a suitable reducing agent (such as sodium dithionite) is added, and the resulting mixture is heated to an appropriate temperature (such as 90 °C to 100 °C) or stirred under microwaves for an appropriate period of time (such as 4 to 8 h). After the end of reaction, the reaction solution is poured into an appropriate amount of water and extracted with a suitable extractant (such as ethyl acetate). After concentration under reduced pressure, the compound of formula (II) is separated by a suitable purification method (such as silica gel column chromatography or preparative thin-layer chromatography).

The compound of formula (III) can be prepared through the reaction between the compound of formula (VII) and the compound of formula (VIII).

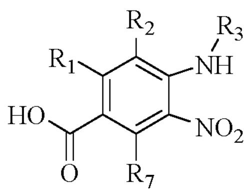

(VII)

wherein $R_1$, $R_2$, $R_3$ and $R_7$ are as defined above.

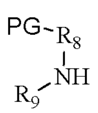

(VIII)

wherein $R_8$ and $R_9$ are as defined above, and PG is a suitable protecting group for amine, such as *tert*-butyloxycarbonyl (Boc) or benzyloxycarbonyl (Cbz).

The method for preparing the compound of formula (III) through the reaction between the compound of formula (VII) and the compound of formula (VIII) is as follows:
For example, the compound of formula (VII) and the compound of formula (VIII) are added into a suitable solvent (such as tetrahydrofuran and DMF), a suitable peptide coupling agent (such as 2-(7-azabenzotriazol-1-yl)- *N,N,N',N'*-tetramethyluronium hexafluorophosphate) and a suitable base (such as *N,N*-diisopropylethylamine) are successively added, and the resulting mixture is stirred at an appropriate temperature (such as ambient temperature) for an appropriate period of time (such as 2 to 10 h). The reaction solution is concentrated under reduced pressure, added with a proper amount of water and extracted with a suitable extractant (such as ethyl acetate), and the extract is concentrated. The crude product is separated by a conventional purification method (such as silica gel column chromatography) to give the compound of formula (III).

In some embodiments, the compound of formula (IV) can be prepared by oxidizing the compound of formula (IX).

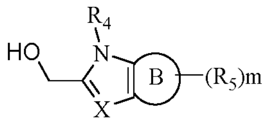

(IX)

wherein $R_4$, X, ring B, $R_5$ and m are as defined above.

The method for preparing the compound of formula (IV) by oxidizing the compound of formula (IX) is as follows:
For example, the compound of formula (IX) is added into a suitable solvent (such as dichloromethane), and a suitable oxidant (such as manganese dioxide) is added. The resulting mixture is stirred at an appropriate temperature (such as reflux) for an appropriate period of time (such as 2 h). The reaction solution is filtered, the filter cake is rinsed with an appropriate amount of solvent, and the filtrate is concentrated under reduced pressure to give the compound of formula (IV).

The compound of formula (IX) can be prepared by reducing the compound of formula (X).

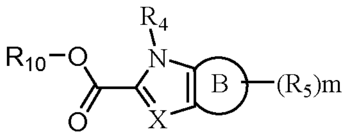

(X)

wherein $R_4$, X, ring B, $R_5$ and m are as defined above, and $R_{10}$ is methyl or ethyl.

The method for preparing the compound of formula (IX) by reducing the compound of formula (X) is as follows:
For example, the compound of formula (X) is added into a suitable solvent (such as tetrahydrofuran), and a suitable reducing agent (such as lithium aluminum hydride or diisobutyl aluminum hydride) is added. The resulting mixture is stirred at an appropriate temperature (such as ambient temperature) for an appropriate period of time (such as 2 to 10 h). A suitable quenching agent (such as water) is added. After filtration, a drying agent (such as anhydrous sodium sulfate) is added for drying. The filtrate is concentrated under reduced pressure to give the compound of formula (IX).

The compound of formula (X) can be prepared through the alkylation reaction between the compound of formula (XI) and $R_4L$.

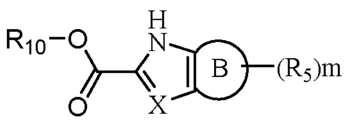

(XI)

wherein $R_4$, X, ring B, $R_5$, $R_{10}$ and m are as defined above, and L is a leaving group (such as halogen or trifluoromethanesulfonate).

The method for preparing the compound of formula (X) by alkylating the compound of formula (XI) is as follows:

For example, the compound of formula (XI) and $R_4L$ are added into a suitable solvent (such as acetonitrile), and a suitable base (such as potassium carbonate or cesium carbonate) is added. The resulting mixture is stirred at an appropriate temperature (such as ambient temperature to 90 °C) for an appropriate period of time (such as 1 to 35 h). After the end of reaction, filtration is performed. The filtrate is concentrated under reduced pressure. The crude product is separated by a conventional purification method (such as silica gel column chromatography) to give the compound of formula (X).

In some embodiments, the compound of formula (IV) can be prepared through the alkylation reaction between the compound of formula (XII) and $R_4L$.

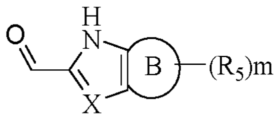

(XII)

wherein X, ring B, $R_5$ and m are as defined above, and L is a leaving group (such as halogen or trifluoromethanesulfonate).

The method for preparing the compound of formula (IV) by alkylating the compound of formula (XII) is as follows: For example, the compound of formula (XII) and $R_4L$ are added into a suitable solvent (such as DMF), and a suitable base (such as sodium hydride or potassium carbonate) is added. The resulting mixture is stirred at an appropriate temperature (such as -20 °C to 40 °C) for an appropriate period of time (such as 1 to 2 h). After the end of reaction, water is added, and extraction is carried out with a suitable extractant (such as ethyl acetate). The extract is concentrated under reduced pressure. The crude product is separated by a conventional purification method (such as silica gel column chromatography) to give the compound of formula (IV).

When X is $CR_6$, the compound of formula (XI) can be prepared in accordance with the following scheme:

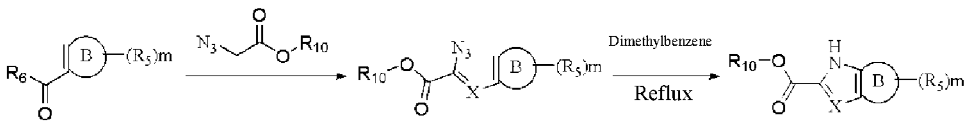


(XI)

wherein $R_6$, ring B, $R_5$, $R_{10}$ and m are as defined above.

When X is N, the compound of formula (IV) can be prepared in accordance with the following scheme:

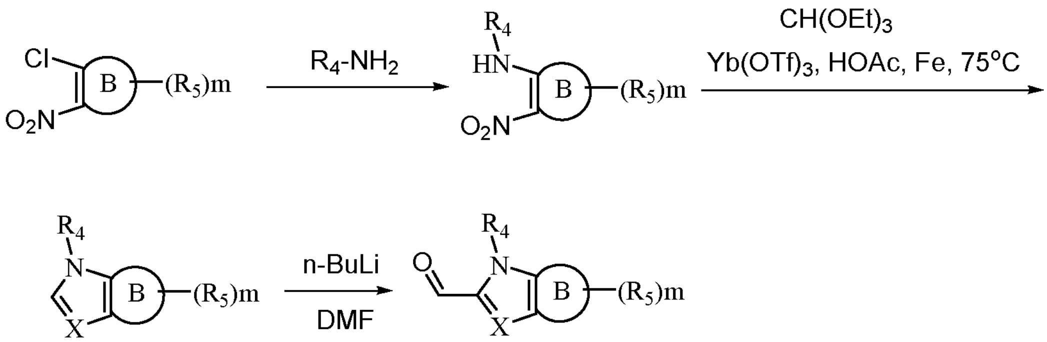


(IV)

wherein $R_4$, ring B, $R_5$ and m are as defined above.

The compound of formula (XII) can be prepared in accordance with the following scheme:

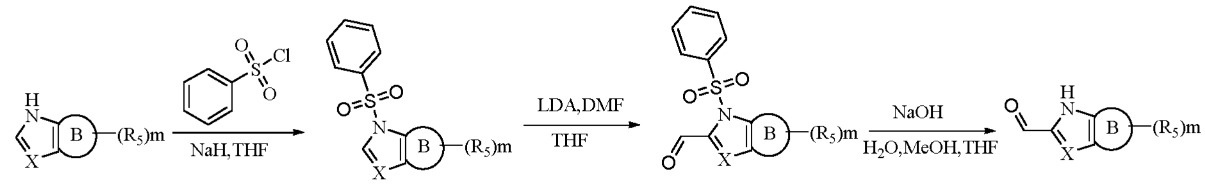


wherein X, ring B, $R_5$ and m are as defined above.

EXAMPLES

Among abbreviations used herein, "DMF" refers to dimethylformamide; "THF" refers to tetrahydrofuran; "EA" refers to ethyl acetate; "DCM" refers to dichloromethane; "TMEDA" refers to tetramethylethylenediamine; "LDA" refers to lithium diisopropylamide; "PE" refers to petroleum ether; "DIPEA" refers to *N*,*N*-diisopropylethylamine; "HATU" refers to 2-(7-azabenzotriazol-1-yl)-*N*,*N*,*N*',*N*'-tetramethyluronium hexafluorophosphate; and "DMSO" refers to dimethyl sulfoxide.

**Example 1: Synthesis of intermediate methyl 3-methoxy-4-(methylamino) - 5-nitrobenzoate**

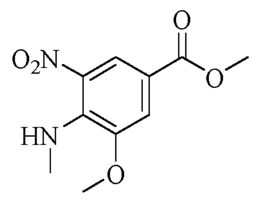

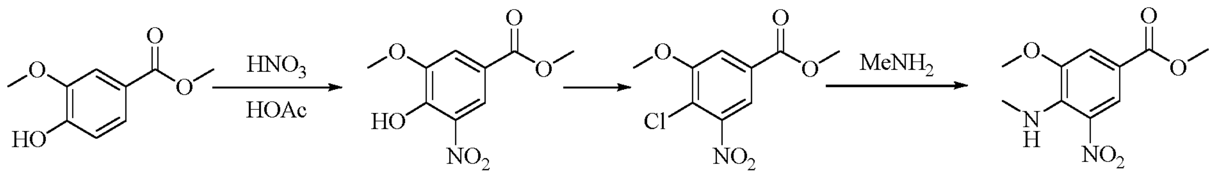


Step 1: Synthesis of methyl 4-hydroxy-3-methoxy-5-nitrobenzoate

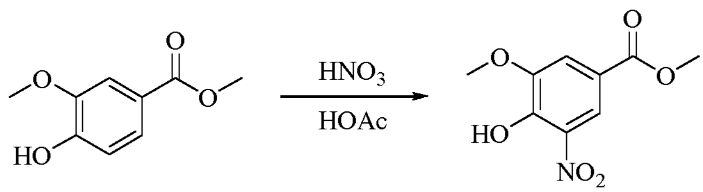


Methyl 4-hydroxy-3-methoxybenzoate (25.4 g, 0.139 mol) was dissolved in glacial acetic acid (100 mL), and a mixed solution of nitric acid (10.2 mL) and acetic acid (52 mL) was slowly added dropwise under an ice bath for about 1 h. The reaction solution was slowly warmed to room temperature and stirred for 4 h. Filtration was performed, and the filter cake was successively washed with water, *n*-hexane and diethyl ether and dried to give a yellow solid product (21.0 g, yield: 66%).

Step 2: Synthesis of methyl 4-chloro-3-methoxy-5-nitrobenzoate

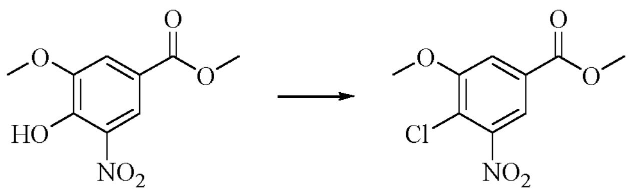

Methyl 4-hydroxy-3-methoxy-5-nitrobenzoate (21 g, 0.095 mol) was dissolved in DMF (200 mL), and the solution was cooled to -20 °C. Oxalyl chloride (23.7 mL) was slowly added dropwise, and the resulting solution was then heated to 80 °C and stirred for 3 h. The reaction solution was cooled to room temperature, poured into ice water and stirred overnight. Filtration was performed, and the filter cake was dissolved in dichloromethane, successively washed with saturated aqueous sodium bicarbonate solution and brine, dried with anhydrous sodium sulfate and concentrated. The crude product was dissolved in ethyl acetate, washed with brine, dried with anhydrous sodium sulfate and concentrated under reduced pressure, and the crude product was recrystallized with methanol to give the target compound (14.5 g, yield: 63.9%).

Step 3: Synthesis of methyl 3-methoxy-4-(methylamino)-5-nitrobenzoate

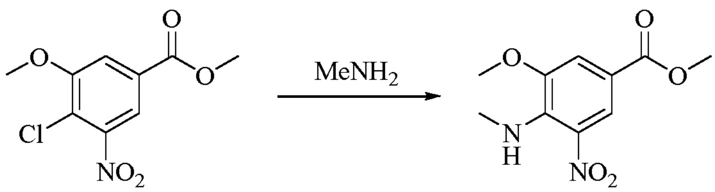


Methyl 4-chloro-3-methoxy-5-nitrobenzoate (14 g, 0.057 mol) was dissolved in DMF (140 mL), and the solution was cooled to 0 °C. A solution of methylamine (2 mol/L in tetrahydrofuran, 114 mL) was added dropwise in nitrogen atmosphere. The reaction solution was then heated to 80 °C, stirred for 3 h, cooled to room temperature, diluted with water and filtered to give an orange solid product (11.1 g, yield: 81%).
$^1$HNMR (400 MHz, DMSO-$d_6$) 6(ppm): 8.07 (d, *J* = 1.6 Hz, 1H), 7.80-7.76 (m, 1H), 7.38 (s, 1H), 3.91 (s, 3H), 3.83 (s, 3H), 2.94 (t, *J* = 5.6 Hz, 3H).

**Example 2: Synthesis of intermediate 1-ethyl-1*H*-pyrrolo[3,2-*b*]pyridine-2-carbaldehyde**

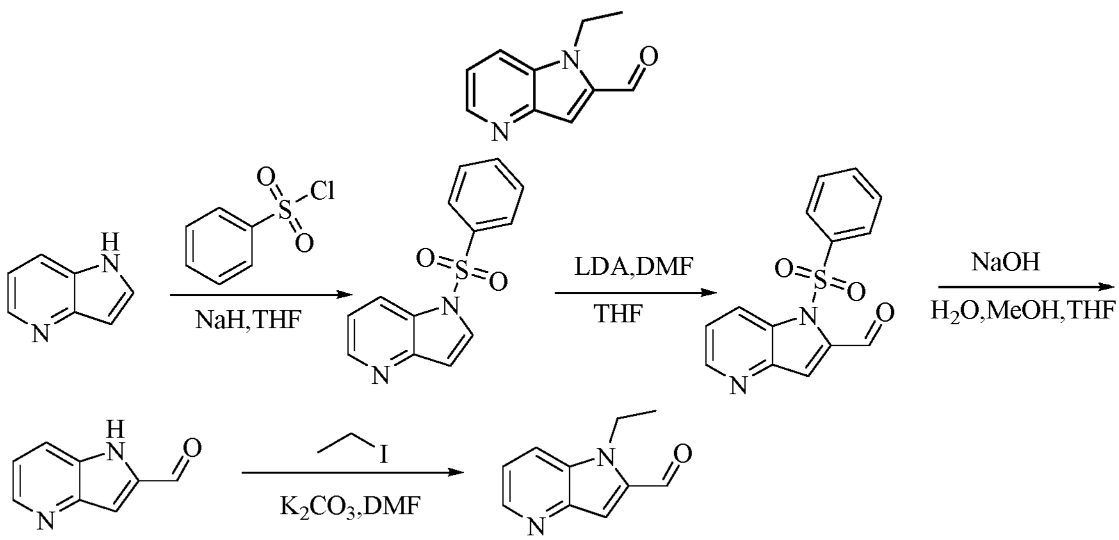

Step 1: Synthesis of 1-(phenylsulfonyl)-1*H*-pyrrolo[3,2-*b*]pyridine

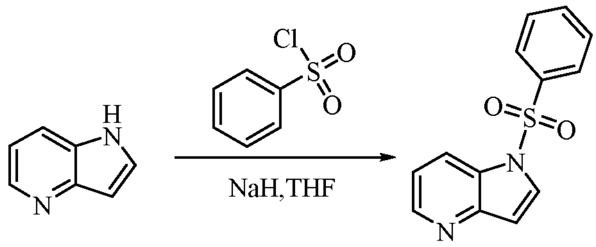


In nitrogen atmosphere, sodium hydride (mass fraction 60%, 0.8 g, 20 mmol, 2.0 eq.) was added into anhydrous THF (30 mL) in batches at room temperature. 1*H*-pyrrolo[3,2-*b*]pyridine (1.19 g, 10 mmol, 1.0 eq.) was dissolved in anhydrous THF (10 mL), and the solution was slowly added dropwise into the system. The mixture was then incubated at the temperature for 30 min. Benzenesulfonyl chloride (3.5 g, 20 mmol, 2.0 eq.) was dissolved in THF (10 mL), and the solution was slowly added dropwise into the system. 15 min later, addition was completed, and reaction was carried out for 4 h at room temperature. After the end of reaction, as detected by LC-MS, 0.1 mol/L aqueous HCl solution (20 mL) was slowly added dropwise to quench the reaction. Water (30 mL) was added, and extraction was performed with EA (50 mL × 2). The organic phases were combined, washed with brine, dried and concentrated, and the crude product was purified in a silica gel column chromatography to give an off-white solid product (1.5 g, yield: 57.7%).

Step 2: Synthesis of 1-(phenylsulfonyl)-1*H*-pyrrolo[3,2-*b*]pyridine-2-carbaldehyde

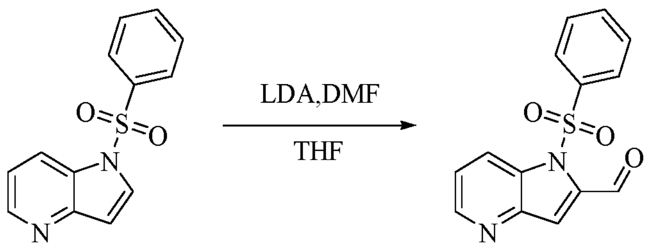


In nitrogen atmosphere, diisopropylamine (1.48 g, 14.5 mmol, 2.5 eq.) was added into anhydrous THF (15 mL), and the solution was cooled to -45 °C. *N*-butyllithium (2.4 mol/L, 6 mL, 14.5 mmol, 2.5 eq.) was added dropwise, and the mixture was then incubated at the temperature for 30 min and cooled to -78 °C. A solution of 1-(phenylsulfonyl)-1*H*-pyrrolo[3,2-*b*]pyridine (1.5 g, 5.8 mmol, 1.0 eq.) in THF (15 mL) was added dropwise, and the mixture was then incubated at the temperature for 1 h. DMF (0.85 g, 11.6 mmol, 2.0 eq.) was added dropwise, and the mixture was then incubated at the temperature for 30 min. Saturated aqueous ammonium chloride solution (20 mL) was added dropwise to quench the reaction, and extraction was performed with EA (50 mL × 3). The organic phases were combined, dried and concentrated, and the crude product was purified in a silica gel column chromatography to give an off-white solid product (1.2 g, yield: 72.3%).

Step 3: Synthesis of 1*H*-pyrrolo[3,2-*b*]pyridine-2-carbaldehyde

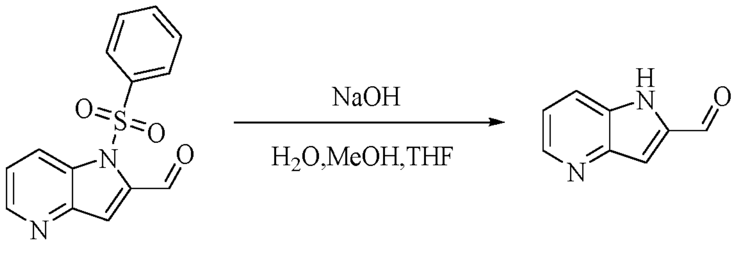

1-(phenylsulfonyl)-1*H*-pyrrolo[3,2-*b*]pyridine-2-carbaldehyde (1.2 g, 4.1 mmol, 1.0 eq.) was dissolved in anhydrous THF (15 mL) at room temperature, the solution was cooled to 0 °C, and a mixed solution (1:1 v/v, 30 mL) of sodium hydroxide (1.67 g, 41 mmol, 10 eq.) in methanol/water was added dropwise. The solution was then slowly warmed to room temperature and reacted for 1 h. After the end of reaction, as detected by LC-MS and TLC, saturated aqueous ammonium chloride solution (20 mL) was added dropwise to quench the reaction, and extraction was performed with DCM (50 mL × 2). The organic phases were combined, washed with brine, dried and concentrated, and the crude product was purified in a silica gel column chromatography to give a yellow solid product (300 mg, yield: 48.9%).

Step 4: Synthesis of 1-ethyl-1*H*-pyrrolo[3,2-*b*]pyridine-2-carbaldehyde

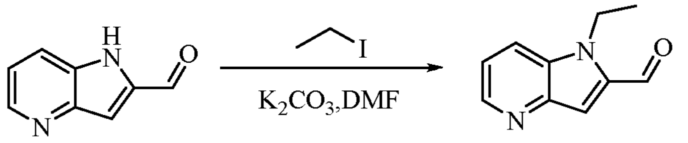


1*H*-pyrrolo[3,2-*b*]pyridine-2-carbaldehyde (294 mg, 2 mmol, 1.0 eq.) was dissolved in anhydrous DMF (5 mL) at room temperature, and potassium carbonate (552.8 mg, 4 mmol, 2.0 eq.) and iodoethane (375 mg, 2.4 mmol, 1.2 eq.) were added. The solution was heated to 40 °C to react for 2 h. After the end of reaction, as detected by LC-MS and TLC, the reaction solution was poured into ice water (10 mL), and extraction was performed with EA (20 mL × 2). The extract was washed with brine, dried and concentrated, and the crude product was purified in a silica gel column chromatography to give a white solid product (150 mg, yield: 42.8%).
$^1$HNMR (300 MHz, DMSO-$d_6$) δ(ppm): 10.01 (s, 1H), 8.54 (d, *J* = 4.2 Hz, 1H), 8.14 (d, *J* = 8.1 Hz, 1H), 7.58 (s, 1H), 7.42-7.37 (m, 1H), 4.58 (q, *J* = 7.2 Hz, 2H), 1.27 (t, *J* = 6.9 Hz, 3H).
LC-MS: 175.1. $[M+H]^+$

**Example 3: Synthesis of intermediate 5-ethyl-5*H*-pyrrolo[2,3-*b*]pyrazine - 6-carbaldehyde**

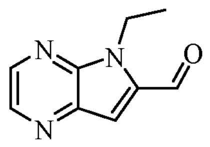

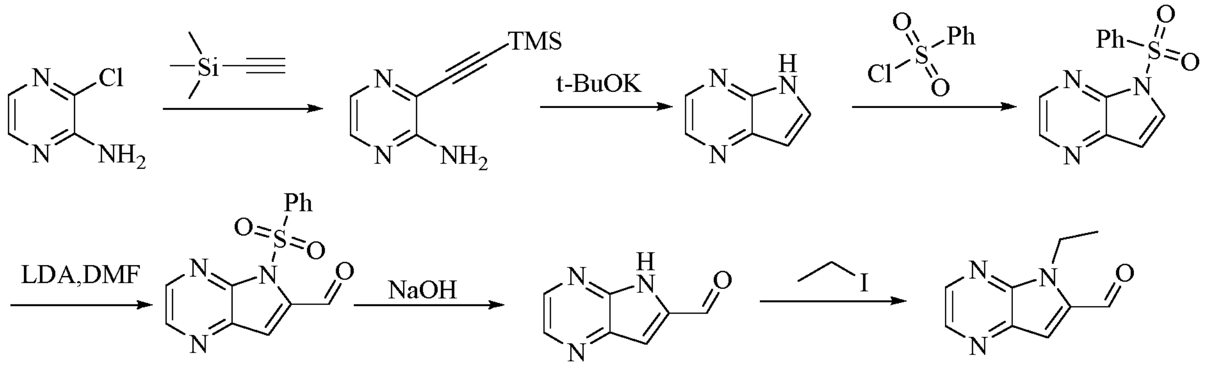


Step 1: Synthesis of 3-((trimethylsilyl)ethynyl)pyrazin-2-amine

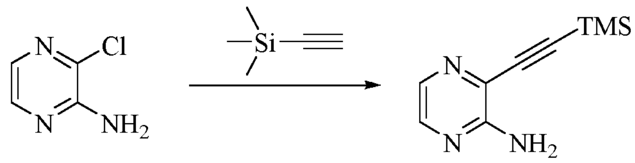

In nitrogen atmosphere, 3-chloropyrazin-2-amine (12.9 g, 0.1 mol, 1.0 eq.) was dissolved in DMF (150 mL). CuI (9.5 g, 0.05 mol, 0.5 eq.), tetrakis(triphenylphosphine)palladium (11.55 g, 0.01 mol, 0.1 eq.) and triethylamine (30.3 g, 0.3 mol, 3.0 eq.) were successively added, and the solution was cooled to 0 °C. Trimethylsilylacetylene (14.7 g, 0.15 mol, 1.5 eq.) was slowly added dropwise. The solution was then heated to 70 °C and stirred for 12 h. After the end of reaction, as detected by TLC and LC-MS, the reaction solution was poured into water (300 mL), added with EA (500 mL) and filtered under vacuum. The filter cake was washed with EA (50 mL × 2), and the filtrate was separated. The organic phase was washed with saturated brine, dried and filtered under vacuum, and the filtrate was concentrated. The crude product was purified in a silica gel column chromatography to give an off-white solid product (9.2 g, yield: 48%).

Step 2: Synthesis of 5*H*-pyrrolo[2,3-*b*]pyrazine

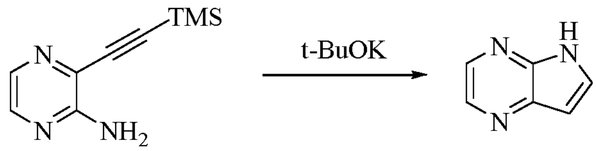


In nitrogen atmosphere, 3-((trimethylsilyl)ethynyl)pyrazin-2-amine (9.55 g, 0.05 mol, 1.0 eq.) was dissolved in THF (100 mL), and potassium *tert*-butoxide (11.2 g, 0.1 mol, 2.0 eq.) was added. The solution was heated to reflux and stirred for 8 h. After the end of reaction, as detected by TLC and LC-MS, the reaction solution was cooled to room temperature, added with water (100 mL) and extracted with EA (200 mL × 3). The organic phases were combined, dried and concentrated, and the crude product was purified in a silica gel column chromatography to give an off-white solid product (3.5 g, yield: 59%).

Step 3: Synthesis of 5-(phenylsulfonyl)-5*H*-pyrrolo[2,3-*b*]pyrazine

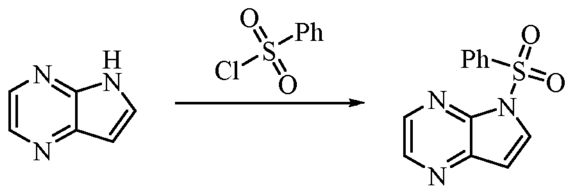

In nitrogen atmosphere, sodium hydride (mass fraction 60%, 0.8 g, 20 mmol, 2.0 eq.) was added into anhydrous THF (30 mL) in batches at room temperature. 5*H*-pyrrolo[2,3-*b*]pyrazine (1.19 g, 10 mmol, 1.0 eq.) was dissolved in anhydrous THF (10 mL), and the solution was slowly added dropwise into the system. Reaction was then carried out at room temperature for 30 min. Benzenesulfonyl chloride (3.5 g, 20 mmol, 2.0 eq.) was dissolved in THF (10 mL), and the solution was slowly added dropwise into the system. 20 min later, addition was completed, and reaction was carried out for 4 h at room temperature. After the end of reaction, as detected by LC-MS, 0.1 mol/L aqueous HCl solution (20 mL) was slowly added dropwise to quench the reaction. Water (30 mL) was added, and extraction was performed with EA (50 mL × 2). The organic phases were combined, washed with brine, dried and concentrated, and the crude product was purified in a silica gel column chromatography to give an off-white solid product (1.5 g, yield: 58%).

Step 4: Synthesis of 5-(phenylsulfonyl)-5*H*-pyrrolo[2,3-*b*]pyrazine-6-carbaldehyde

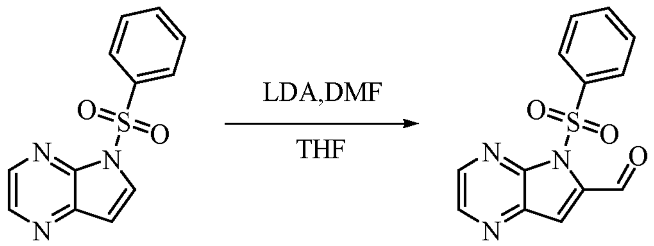


In nitrogen atmosphere, diisopropylamine (1.48 g, 14.5 mmol, 2.5 eq.) was added into anhydrous THF (15 mL), and the solution was cooled to -45 °C. *N*-butyllithium (2.4 mol/L, 6 mL, 14.5 mmol, 2.5 eq.) was added dropwise, and the mixture was incubated at the temperature for 30 min and then cooled to -78 °C. A solution of 5-(phenylsulfonyl)-5*H*-pyrrolo[2,3-*b*]pyrazine (1.5 g, 5.8 mmol, 1.0 eq.) in THF (15 mL) was added dropwise, and the mixture was then incubated at the temperature for 1 h. DMF (0.85 g, 11.6 mmol, 2.0 eq.) was added dropwise, and the mixture was then incubated at the temperature for 30 min. Saturated aqueous ammonium chloride solution (20 mL) was added dropwise to quench the reaction, and extraction was performed with EA (50 mL × 3). The organic phases were combined, dried and concentrated, and the crude product was purified in a silica gel column chromatography to give an off-white solid product (1.2 g, yield: 73%).

Step 5: Synthesis of 5*H*-pyrrolo[2,3-*b*]pyrazine-6-carbaldehyde

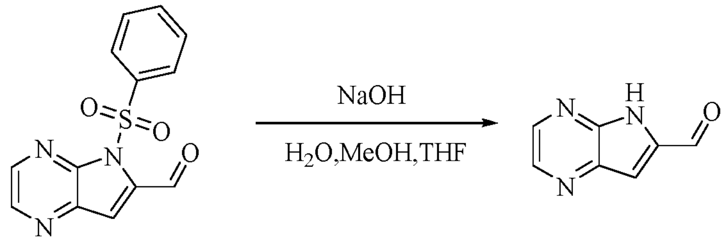


5-(phenylsulfonyl)-5*H*-pyrrolo[2,3-*b*]pyrazine-6-carbaldehyde (1.2 g, 4.1 mmol, 1.0 eq.) was dissolved in anhydrous THF (15 mL) at room temperature, the solution was cooled to 0 °C, and a mixed solution (1:1 v/v, 30 mL) of sodium hydroxide (1.67 g, 41 mmol, 10 eq.) in methanol/water was added dropwise. The resulting solution was then slowly warmed to room temperature and reacted for 1 h. After the end of reaction, as detected by LC-MS and TLC, saturated aqueous ammonium chloride solution (20 mL) was added dropwise to quench the reaction, and extraction was performed with DCM (50 mL × 2). The organic phases were combined, washed with brine, dried and concentrated, and the crude product was purified in a silica gel column chromatography to give a yellow solid product (300 mg, yield: 49%).

Step 6: Synthesis of 5-ethyl-5*H*-pyrrolo[2,3-*b*]pyrazine-6-carbaldehyde

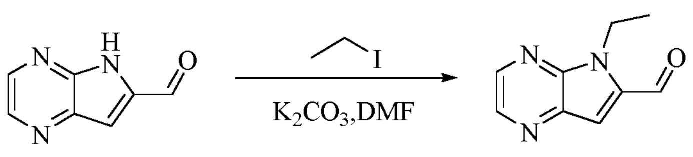


5*H*-pyrrolo[2,3-*b*]pyrazine-6-carbaldehyde (294 mg, 2 mmol, 1.0 eq.) was dissolved in anhydrous DMF (5 mL) at room temperature, and potassium carbonate (552.8 mg, 4 mmol, 2.0 eq.) and iodoethane (375 mg, 2.4 mmol, 1.2 eq.) were added. The solution was heated to 40 °C to react for 2 h. After the end of reaction, as detected by LC-MS and TLC, the reaction solution was poured into ice water (10 mL), and extraction was performed with EA (20 mL × 2). The extract was washed with brine, dried and concentrated, and the crude product was purified in a silica gel column chromatography to give a white solid product (150 mg, yield: 43%).
$^1$HNMR (300 MHz, DMSO-$d_6$) δ(ppm): 10.00 (s, 1H), 8.53 (d, *J* = 1.2 Hz, 1H), 8.41 (s, 1H), 7.37 (s, 1H), 6.25(d, *J* = 2.7 Hz, 1H), 4.69 (q, *J* = 7.2 Hz, 2H), 1.35 (t, *J* = 6.9 Hz, 3H).

LC-MS: 176.1 $[M+H]^+$.

**Example 4: Synthesis of intermediate 1-ethyl-1*H*-pyrrolo[2,3-*b*]pyridine- 2-carbaldehyde**

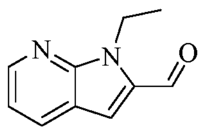

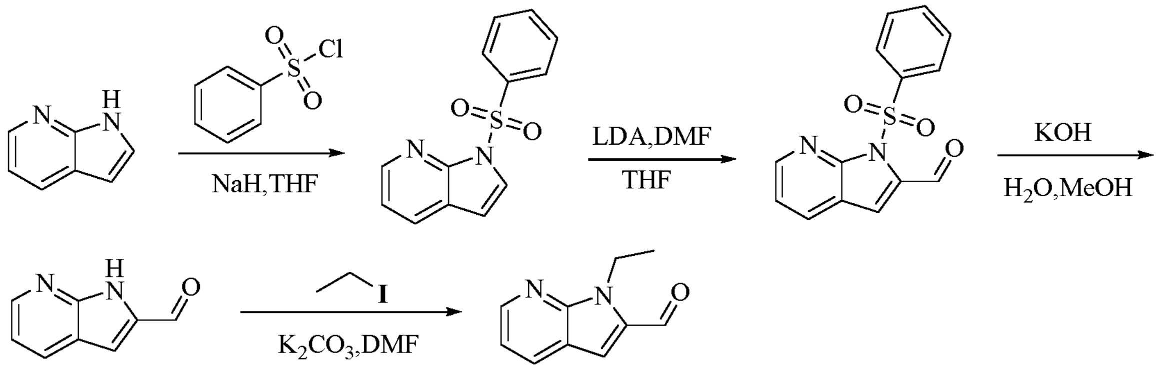


Step 1: Synthesis of 1-(phenylsulfonyl)-1*H*-pyrrolo[2,3-*b*]pyridine

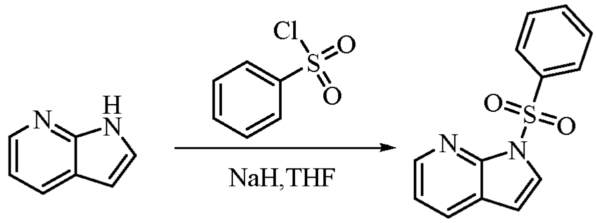


1*H*-pyrrolo[2,3-*b*]pyridine (10 g, 85 mmol, 1.0 eq.) was dissolved in anhydrous THF (150 mL), and the solution was added with sodium hydride (mass fraction 60%, 5 g, 125 mmol, 1.5 eq.) in batches under an ice bath to react for 30 min. Benzenesulfonyl chloride was slowly added dropwise, and then the solution reacted at room temperature for 2 h. After the end of reaction, as detected by TLC, the reaction solution was added with water (100 mL) and extracted with EA. The organic phases were combined, washed with brine, dried and concentrated, and the crude product was purified in a silica gel column chromatography to give the target compound (14 g, yield: 65%).

Step 2: Synthesis of 1-(phenylsulfonyl)-1*H*-pyrrolo[2,3-*b*]pyridine-2-carbaldehyde

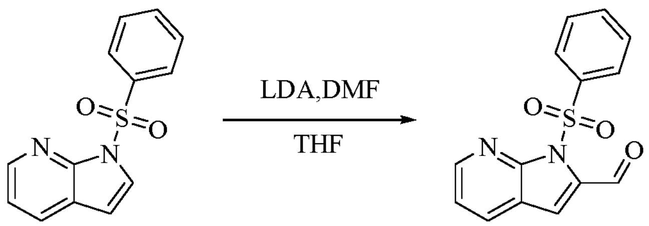

In nitrogen atmosphere, diisopropylamine (4.13 mL, 0.029 mol) was added into anhydrous THF (50 mL), and the solution was cooled to -78 °C. *N*-butyllithium (2.4 mol/L, 10.42 mL, 0.026 mol) was added dropwise, and the solution reacted at room temperature for 1 h. 1-(phenylsulfbnyl)-1*H*-pyrrolo [2,3-*b*]pyridine (5 g, 0.019 mol) and TMEDA (4.38 mL) were dissolved in anhydrous THF (50 mL), and the solution was cooled to -30 °C. The LDA solution prepared above was added dropwise in nitrogen atmosphere, and reaction was carried out for 2.5 h. DMF (3 mL) was slowly added dropwise, and reaction was carried out for 2 h. After the end of reaction, as detected by TLC and LC-MS, saturated aqueous ammonium chloride solution (100 mL) and EA (100 mL) were added, and liquid was separated. The organic phase was dried with anhydrous sodium sulfate and concentrated. The crude product was purified in a silica gel column chromatography to give a yellow solid product (4 g, yield: 72%).

Step 3: Synthesis of 1*H*-pyrrolo[2,3-*b*]pyridine-2-carbaldehyde

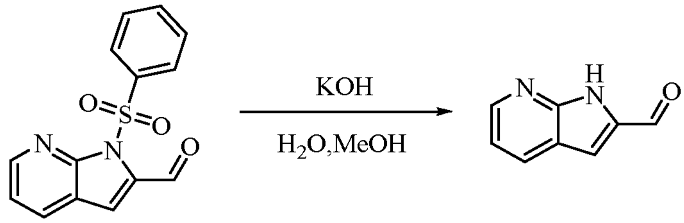


1-(phenylsulfonyl)-1*H*-pyrrolo[2,3-*b*]pyridine-2-carbaldehyde (2.5 g, 8.73 mmol) was dissolved in methanol (50 mL), a solution (5 mL) of potassium hydroxide (1.46 g, 26 mmol) in water was added dropwise in nitrogen atmosphere, and the resulting solution was stirred for 0.5 h. After the end of reaction, as detected by TLC, the reaction solution was added with water (150 mL) and extracted with DCM. The organic phases were combined, washed with brine, dried with anhydrous sodium sulfate and concentrated under reduced pressure, and the crude product was purified in a silica gel column chromatography to give a yellow solid product (1.0 g, yield: 55%).

Step 4: Synthesis of 1-ethyl-1*H*-pyrrolo[2,3-*b*]pyridine-2-carbaldehyde

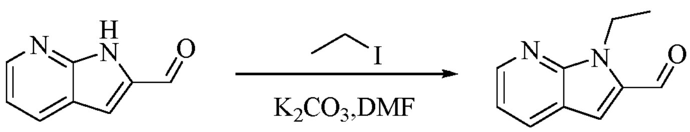


1*H*-pyrrolo[2,3-*b*]pyridine-2-carbaldehyde (1.0 g, 6.8 mmol) and potassium carbonate (2.8 g, 20 mmol) were dissolved in DMF (20 mL) at room temperature, and iodoethane (1.2 mL) was added dropwise at -20 °C in nitrogen atmosphere. The reaction solution reacted at room temperature for 1 h. After the end of reaction, as detected by TLC, the reaction solution was added with water (50 mL) and extracted with DCM (50 mL × 3). The organic phases were combined, washed with brine, dried with anhydrous sodium sulfate and concentrated, and the crude product was purified in a silica gel column chromatography to give a white solid product (380 mg, yield: 32%).
$^1$HNMR (400 MHz, DMSO-$d_6$) δ(ppm): 9.96 (s, 1H), 8.54 (dd, *J* 1= 4.8 Hz, *J* 2= 1.6 Hz, 1H), 8.24 (dd, *J* 1= 8.0 Hz, *J*2= 1.6 Hz, 1H), 7.49 (s, 1H), 7.26-7.23 (m, 1H), 4.64 (q, *J* = 7.2 Hz, 2H), 1.28 (t, *J* = 7.2 Hz, 3H).

**Example 5: Synthesis of intermediate 6-ethyl-6*H*-furo[2,3-*b*]pyrrole-5-carbaldehyde**

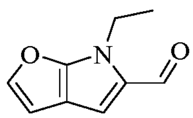

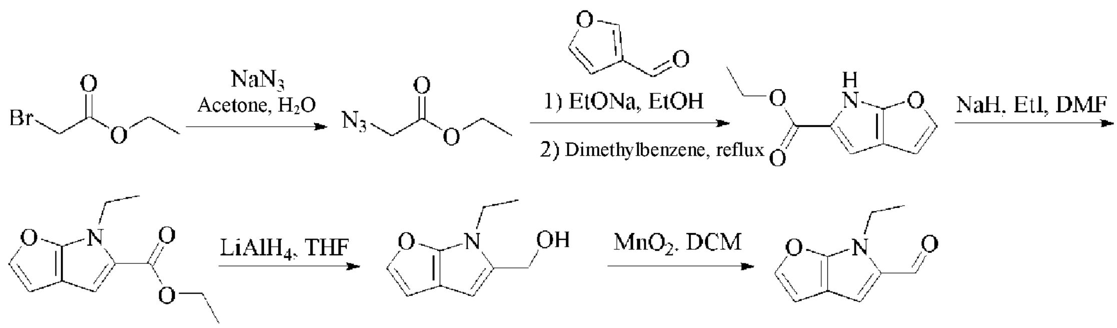


Step 1: Synthesis of ethyl 2-azidoacetate

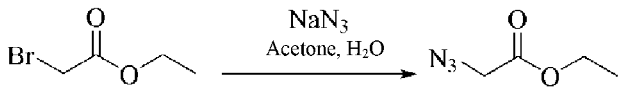


Ethyl 2-bromoacetate (200 g, 1.2 mol, 1.0 eq.) was added into acetone (1250 mL), the solution was cooled to 0 °C, and a solution of sodium azide (194.5 g, 3.0 mol, 2.5 eq.) in water (1000 mL) was added dropwise. The mixture was then heated to reflux for 1 h, cooled to room temperature and extracted with DCM (1000 mL × 3). The organic phases were combined, washed with saturated aqueous sodium bicarbonate solution (800 mL × 2) and water (800 mL × 2) in sequence, dried and concentrated under reduced pressure at a low temperature to give the target compound (145 g, yield: 93%), which was directly used in the next step.

Step 2: Synthesis of ethyl 6*H*-furo[2,3-*b*]pyrrole-5-carboxylate

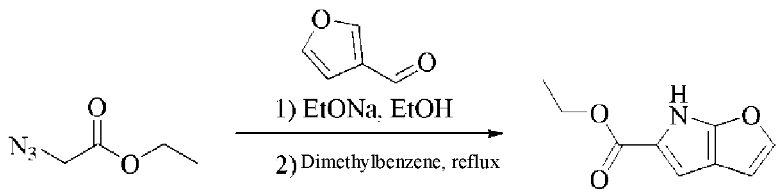


In nitrogen atmosphere, sodium turnings (7.64 g, 0.33 mol, 4.0 eq.) were added into absolute ethanol (400 mL) and stirred until the end of reaction. The reaction solution was cooled to 0 °C, and a solution of ethyl 2-azidoacetate (43 g, 0.33 mol, 4.0 eq.) and furan-3-carbaldehyde (8 g, 0.083 mol, 1.0 eq.) in ethanol (40 mL) was added dropwise. The mixture reacted at 0 °C for 1 h, and then reacted at room temperature for 1 h. After the end of reaction, as detected by TLC, saturated aqueous ammonium chloride solution (200 mL) was added dropwise to quench the reaction, and extraction was carried out with methyl *tert*-butyl ether (300 mL × 3). The organic phases were combined, washed with saturated brine (300 mL × 3), dried and concentrated to give a crude product. The obtained crude product was added into dimethylbenzene (100 mL), and the solution was heated to reflux and stirred for 4 h. After the end of reaction, as detected by TLC and LC-MS, the reaction solution was concentrated. The crude product was purified in a silica gel column chromatography to give a yellow solid product (5.14 g, yield: 33%).

Step 3: Synthesis of ethyl 6-ethyl-6*H*-furo[2,3-*b*]pyrrole-5-carboxylate

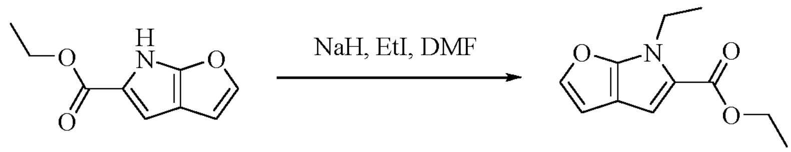


In nitrogen atmosphere, sodium hydride (mass fraction 60%, 1.5 g, 60.2 mmol, 2.0 eq.) was added into DMF(126 mL), and ethyl 6*H*-furo[2,3-*b*]pyrrole -5-carboxylate (5.4 g, 30.1 mmol, 1.0 eq.) was then added in batches. Stirring was then performed at room temperature for 20 min, and iodoethane (14.1 g, 90.3 mmol, 3.0 eq.) was added dropwise. Stirring was then performed at room temperature for 30 min. After the end of reaction, as detected by TLC, the reaction solution was cooled to 0 °C, saturated aqueous ammonium chloride solution (100 mL) was added dropwise to quench the reaction, and extraction was carried out with methyl *tert-butyl* ether (100 mL × 3). The organic phases were combined, washed with saturated brine (100 mL × 3), dried and concentrated to give a yellow oily product (5.4 g of crude product), which was directly used in the next step without purification.

Step 4: Synthesis of (6-ethyl-6*H*-furo[2,3-*b*]pyrrol-5-yl)methanol

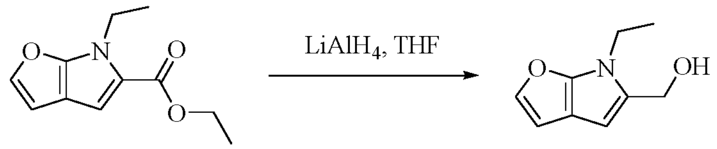


In nitrogen atmosphere, lithium aluminum hydride (0.83 g, 21.9 mmol, 1.5 eq.) was added into THF (30 mL), a solution of ethyl 6-ethyl-6*H*-furo[2,3-*b*]pyrrole-5-carboxylate (3.01 g, 14.5 mmol, 1.0 eq.) in THF (30 mL) was then added dropwise at 0 °C, and the mixture was then stirred at 0 °C for 2 h. After the end of reaction, as detected by TLC, water (1.04 mL), 15% w/w aqueous sodium hydroxide solution (1.04 mL) and water (3.12 mL) were added dropwise at 0 °C, anhydrous sodium sulfate was added, and stirring was performed for 15 min. After filtration under vacuum, the filtrate was concentrated to give the target compound (2.4 g of crude product), which was directly used in the next step.

Step 5: Synthesis 6-ethyl-6*H*-furo[2,3-*b*]pyrrole-5-carbaldehyde

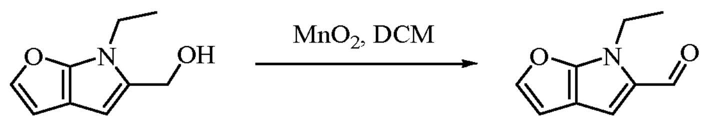


(6-ethyl-6*H*-furo[2,3-*b*]pyrrol-5-yl)methanol (2.4 g of crude product) was dissolved in DCM (120 mL), manganese dioxide (12.63 g, 10 eq.) was added, and the solution was heated to reflux for 1 h. After the end of reaction, as detected by HPLC, celite was added, and filtration under vacuum was carried out. The filter cake was washed with DCM (20 mL × 3), and the filtrate was concentrated to give the target compound (1.2 g).
$^1$HNMR (300 MHz, $CDCl_3$) δ(ppm): 9.48 (s, 1H), 7.34 (s, 1H), 6.79 (s, 1H), 6.56 (s, 1H), 4.51 (q, *J* = 7.2 Hz, 2H), 1.47 (t, *J* = 7.2 Hz, 3H).
LC-MS: 164.1 $[M+H]^+$.

**Example 6: Synthesis of intermediate 6-ethyl-6*H*-thieno[2,3-*b*]pyrrole-5-carbaldehyde**

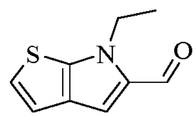

Steps:

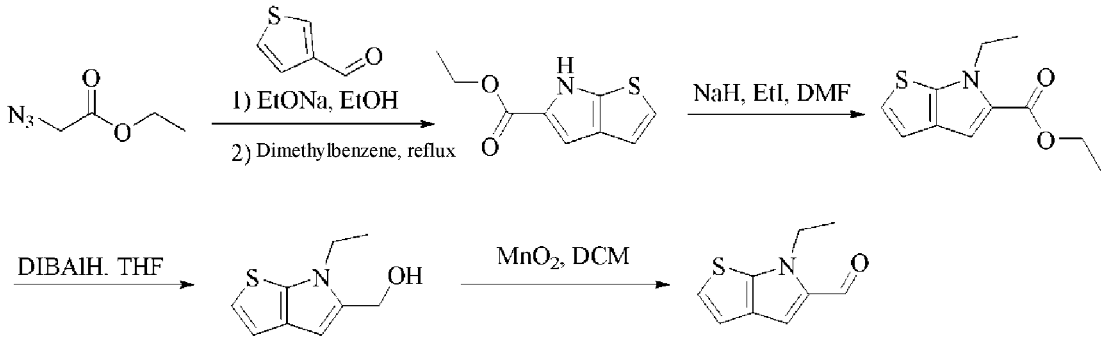


Step 1: Synthesis of ethyl 6*H*-thieno[2,3-*b*]pyrrole-5-carboxylate

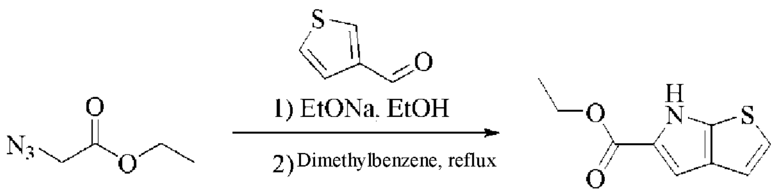


In nitrogen atmosphere, sodium turnings (7.64 g, 0.33 mol, 4.0 eq.) were added into absolute ethanol (400 mL) and stirred until the end of reaction. The reaction solution was then cooled to 0 °C, and a solution of ethyl 2-azidoacetate (43 g, 0.33 mol, 4.6 eq.) and thiophene-3- carbaldehyde (8 g, 0.071 mol, 1.0 eq.) in ethanol (40 mL) was added dropwise. The mixture reacted at 0 °C for 1 h, and then reacted at room temperature for 1 h. After the end of reaction, as detected by TLC, saturated aqueous ammonium chloride solution (200 mL) was added dropwise to quench the reaction, and extraction was carried out with methyl *tert-butyl* ether (300 mL × 3). The organic phases were combined, washed with saturated brine (300 mL × 3), dried and concentrated to give a crude product. The obtained crude product was added into dimethylbenzene (100 mL), and the solution was heated to reflux and stirred for 4 h. After the end of reaction, as detected by TLC and LC-MS, the reaction solution was concentrated. The crude product was purified in a silica gel column chromatography to give a yellow solid product (2.0 g, yield: 15%).

Step 2: Synthesis of ethyl 6-ethyl-6*H*-thieno[2,3-*b*]pyrrole-5-carboxylate

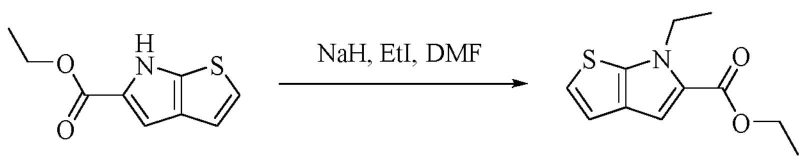


In nitrogen atmosphere, sodium hydride (mass fraction 60%, 0.83 g, 20.6 mmol, 2.0 eq.) was added into DMF (47 mL), and ethyl 6*H*-thieno[2,3-*b*] pyrrole-5-carboxylate (2.0 g, 10.3 mmol, 1.0 eq.) was then added in batches. Stirring was then performed at room temperature for 20 min, and iodoethane (4.8 g, 30.9 mmol, 3.0 eq.) was added dropwise. Stirring was then performed at room temperature for 30 min. After the end of reaction, as detected by TLC, the reaction solution was cooled to 0 °C, saturated aqueous ammonium chloride solution (50 mL) was added dropwise to quench the reaction, and extraction was carried out with methyl *tert-butyl* ether (50 mL × 3). The organic phases were combined, washed with saturated brine (50 mL × 3), dried and concentrated to give a yellow oily product (2.0 g of crude product), which was directly used in the next step without purification.

Step 3: Synthesis of (6-ethyl-6*H*-thieno [2,3-*b*]pyrrol-5-yl)methanol

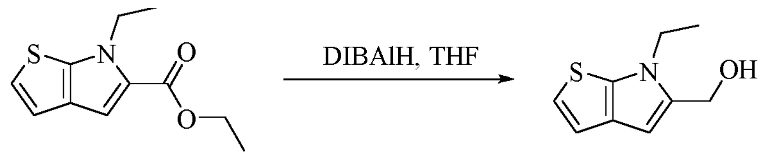


In nitrogen atmosphere, ethyl 6-ethyl-6*H*-thieno[2,3-*b*]pyrrole-5-carboxylate (2.0 g, 8.96 mmol, 1.0 eq.) was added into THF (20 mL), and the solution was cooled to 0 °C. Diisobutyl aluminum hydride (2M in toluene, 16.2 mL, 22.4 mmol, 2.5 eq.) was added dropwise. The mixture was then stirred at 0 °C for 2 h. After the end of reaction, as detected by TLC, water (1 mL), 15% w/w aqueous sodium hydroxide solution (1 mL) and water (2.4 mL) were successively added dropwise at 0 °C. Anhydrous sodium sulfate was then added, and stirring was performed for 15 min. After filtration under vacuum, the solids were washed with ethyl acetate, and the filtrate was concentrated to give a crude product (1.68 g), which was directly used in the next step.

Step 4: Synthesis of 6-ethyl-6*H*-thieno[2,3-*b*]pyrrole-5-carbaldehyde

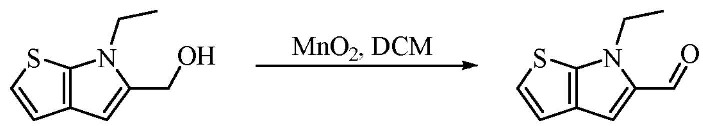


(6-ethyl-6*H*-thieno[2,3-*b*]pyrrol-5-yl)methanol (1.68 g of crude product) was dissolved in DCM (84 mL), manganese dioxide (8.06 g, 10 eq.) was added, and the solution was heated to reflux for 1 h. After the end of reaction, as detected by HPLC, celite was added, and filtration under vacuum was carried out. The filter cake was washed with DCM (20 mL × 3), and the filtrate was concentrated to give the target compound (1.2 g).
$^1$HNMR (300 MHz, $CDCl_3$) δ(ppm): 9.56 (s, 1H), 7.04 (d, *J* = 7.2 Hz, 1H), 6.96 (d, *J* = 7.2 Hz, 1H), 4.52 (q, *J* = 7.2 Hz, 2H), 1.46 (t, *J* = 7.2 Hz, 3H). LC-MS: 180.1 $[M+H]^+$.

**Example 7: Synthesis of intermediate 6-ethyl-2-methyl-6*H*-thieno[2,3-*b*] pyrrole-5-carbaldehyde**

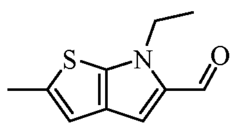

Step 1: Synthesis of 2-methylthiophene-4-carbaldehyde

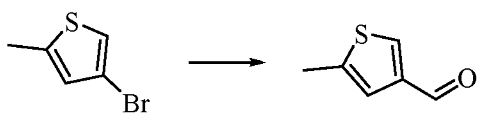

4-bromo-2-methylthiophene (25 g, 141.2 mmol, 1.0 eq.) was added to diethyl ether (170 mL). The mixture was cooled to -78 °C, and then added with *n*-butyllithium (2.5 mol/L in *n*-hexane, 84.72 mL, 211.8 mmol, 1.5 eq.) dropwise. The mixture was then incubated at the temperature for 1 h, and added with a solution of DMF (15.48 g, 211.8 mmol, 1.5 eq.) in diethyl ether (56 mL) dropwise. The resulting mixture was then incubated at the temperature for 1 h. After the end of reaction as detected by HPLC, saturated aqueous ammonium chloride (300 mL) was added dropwise at -78 °C to quench the reaction, and methyl *tert-butyl* ether (200 mL × 3) was added for extraction. The organic phase was

washed with saturated brine (300 mL × 2), dried with anhydrous sodium sulfate, filtered and concentrated to give a crude product (23.28 g), which was distilled under reduced pressure by an oil pump to give the target compound (10.9 g, yield: 61%).

Step 2: Synthesis of ethyl 2-methyl-6*H*-thieno[2,3-*b*]pyrrole-5-carboxylate

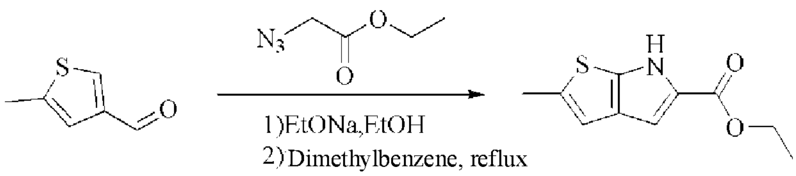


In nitrogen atmosphere, sodium turnings (5 g, 217.4 mmol, 5.5 eq.) were added to absolute ethanol (250 mL) and stirred until dissolved, and then ethyl 2-azidoacetate (20.47 g, 158.50 mmol, 4.0 eq.) and 2-methylthiophene-4-carbaldehyde (5 g, 39.63 mmol, 1.0 eq.) in ethanol (25 mL) was added dropwise. The mixture was stirred at 0 °C for 1 h, and then stirred at room temperature for 1 h. After the end of reaction as detected by TLC, saturated aqueous ammonium chloride solution (300 mL) was added dropwise to quench the reaction, and methyl *tert-butyl* ether (300 mL × 3) was added for extraction. The organic phases were combined, washed with saturated brine (300 mL × 3), dried with anhydrous sodium sulfate, filtered and concentrated under reduced pressure to give a crude product. The crude product was added to methyl *tert-butyl* ether (30 mL), washed with saturated brine (30 mL × 3), dried and concentrated under reduced pressure. The resulting intermediate was added to dimethylbenzene (65 mL) and the mixture was heated to reflux for 2 h. After the end of reaction as detected by TLC and LC-MS, the solution was concentrated to give a crude product, which was then purified in a silica gel column chromatography to give the target compound (4.1 g, yield: 49%).

Step 3: Synthesis of ethyl 6-ethyl-2-methyl-6*H*-thieno[2,3-*b*]pyrrole-5-carboxylate

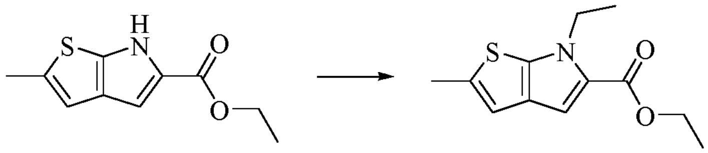

In nitrogen atmosphere, sodium hydride (0.94 g, 39.19 mmol, 2.0 eq.) was added to DMF (96 mL), and ethyl 2-methyl-6*H*-thieno[2,3-*b*]pyrrole- 5-carboxylate (4.1 g, 19.59 mmol, 1.0 eq.) was added in batches. The mixture was then stirred at room temperature for 20 min, and added with iodoethane (9.17 g, 58.78 mmol, 3.0 eq.) dropwise. The resulting mixture was then stirred at room temperature for 30 min. After the end of reaction, as detected by TLC, the reaction solution was cooled to 0 °C, then saturated aqueous ammonium chloride solution (50 mL) was added dropwise to quench the reaction, and methyl *tert-butyl* ether (50 mL × 3) was added for extraction. The organic phases were combined, washed with saturated brine (100 mL × 2), dried with anhydrous sodium sulfate and filtered, and the filtrate was concentrated under reduced pressure to give the target compound (4.25 g of crude product).

Step 4: Synthesis of (6-ethyl-2-methyl-6*H*-thieno[2,3-*b*]pyrrol-5-yl)methanol

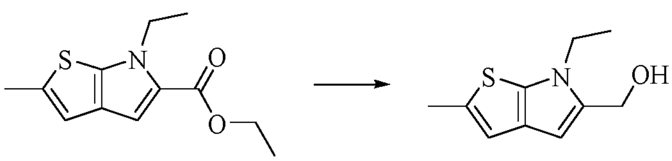

In nitrogen atmosphere, ethyl 6-ethyl-2-methyl-6*H*-thieno[2,3-*b*]pyrrole-5-carboxylate (4.25 g, 17.91 mmol, 1.0 eq.) was added to THF (45 mL). The mixture was cooled to 0 °C, and then added with diisobutyl aluminum hydride (1.5

mol/L in toluene, 29.85 mL, 44.77 mmol, 2.5 eq.) dropwise. The resulting mixture was then stirred at 0 °C for 2 h. After the end of reaction, as detected by TLC, water (1.8 mL), 15% w/w aqueous sodium hydroxide solution (1.8 mL), and water (4.5 mL) were successively added dropwise at 0 °C. Anhydrous sodium sulfate was then added. The resulting mixture was stirred for 15 min and filtered under vacuum. The filter cake was washed with ethyl acetate, and the filtrate was concentrated to give a crude product (3.25 g), which was slurried with PE (30 mL) and filtered under vacuum to give the target compound (2.3 g, yield: 65.9 %).

Step 5: Synthesis of 6-ethyl-2-methyl-6*H*-thieno[2,3-*b*]pyrrole-5-carbaldehyde

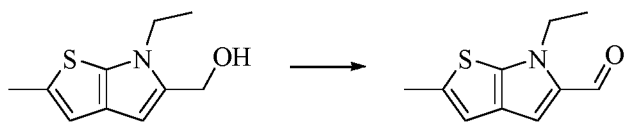

(6-ethyl-2-methyl-6*H*-thieno[2,3-*b*]pyrrol-5-yl)methanol (2.3 g, 11.78 mmol, 1.0 eq.) was dissolved in DCM (115 mL), manganese dioxide (10.24 g, 117.78 mmol, 10 eq.) was added, and the mixture was heated to reflux for 2 h. After the end of reaction, as detected by HPLC, celite was added, and the mixture was filtered under vacuum. The solids were washed with DCM (50 mL × 3), and the filtrate was concentrated under reduced pressure to give the target compound (1.95 g, yield: 85%).
$^1$H NMR (400 MHz, $CDCl_3$) δ(ppm): 9.49 (s, 1H), 6.92 (s, 1H), 6.67 (d, *J* = 1.2 Hz, 1H), 4.49 (q, *J* = 7.2 Hz, 2H), 2.52 (d, *J* = 1.2 Hz, 3H), 1.42 (t, *J* = 7.2 Hz, 3H).
Molecular formula: $C_{10}H_{11}NOS$, molecular weight: 193.26, LC-MS (Pos, *m*/*z*) = 194.1 $[M+H]^+$

**Example 8: Synthesis of intermediate 6-ethyl-3-methyl-6*H*-thieno[2,3-*b*] pyrrole-5-carbaldehyde**

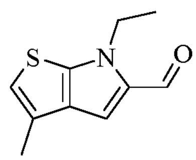

Step 1: Synthesis of 4-methylthiophene-3-carbaldehyde

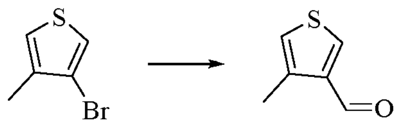

3-bromo-4-methylthiophene (30 g, 169.43 mmol, 1.0 eq.) was added to diethyl ether (200 mL). The mixture was cooled to -78 °C, and then added with *n*-butyllithium (2.5 mol/L in *n*-hexane, 102 mL, 254.15 mmol, 1.5 eq.) dropwise. The mixture was then incubated at the temperature for 1 h, and added with a solution of DMF (18.58 g, 254.15 mmol, 1.5 eq.) in diethyl ether (67 mL) dropwise. The resulting mixture was then incubated at the temperature for 1 h. After the end of reaction, as detected by HPLC, saturated aqueous ammonium chloride solution (400 mL) was added dropwise at -78 °C to quench the reaction, and methyl *tert-butyl* ether (300 mL × 3) was added for extraction. The organic phases were combined, washed with saturated brine (300 mL × 2), dried with anhydrous sodium sulfate and concentrated under reduced pressure to give a crude product (28.5 g), which was distilled under reduced pressure by an oil pump to give the target compound (9.4 g, yield: 44 %).

Step 2: Synthesis of ethyl 3-methyl-6*H*-thieno[2,3-*b*]pyrrole-5-carboxylate

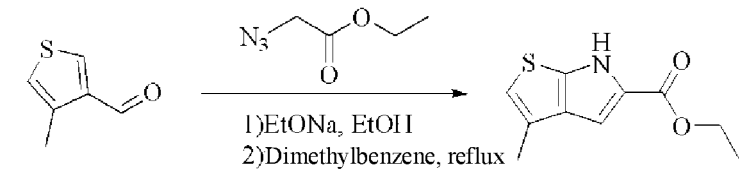


In nitrogen atmosphere, sodium turnings (6.86 g, 297.99 mmol, 4.0 eq.) were added to absolute ethanol (470 mL) and stirred until dissolved, and a solution of ethyl 2-azidoacetate (38.48 g, 297.99 mmol, 4.0 eq.) and 4-methylthiophene-3-carbaldehyde (9.4 g, 74.50 mmol, 1.0 eq.) in ethanol (47 mL) was added dropwise. The mixture was then stirred at 0 °C for 1 h, and stirred at room temperature for 1 h. After the end of reaction, as detected by TLC, saturated aqueous ammonium chloride solution (500 mL) was added dropwise to quench the reaction, and methyl *tert-butyl* ether (500 mL × 3) was added for extraction. The organic phases were combined, washed with saturated brine (500 mL × 3), dried with anhydrous sodium sulfate and concentrated under reduced pressure to give a crude product. The crude product was added to methyl *tert-butyl* ether (100 mL), washed with saturated brine (50 mL × 3), dried with anhydrous sodium sulfate and concentrated under reduced pressure. The resulting intermediate was added to dimethylbenzene (120 mL) and heated to reflux for 2 h. After the end of reaction, as detected by TLC and LC-MS, the reaction solution was concentrated to give a crude product, which was then purified in a silica gel column chromatography to give the target compound (7.7 g, yield: 49.3%).

Step 3: Synthesis of ethyl 6-ethyl-3-methyl-6*H*-thieno[2,3-*b*]pyrrole-5-carboxylate

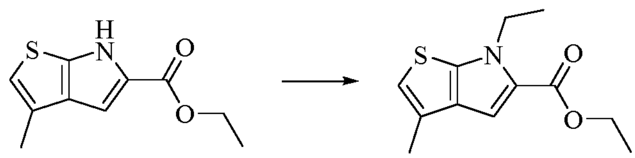

In nitrogen atmosphere, sodium hydride (1.77 g, 73.59 mmol, 2.0 eq.) was added to DMF (180 mL), and ethyl 3-methyl-6*H*-thieno[2,3-*b*]pyrrole -5-carboxylate (7.7 g, 36.80 mmol, 1.0 eq.) was then added in batches. The mixture was then stirred at room temperature for 20 min, and added with iodoethane (17.22 g, 110.39 mmol, 3.0 eq.) dropwise. The resulting mixture was then stirred at room temperature for 30 min. After the end of reaction, as detected by TLC, the reaction solution was cooled to 0 °C, then saturated aqueous ammonium chloride solution (50 mL) was added dropwise to quench the reaction, and methyl *tert-butyl* ether (200 mL × 3) was added for extraction. The organic phases were combined, washed with saturated brine (200 mL × 5), dried and concentrated under reduced pressure to give the target compound (9.8 g of crude product).

Step 4: Synthesis of (6-ethyl-3-methyl-6*H*-thieno[2,3-*b*]pyrrol-5-yl)methanol

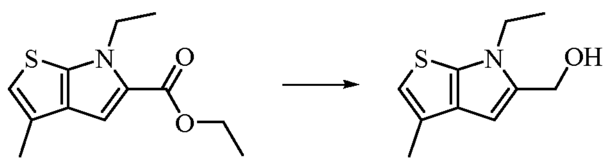

In nitrogen atmosphere, ethyl 6-ethyl-3-methyl-6*H*-thieno[2,3-*b*]pyrrole-5-carboxylate (9.8 g, 41.29 mmol, 1.0 eq.) was added to THF (100 mL). The mixture was cooled to 0 °C, and then added with diisobutyl aluminum hydride (1.5 mol/L in toluene, 69 mL, 103.24 mmol, 2.5 eq.) dropwise. The resulting mixture was then stirred at 0 °C for 2 h. After the end of reaction, as detected by TLC, water (4.2 mL), 15% w/w aqueous sodium hydroxide solution (4.2 mL) and water (10.3 mL) were successively added dropwise at 0 °C. Anhydrous sodium sulfate was then added. The resulting mixture was stirred for 15 min and filtered under vacuum. The solids were washed with ethyl acetate, and the filtrate was concentrated under reduced pressure to give a crude product (13.1 g), which was slurried with PE (30 mL) and filtered under vacuum to give the target compound (6.1 g, yield: 75.6%).

Step 5: Synthesis of 6-ethyl-3-methyl-6*H*-thieno[2,3-*b*]pyrrole-5-carbaldehyde

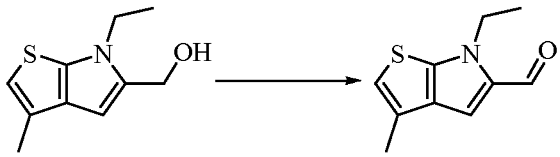

(6-ethyl-3-methyl-6*H*-thieno[2,3-*b*]pyrrole-5-yl)methanol (6.1 g, 31.24 mmol, 1.0 eq.) was dissolved in DCM (300 mL), manganese dioxide (27.16 g, 312.37 mmol, 10 eq.) was added, and the mixture was heated to reflux for 2 h. After the end of reaction, as detected by HPLC, celite was added, and the mixture was filtered under vacuum. The filter cake was washed with DCM (100 mL × 3), and the filtrate was concentrated under reduced pressure to give the target compound (4.5 g, yield: 74%).

$^1$HNMR (300 MHz, $CDCl_3$) δ(ppm): δ 9.52 (s, 1H), 7.00 (s, 1H), 6.52 (s, 1H), 4.49 (q, *J* = 7.2 Hz, 2H), 2.32 (s, 3H), 1.45 (t, *J* = 7.2 Hz, 3H).

Molecular formula: $C_{10}H_{11}NOS$, molecular weight: 193.26, LC-MS (Pos, *m*/*z*) = 194.1 $[M+H]^+$

**Example 9: Synthesis of (*R*)-(3-aminopiperidin-1-yl)(2-(5-ethyl-5*H*-pyrrolo[2,3-*b*]pyrazin-6-yl)-7-methoxy-1-methyl-1*H*-benzo[*d*]imidazol-5-yl)methanone (Compound 1)**

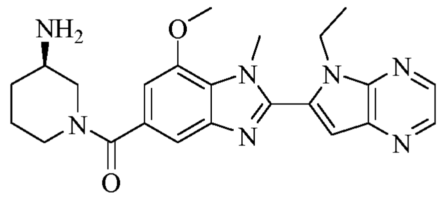

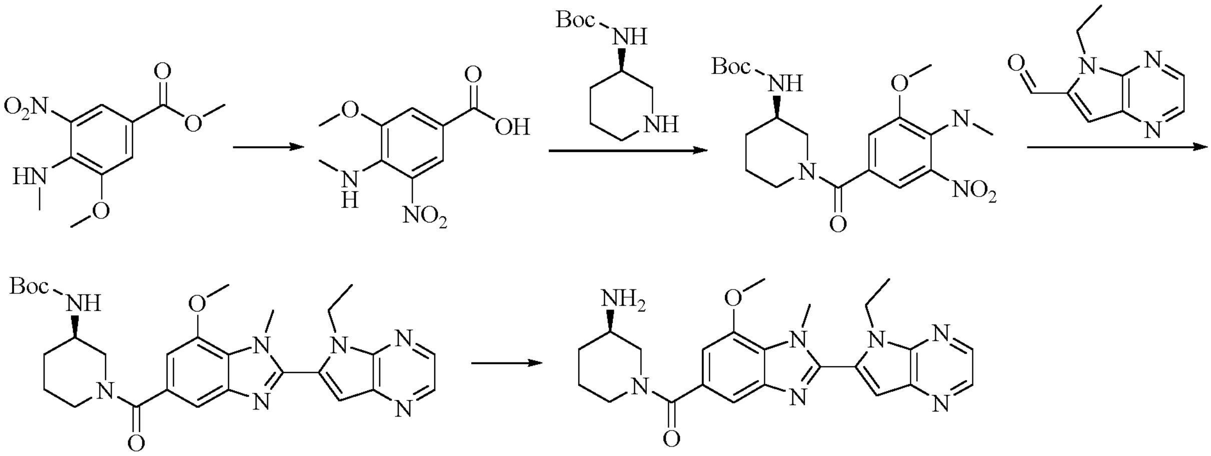

Step 1: Synthesis of 3-methoxy-4-(methylamino)-5-nitrobenzoic acid

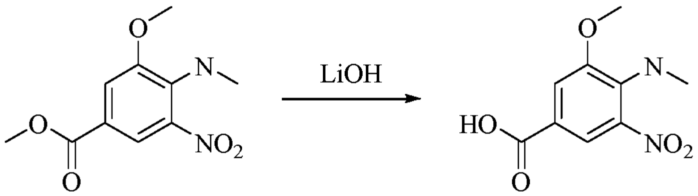


Methyl 3-methoxy-4-(methylamino)-5-nitrobenzoate (1.0 g, 4.16 mmol, 1.0 eq.) was dissolved in a mixed solution of methanol (5 mL) and tetrahydrofuran (5 mL), and then a solution of lithium hydroxide monohydrate (500 mg, 11.9 mmol, 2.9 eq.) in water (5 mL) was added. The resulting solution was stirred at 50 °C for 2 h. After the end of reaction, as detected by TLC, the reaction solution was concentrated under reduced pressure to remove most of the organic solvent, and the pH was adjusted to 5 with hydrochloric acid (1 mol/L). The resulting solution was filtered to give a brownish-yellow solid 3-methoxy-4-(methylamino)-5-nitrobenzoic acid (600.0 mg, yield: 63.7%).

Step 2: Synthesis of *tert-butyl* (*R*)-(1-(3-methoxy-4-(methylamino)-5-nitrobenzoyl)piperidin-3-yl)carbamate

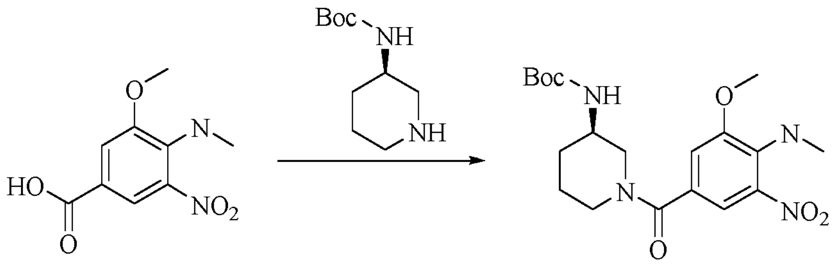

3-methoxy-4-(methylamino)-5-nitrobenzoic acid (500.0 mg, 2.21 mmol, 1.0 eq.) was dissolved in a mixed solvent of tetrahydrofuran (30 mL) and *N,N*-dimethylformamide (1 mL), and *N,N*-diisopropylethylamine (570.8 mg, 4.42 mmol, 2.0 eq.) and 2-(7-azabenzotriazol-1-yl)-*N,N,N',N'*-tetramethyluronium hexafluorophosphate (923.9 mg, 2.43 mmol, 1.1 eq.) were added under an ice bath. The mixture was stirred for 40 min under an ice bath, and then added with a solution of *tert*-butyl (*R*)-piperidin-3-yl carbamate (530.7 mg, 2.65 mmol, 1.2 eq.) in tetrahydrofuran (5 mL) dropwise. The resulting solution was then stirred at room temperature for 10 h. After the end of reaction, as detected by TLC, the solution was concentrated and added with water (50 mL) and ethyl acetate (100 mL) before liquid separation. The organic phase was dried with anhydrous sodium sulfate and concentrated to give a crude product, which was then purified in a silica gel column chromatography (200-300 mesh silica gel, dichloromethane:methanol = 40:1 v/v) to give the target compound (960.3 mg, yield: 100%).

Step 3: Synthesis of *tert*-butyl (*R*)-(1-(2-(5-ethyl-5*H*-pyrrolo[2,3-*b*] pyrazin-6-yl)-7-methoxy-1-methyl-1*H*-benzo[*d*]imidazole-5-carbonyl)piperidin-3-yl)carbamate

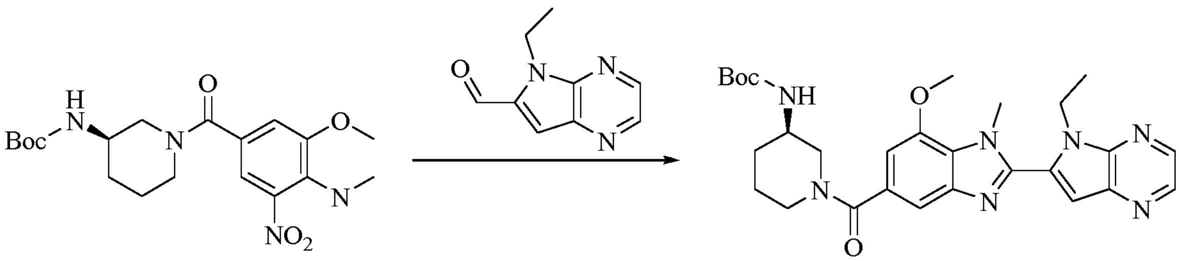

*Tert-butyl* (*R*)-(1-(3-methoxy-4-(methylamino)-5-nitrobenzoyl)piperidin-3-yl)carbamate (580.7 mg, 1.42 mmol, 1.0 eq.) was dissolved in ethanol (7 mL), and the solution was then added with 5-ethyl-5*H*-pyrrolo[2,3-*b*]pyrazine-6-carbaldehyde (250.0 mg, 1.42 mmol, 1.0 eq.), sodium dithionite (742.7 mg, 4.26 mmol, 3.0 eq.) and water (4 mL) to

react at 100 °C for 5 h under microwaves. After the end of reaction, as detected by TLC, the solution was concentrated under reduced pressure and added with water (30 mL) and dichloromethane (70 mL) before liquid separation. The organic phase was dried with anhydrous sodium sulfate and concentrated to give a crude product, which was then separated in a silica gel column chromatography (200-300 mesh silica gel, dichloromethane:methanol = 60:1 v/v) to give the target compound (513.7 mg, yield: 67.8%).

Step 4: Synthesis of (*R*)-(3-aminopiperidin-1-yl)(2-(5-ethyl-5*H*-pyrrolo [2,3-*b*]pyrazin-6-yl)-7-methoxy-1-methyl-1*H*-benzo[*d*]imidazol-5-yl)methanone

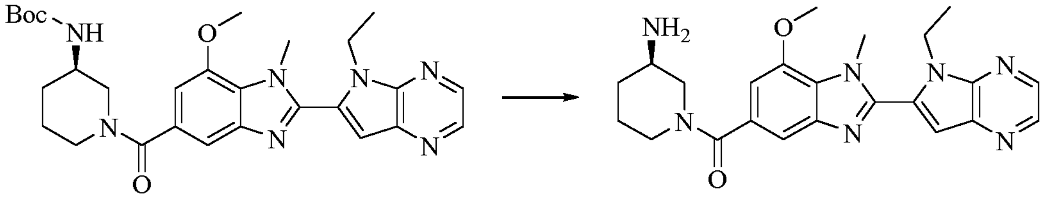

*Tert-butyl* (*R*)-(1-(2-(5-ethyl-5*H*-pyrrolo[2,3-*b*]pyrazin-6-yl)-7-methoxy-1-methyl-1*H*-benzo[*d*]imidazole-5-carbonyl)piperidin-3-yl)carbamate (513.7 mg, 0.96 mmol, 1.0 eq.) was dissolved in dichloromethane (4 mL), and trifluoroacetic acid (2 mL) was added dropwise. The mixture then reacted at room temperature for 2 h. After the end of reaction, as detected by TLC, saturated aqueous sodium bicarbonate solution (10 mL) was added under an ice bath to adjust the pH to 7-8, and dichloromethane (15 mL) was added for extraction. The organic phase was dried with anhydrous sodium sulfate to give a crude product, which was then purified in a preparative thin-layer chromatography to give (*R*)-(3-aminopiperidin-1-yl)(2-(5-ethyl-5*H*-pyrrolo [2,3-*b*]pyrazin-6-yl)-7-methoxy-1-methyl-1*H*-benzo[*d*]imidazol-5-yl)methanone (20.0 mg, yield: 4.8%).
$^1$HNMR (400MHz, DMSO-$d_6$) δ(ppm): 8.57 (d, *J* = 2.4 Hz, 1H), 8.45 (d, *J* = 2.4 Hz, 1H), 7.37 (s, 1H), 7.32 (s, 1H), 6.91 (s, 1H), 4.60-4.65 (m, 2H), 4.17 (s, 3H), 3.99 (s, 3H), 2.97-3.05 (m, 2H), 2.80-2.90 (m, 2H), 1.98-2.02 (m, 1H), 1.90-1.92 (m, 2H), 1.65-1.80 (m, 2H), 1.47-1.55 (m, 2H), 1.25-1.40 (m, 3H).
Molecular formula: $C_{23}H_{27}N_7O_2$, molecular weight: 433.52, LC-MS (Pos, *m/z*) = 434.2 [M+H]$^+$.

Example 10: **Synthesis of (*R*)-(3-aminopiperidin-1-yl)(2-(1-ethyl-1*H*-pyrrolo[3,2-*b*]pyridin-2-yl)-7-methoxy-1-methyl-1*H*-benzo[*d*]imidazol-5-yl)methanone (Compound 2)**

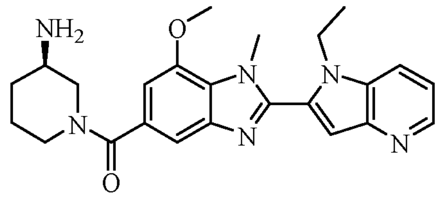

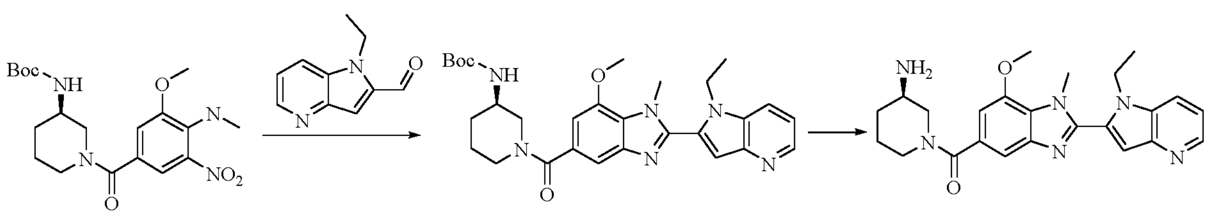

Step 1: Synthesis of *tert*-butyl (*R*)-(1-(2-(1-ethyl-1*H*-pyrrolo[3,2-*b*] pyridin-2-yl)-7-methoxy-1-methyl-1*H*-benzo[*d*]imidazole-5-carbonyl)piperidin-3-yl)carbamate

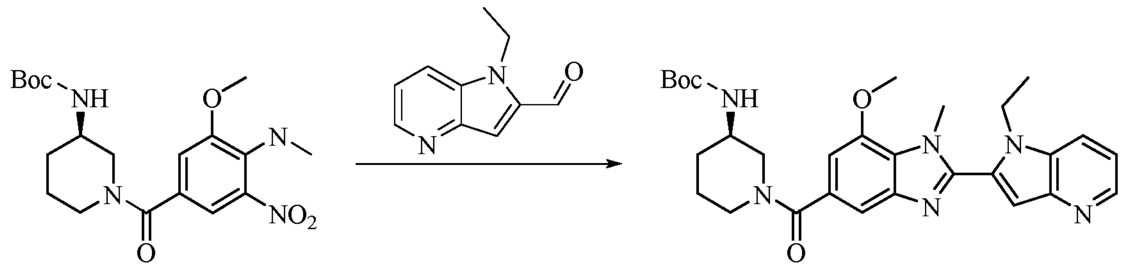

*Tert-butyl* (*R*)-(1-(3-methoxy-4-(methylamino)-5-nitrobenzoyl)piperidin-3-yl)carbamate (245.0 mg, 0.60 mmol, 1.5 eq.) was dissolved in ethanol (10 mL), and the solution was then added with 1-ethyl-1*H*-pyrrolo[3,2-*b*]pyridine - 2-carbaldehyde (70.1 mg, 0.40 mmol, 1.0 eq.), sodium dithionite (208.9 mg, 1.20 mmol, 3.0 eq.) and water (2 mL) to react at 100 °C for 5 h under microwaves. After the end of reaction, as detected by TLC, the reaction solution was concentrated under reduced pressure and added with water (15 mL) and dichloromethane (30 mL) before liquid separation. The organic phase was dried with anhydrous sodium sulfate and concentrated to give a crude product, which was then separated in a silica gel column chromatography (200-300 mesh silica gel, dichloromethane:methanol = 50:1 v/v) to give the target compound (213.6 mg, yield: 87.2%).

Step 2: Synthesis of (*R*)-(3-aminopiperidin-1-yl)(2-(1-ethyl-1*H*-pyrrolo [3,2-*b*]pyridin-2-yl)-7-methoxy-1-methyl-1*H*-benzo[*d*]imidazol-5-yl)methanone

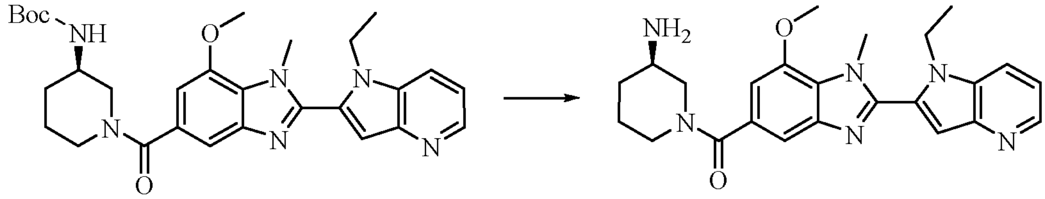

*Tert-butyl* (*R*)-(1-(2-(1-ethyl-1*H*-pyrrolo[3,2-*b*]pyridin-2-yl)-7-methoxy-1-methyl-1*H*-benzo[*d*]imidazole-5-carbonyl)piperidin-3-yl)carbamate (213.6 mg, 0.40 mmol, 1.0 eq.) was dissolved in ethanol (3 mL), and a solution of hydrogen chloride in ethanol (30% w/w, 3 mL) was added dropwise. The resulting solution then reacted at room temperature for 10 h. After the end of reaction, as detected by TLC, the reaction solution was concentrated, then saturated aqueous sodium bicarbonate solution was added to adjust the pH to 7-8, and dichloromethane (15 mL) was added for extraction. The organic phase was dried with anhydrous sodium sulfate and concentrated to give a crude product, which was then purified in a preparative thin-layer chromatography to give the target compound (16.8 mg, yield: 9.7%).
$^1$HNMR (400MHz, DMSO-$d_6$) δ(ppm): 8.46-8.47 (m, 1H), 8.07-8.09 (m, 2H), 7.34 (s, 1H), 7.27-7.30 (m, 1H), 7.18 (s, 1H), 6.86-6.88 (m, 1H), 4.52-4.56 (m, 2H), 4.13 (s, 3H), 3.98 (s, 3H), 2.79-2.87 (m, 2H), 1.97-2.00 (m, 2H), 1.89-1.92 (m, 2H), 1.65-1.75 (m, 2H), 1.45-1.48 (m, 2H), 1.20-1.31 (m, 3H).
Molecular formula: $C_{24}H_{28}N_6O_2$, molecular weight: 432.53, LC-MS (Pos, *m/z*) = 433.1 [M+H]$^+$.

**Example 11: Synthesis of (*R*)-(3-aminopiperidin-1-yl)(2-(6-ethyl-6*H*-thieno[2,3-*b*]pyrrol-5-yl)-7-methoxy-1-methyl-1*H*-benzo[*d*]imidazol-5-yl)methanone (Compound 3)**

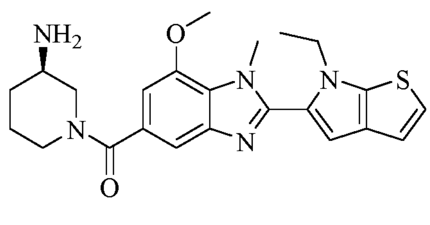

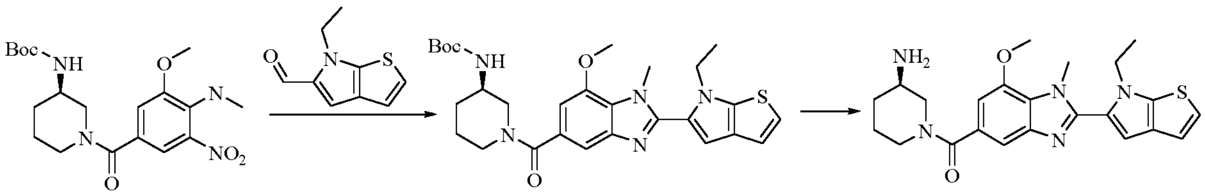

Step 1: Synthesis of *tert*-butyl (*R*)-(1-(2-(6-ethyl-6*H*-thieno[2,3-*b*]pyrrol-5-yl)-7-methoxy-1-methyl-1*H*-benzo[*d*]imidazole-5-carbonyl)piperidin-3-yl)carbamate

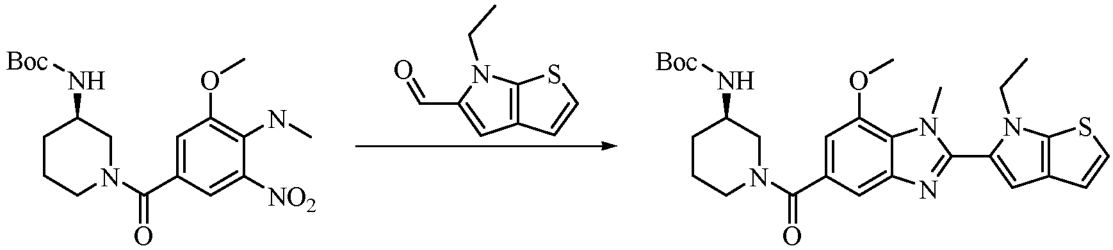

*Tert-butyl* (*R*)-(1-(3-methoxy-4-(methylamino)-5-nitrobenzoyl)piperidin-3-yl)carbamate (469.7 mg, 1.150 mmol, 1.0 eq.) was dissolved in ethanol (16 mL), and the solution was then added with 6-ethyl-6*H*-thieno[2,3-*b*]pyrrole -5-carbaldehyde (200.0 mg, 1.15 mmol), water (2 mL) and sodium dithionite (600.65 mg, 3.45 mmol) to react at 100 °C for 5 h under microwaves. After the end of reaction, as detected by TLC, the reaction solution was concentrated under reduced pressure and added with water (10 mL) and ethyl acetate (20 mL) before liquid separation. The organic phase was concentrated to give a crude product, which was then purified in a silica gel column chromatography (DCM:MeOH = 60:1 v/v) to give a white solid product (300.0 mg, yield: 48.6%).

Step 2: Synthesis of (*R*)-(3-aminopiperidin-1-yl)(2-(6-ethyl-6*H*-thieno [2,3-*b*]pyrrol-5-yl)-7-methoxy-1-methyl-1*H*-benzo[*d*]imidazol-5-yl)methanone

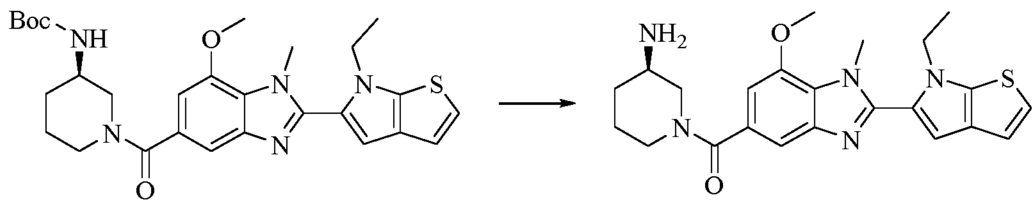

*Tert-butyl* (*R*)-(1-(2-(6-ethyl-6*H*-thieno[2,3-*b*]pyrrol-5-yl)-7-methoxy-1-methyl-1*H*-benzo[*d*]imidazole-5-carbonyl)piperidin-3-yl)carbamate (150.0 mg, 0.28 mmol, 1.0 eq.) was dissolved in dichloromethane (4 mL), a solution of hydrogen chloride in ethanol (30% w/w, 2 mL) was added under an ice bath, and the mixture reacted at room temperature for 2 h. After the end of reaction, as detected by TLC, the reaction solution was concentrated, then aqueous sodium bicarbonate solution was added to adjust the pH to 7-8, and ethyl acetate was added for extraction. The organic phases were combined, dried with anhydrous sodium sulfate, filtered and concentrated to give a crude product, which was then purified in a preparative thin-layer chromatography (DCM:MeOH = 8:1 v/v) to give (*R*)-(3-aminopiperidin-1-yl)(2-(6-ethyl-6*H*-thieno[2,3-*b*]pyrrol-5-yl)-7-methoxy-1-methyl-1*H*-benzo[*d*]imidazol-5-yl)methanone (15.0 mg, yield: 12.3%).
$^1$HNMR (400MHz, DMSO-$d_6$) δ(ppm): 7.26 (s, 1H), 7.17-7.16 (d, 1H), 7.12-7.11 (d, 1H), 6.90-6.86 (d, 1H), 6.63 (s, 1H), 4.40-4.38 (d, 2H), 4.09 (s, 3H), 3.96 (s, 3H), 2.02-1.84 (m, 4H), 1.47 (s, 2H), 1.35-1.31 (t, 3H).
Molecular formula: $C_{23}H_{27}N_5O_2S$, molecular weight: 437.19, LC-MS (Pos, *m/z*) = 438.1 $[M+H]^+$.

**Example 12: Synthesis of (*R*)-(3-aminopiperidin-1-yl)(2-(6-ethyl-6*H*-furo [2,3-*b*]pyrrol-5-yl)-7-methoxy-1-methyl-1*H*-benzo[*d*]imidazol-5-yl)methanone (Compound 4)**

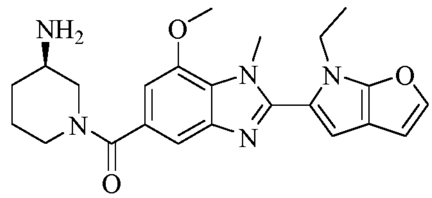

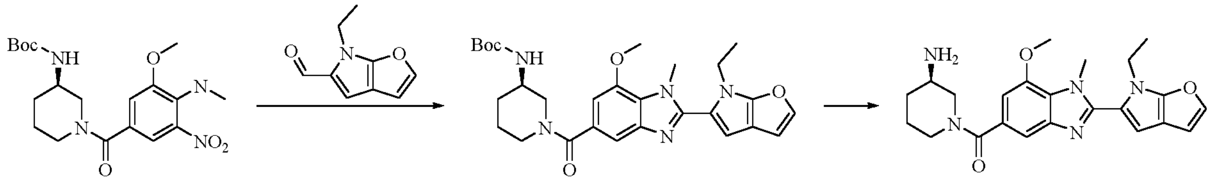

Step 1: Synthesis of *tert*-butyl (*R*)-(1-(2-(6-ethyl-6*H*-furo[2,3-*b*]pyrrol-5-yl)-7-methoxy-1-methyl-1*H*-benzo[*d*]imidazole-5-carbonyl)piperidin-3-yl)carbamate

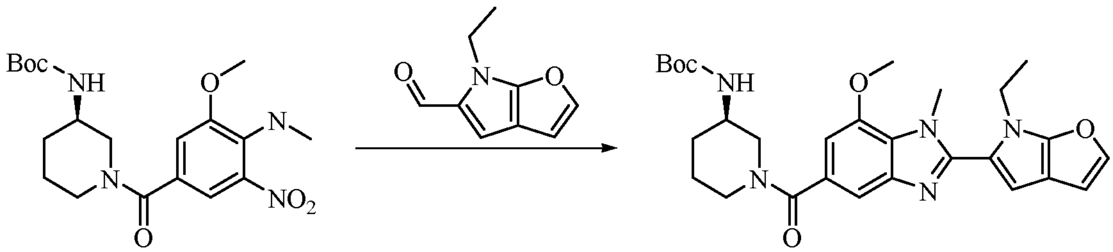

*Tert-butyl* (*R*)-(1-(3-methoxy-4-(methylamino)-5-nitrobenzoyl)piperidin-3-yl)carbamate (600.7 mg, 1.47 mmol, 1.2 eq.) was dissolved in ethanol (6 mL), and the solution was then added with 6-ethyl-6*H*-furo[2,3-*b*]pyrrole-5-carbaldehyde (200.0 mg, 1.22 mmol, 1.0 eq.), sodium dithionite (639.8 mg, 3.67 mmol, 3.0 eq.) and water (2 mL) to react at 100 °C for 5 h under microwaves. After the end of reaction, as detected by TLC, the reaction solution was concentrated under reduced pressure and added with water (25 mL) and dichloromethane (60 mL) before liquid separation. The organic phase was dried with anhydrous sodium sulfate and concentrated to give a crude product, which was then separated in a silica gel column chromatography (200-300 mesh silica gel, dichloromethane:methanol = 100:1 v/v) to give the target compound (60.3 mg, yield: 1.6%).

Step 2: Synthesis of (*R*)-(3-aminopiperidin-1-yl)(2-(6-ethyl-6*H*-furo[2,3-*b*]pyrrol-5-yl)-7-methoxy-1-methyl-1*H*-benzo[*d*] imidazol-5-yl)methanone

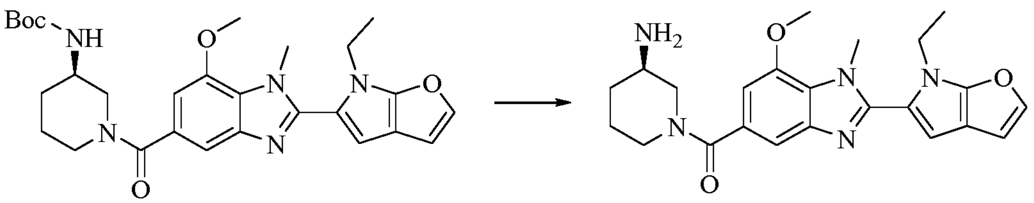

*Tert-butyl* (*R*)-(1-(2-(6-ethyl-6*H*-furo[2,3-*b*]pyrrol-5-yl)-7-methoxy-1-methyl-1*H*-benzo[*d*]imidazole-5-carbonyl)piperidin-3-yl)carbamate (32.5 mg, 0.06 mmol, 1.0 eq.) was dissolved in dichloromethane (4 mL), and 2,6-dimethylpyridine (40.0 mg, 0.37 mmol, 6.0 eq.) was added dropwise under an ice bath. The mixture was then stirred under an ice bath for 30 min. Trimethylsilyl trifluoromethanesulfonate (41.3 mg, 0.18 mmol, 2.0 eq.) was added dropwise, and the resulting mixture reacted under an ice bath for 4 h. After the end of reaction, as detected by TLC, saturated aqueous ammonium chloride solution (5 mL) was added, and dichloromethane (15 mL) was added for extraction. The organic phase was dried with anhydrous sodium sulfate and concentrated to give a crude product, which was then purified in a preparative thin-layer chromatography to give the target compound (*R*)-(3-aminopiperidin-1-yl)(2-(6-ethyl-6*H*-furo[2,3-*b*]pyrrol-5-yl)-7-methoxy-1-methyl-1*H*-benzo[*d*]imidazol-5-yl)methanone (1.8 mg, yield: 6.9%).
$^1$HNMR (400MHz, DMSO-$d_6$) $\delta$(ppm): 8.25(brs, 3H), 7.62(s, 1H), 7.33(s, 1H), 6.86(s, 1H), 6.72(s, 1H), 6.63(s, 1H), 4.40-4.42(m, 2H), 4.11(s, 3H), 3.97 (s, 3H), 3.15-3.24 (m, 2H), 1.98-2.07 (m, 1H), 1.75-1.77 (m, 1H), 1.49-1.65 (m, 2H), 1.32-1.35 (m, 3H).
Molecular formula: $C_{23}H_{27}N_5O_3$, molecular weight: 421.50, LC-MS (Pos, *m/z*) = 422.0 $[M+H]^+$.

**Example 13: Synthesis of (*R*)-(3-aminopiperidin-1-yl)(2-(3-ethyl-3*H*-thieno[2,3-*d*]imidazol-2-yl)-7-methoxy-1-methyl-1*H*-benzo[*d*]imidazol-5-yl)methanone (Compound 8)**

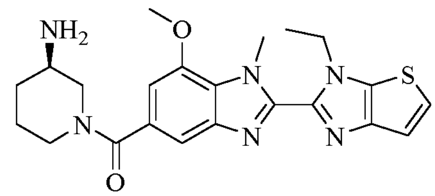

Step 1: Synthesis of *tert-butyl* (*R*)-(1-(3-amino-5-methoxy-4-(methylamino) benzoyl)piperidin-3-yl)carbamate

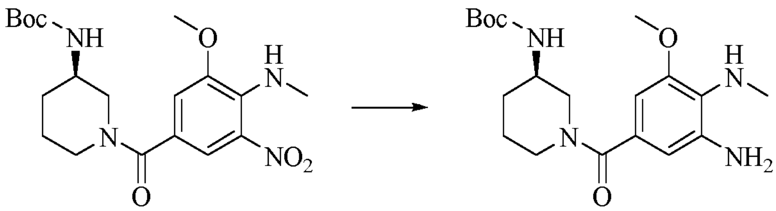

*Tert-butyl* (*R*)-(1-(3-methoxy-4-(methylamino)-5-nitrobenzoyl)piperidin-3-yl)carbamate (500.0 mg, 1.224 mmol, 1.0 eq.) and sodium dithionite (639.0 mg, 3.672 mmol, 3.0 eq.) were dissolved in a mixed solution of ethanol (6.0 mL) and water (3.0 mL). In nitrogen atmosphere, the solution reacted at 90 °C overnight. The reaction was not completed, as detected by TLC, and sodium dithionite (211.0 mg, 1.224 mmol, 1.0 eq.) was supplemented. The reaction was completed 1 h later, as detected by TLC. The reaction solution was concentrated under reduced pressure to give a crude product, which was then purified in a silica gel column chromatography (DCM: MeOH = 200:1-60:1 v/v) to give the target compound (450.0 mg, yield: 97.2%).

Step 2: Synthesis of *tert-butyl* (*R*)-(1-(2-(3-ethyl-3*H*-thieno[2,3-*d*]imidazol-2-yl)-7-methoxy-1-methyl-1*H*-benzo[*d*]imidazole-5-carbonyl)piperidin-3-yl) carbamate

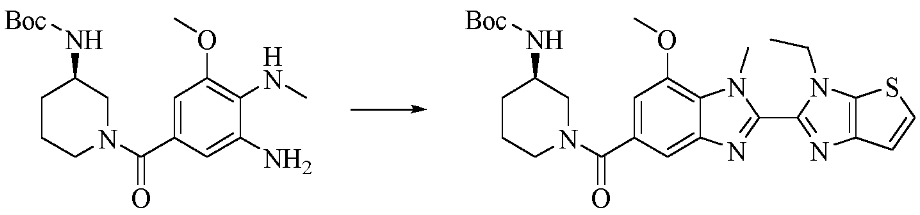

*Tert-butyl* (*R*)-(1-(3-amino-5-methoxy-4-(methylamino)benzoyl)piperidin -3-yl)carbamate (250.0 mg, 0.660 mmol, 1.0 eq.), 3-ethyl-3*H*-thieno[2,3-*d*]imidazol-2-carbaldehyde (143.0 mg, 0.793 mmol, 1.2 eq.) and potassium carbonate (274.0 mg, 1.982 mmol, 3.0 eq.) were dissolved in 1,4-dioxane (3.0 mL) and water (0.5 mL), and the solution reacted at 110 °C overnight. After the end of reaction, as detected by TLC, the reaction solution was concentrated under reduced pressure, added with water (50.0 mL) and extracted with ethyl acetate (20.0 mL), dried and concentrated under reduced pressure to give a crude product, which was then purified in a silica gel column chromatography (DCM: MeOH = 100:1 to 20:1 v/v) to give the target compound (125.0 mg, yield: 35.1%).

Step 3: Synthesis of (*R*)-(3-aminopiperidin-1-yl)(2-(3-ethyl-3*H*-thieno[2,3-*d*] imidazol-2-yl)-7-methoxy-1-methyl-1*H*-benzo[*d*]imidazol-5-yl)methanone

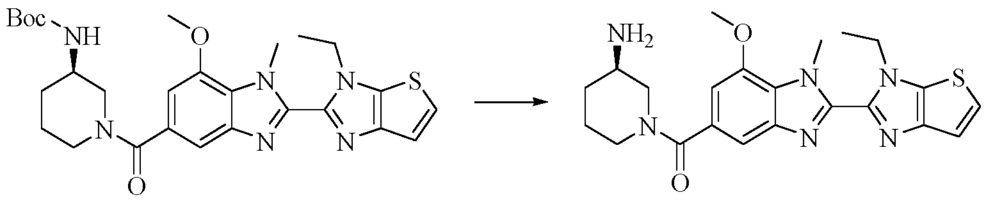

(Compound 8)

*Tert-butyl* (*R*)-(1-(2-(3-ethyl-3*H*-thieno[2,3-*d*]imidazol-2-yl)-7-methoxy-1-methyl-1*H*-benzo[*d*]imidazole-5-carbonyl)piperidin-3-yl)carbamate (125.0 mg, 0.232 mmol, 1.0 eq.) was dissolved in dichloromethane. The solution was cooled to 0 °C and added with trifluoroacetic acid (1.5 mL). After the end of reaction 2 h later, as detected by TLC, the reaction solution was concentrated under reduced pressure, added with water (10.0 mL), added with ammonia solution to adjust the pH to 9-10, extracted with ethyl acetate (10.0 mL × 2), and concentrated under reduced pressure to give a crude product, which was then purified in a silica gel column chromatography (DCM: MeOH = 60:1 to 11:1 v/v) to give the target compound (90.0 mg, yield: 88.4%).
$^1$H NMR (400 MHz, DMSO-$d_6$) δ(ppm): 7.44-7.42 (m, 1H), 7.31-7.29 (m, 2H), 6.87 (s, 1H), 4.76-4.70 (m, 2H), 4.38 (s, 3H), 3.98 (s, 3H), 2.93 (m, 1H), 2.68-2.67 (m, 2H), 1.89-1.85 (m, 2H), 1.75-1.65 (m, 2H), 1.50-1.46 (m, 3H), 1.26-1.24 (m, 2H).
Molecular formula: $C_{22}H_{26}N_6O_2S$, molecular weight: 438.55, LC-MS (Pos, *m/z*) = 439.36 [M+H]$^+$.

**Example 14: Synthesis of (*R*)-(3-aminopiperidin-1-yl)(2-(6-ethyl-2-methyl-6*H*-thieno[2,3-*b*]pyrrol-5-yl)-7-methoxy-1-methyl-1*H*-benzo[*d*]imidazol-5-yl)methanone (Compound 9)**

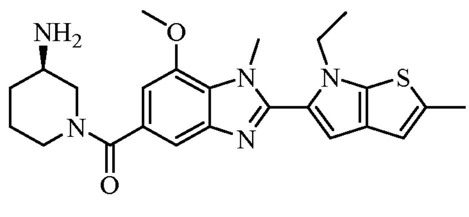

Step 1: Synthesis of 3-methoxy-4-(methylamino)-5-nitrobenzoic acid

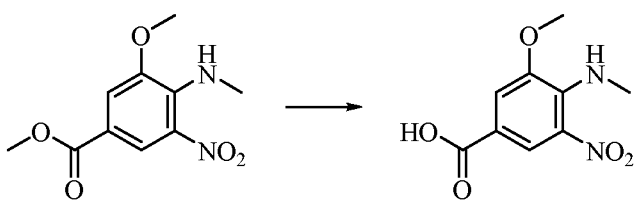

Methyl 3-methoxy-4-(methylamino)-5-nitrobenzoate (10.0 g, 41 mmol, 1.0 eq.) was dissolved in tetrahydrofuran (200 mL), and an aqueous solution (40 mL) of lithium hydroxide monohydrate (7.0 g, 166 mmol, 4.0 eq.) was added dropwise. The resulting mixture was stirred at 45 °C for 1 h. After the end of reaction, as detected by TLC, the reaction solution was concentrated under reduced pressure, added with water (200 mL), added with 2 mol/L hydrochloric acid under an ice bath to adjust the pH to 2-3, and extracted with dichloromethane (150 mL). The organic phase was dried with anhydrous sodium sulfate, and concentrated under reduced pressure to give the target compound (8.1 g, yield: 87.9%).

Step 2: Synthesis of *tert-butyl* (*R*)-(1-(3-methoxy-4-(methylamino)-5-nitrobenzoyl) piperidin-3 -yl)carbamate

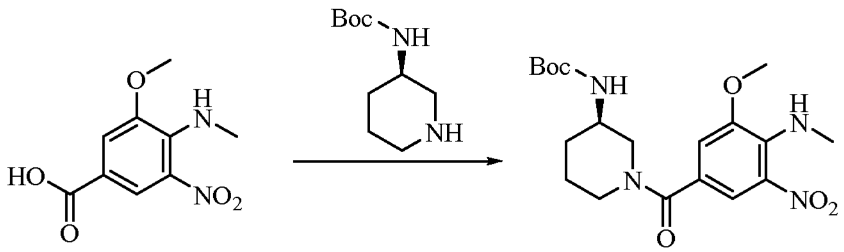

3-methoxy-4-(methylamino)-5-nitrobenzoic acid (2.23 g, 9.85 mmol, 1.0 eq.) was added to a mixed solution of DMF (15 mL) and tetrahydrofuran (60 mL). DIPEA (2.54 g, 19.7 mmol, 2.0 eq.) and HATU (4.12 g, 10.83 mmol, 1.1 eq.) were added under an ice bath. The mixture reacted for 2 h, and then was added with *tert*-butyl (*R*)-piperidin-3-yl carbamate (2.36 g, 11.83 mmol, 1.2 eq.). The reaction solution was then stirred at room temperature overnight. After the end of reaction, as detected by TLC, the reaction solution was concentrated under reduced pressure and added with ethyl acetate (150 mL) and water (200 mL) before liquid separation. The organic phase was dried with anhydrous sodium sulfate and concentrated under reduced pressure to give the target compound (4.7 g of crude product).

Step 3: Synthesis of *tert*-butyl (*R*)-(1-(2-(6-ethyl-2-methyl-6*H*-thieno(2,3-*b*] pyrrol-5-yl)-7-methoxy-1-methyl-1*H*-benzo[*d*] imidazole-5-carbonyl)piperidin-3-yl)carbamate

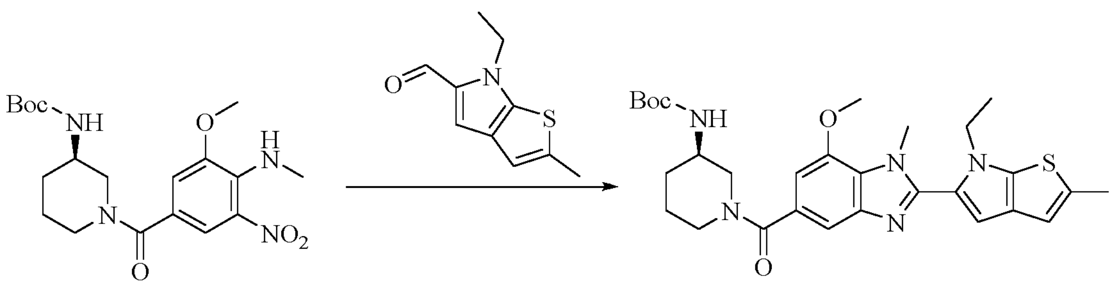

*Tert-butyl* (*R*)-(1-(3-methoxy-4-(methylamino)-5-nitrobenzoyl)piperidin-3-yl)carbamate (721.6 mg, 1.51 mmol, 1.0 eq.) was added to ethanol (9.0 mL), and the solution was then added with an aqueous solution (3.0 mL) of 6-ethyl-2-methyl-6*H*-thieno[2,3-*b*]pyrrole-5-carbaldehyde (291.82 mg, 1.51 mmol, 1.0 eq.) and sodium dithionite (788.67 mg, 4.53 mmol, 3.0 eq.) to react at 100 °C for 4 h under microwaves. After the end of reaction, as detected by TLC, the reaction solution was concentrated under reduced pressure and added with dichloromethane (20 mL) and water (15 mL) before liquid separation. The organic phase was dried with anhydrous sodium sulfate and concentrated to give a crude product, which was then purified in a silica gel column chromatography (dichloromethane:methanol = 80:1 v/v) to give the target compound (353.7 mg, yield: 42.4%).

Step 4: Synthesis of (*R*)-(3-aminopiperidin-1-yl)(2-(6-ethyl-2-methyl-6*H*-thieno[2,3-*b*]pyrrol-5-yl)-7-methoxy-1-methyl-1*H*-benzo[*d*]imidazol-5-yl)methanone

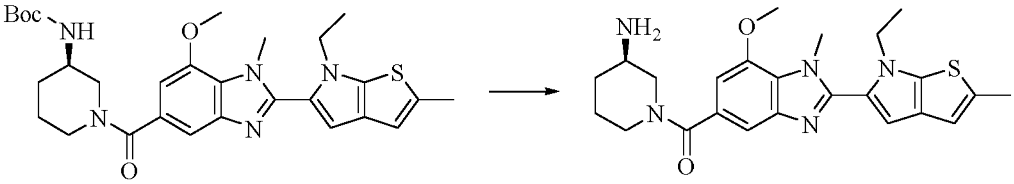

(Compound 9)

*Tert-butyl* (*R*)-(1-(2-(6-ethyl-2-methyl-6*H*-thieno[2,3-*b*]pyrrol-5-yl)-7-methoxy-1-methyl-1*H*-benzo[*d*]imidazole-5-carbonyl)piperidin-3-yl)carbamate (300.0 mg, 0.54 mmol, 1.0 eq.) was added to dichloromethane (6 mL), trifluoroacetic acid (3 mL) was added dropwise under an ice bath, and the mixture was stirred at room temperature for 2 h. After the end of reaction, as detected by TLC, saturated aqueous sodium bicarbonate solution (10 mL) was added dropwise under an ice bath to adjust the pH to 7-8, and dichloromethane (15 mL) was added for extraction. The organic phase was dried with anhydrous sodium sulfate, and concentrated under reduced pressure to give the target compound (177.9 mg, yield: 72.9%).

$^1$HNMR (400MHz, DMSO-$d_6$) δ(ppm):7.24 (s, 1H), 6.81 (m, 2H), 6.77 (s, 1H), 4.33-4.38 (m, 2H), 4.09 (s, 3H), 3.96 (s, 3H), 2.86-2.92 (s, 1H), 2.67-2.69 (s, 3H), 2.50-2.51(s, 2H), 1.85-1.87 (m, 1H), 1.67 (s, 1H), 1.43-1.49 (m, 3H),1.31-1.33(m, 3H), 1.24-1.29 (m, 1H).

Molecular formula: $C_{24}H_{29}N_5O_2S$, molecular weight: 451.59, LC-MS (Pos,*m*/*z*) = 452.9 $[M+H]^+$.

**Example 15: Synthesis of (*R*)-(3-aminopiperidin-1-yl)(2-(6-ethyl-3-methyl-6*H*-thieno[2,3-*b*]pyrrol-5-yl)-7-methoxy-1-methyl-1*H*-benzo[*d*]imidazol-5-yl)methanone (Compound 10)**

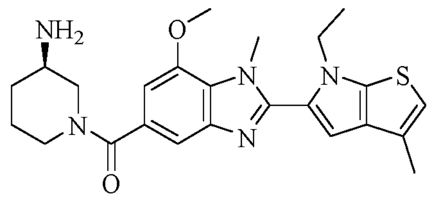

Step 1: Synthesis of *tert*-butyl (*R*)-(1-(2-(6-ethyl-3-methyl-6*H*-thieno[2,3-*b*] pyrrol-5-yl)-7-methoxy-1-methyl- 1*H*-benzo[*d*] imidazole-5-carbonyl)piperidin-3-yl)carbamate

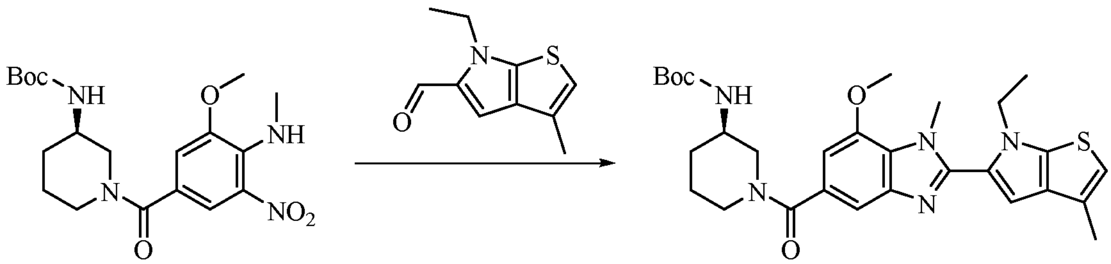

*Tert-butyl* (*R*)-(1-(3-methoxy-4-(methylamino)-5-nitrobenzoyl)piperidin-3-yl)carbamate (347.0 mg, 0.84 mmol, 1.0 eq.) was dissolved in ethanol (5.0 mL), and the solution was then added with an aqueous solution (2.0 mL) of 6-ethyl-3-methyl-6*H*-thieno[2,3-*b*]pyrrole-5-carbaldehyde (164.1 mg, 0.84 mmol, 1.0 eq.) and sodium dithionite (438.7 mg, 2.52 mmol, 3.0 eq.) to react at 100 °C for 5 h under microwaves. After the end of reaction, as detected by TLC, the reaction solution was concentrated under reduced pressure to give a crude product which was then purified in a silica gel column chromatography (dichloromethane:methanol = 100:1 v/v) to give the target compound (170.0 mg, yield: 36.7%).

Step 2: Synthesis of (*R*)-(3-aminopiperidin-1-yl)(2-(6-ethyl-3-methyl-6*H*-thieno[2,3-*b*]pyrrol-5-yl)-7-methoxy-1-methyl-1*H*-benzo[*d*]imidazol-5-yl)methanone

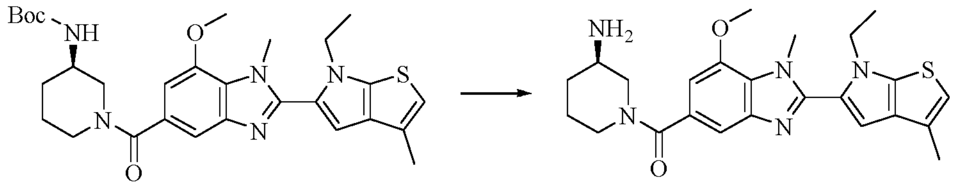
(Compound 10)

*Tert-butyl* (*R*)-(1-(2-(6-ethyl-3-methyl-6*H*-thieno[2,3-*b*]pyrrol-5-yl)-7-methoxy-1-methyl-1*H*-benzo[*d*]imidazole-5-carbonyl)piperidin-3-yl)carbamate (170.0 mg, 0.3 mmol, 1.0 eq.) was dissolved in dichloromethane (3.0 mL), trifluoroacetic acid (1.5 mL) was added dropwise under an ice bath, and the mixture was stirred at room temperature for 1 h. After the end of reaction, as detected by TLC, saturated aqueous sodium bicarbonate solution (5 mL) was added dropwise under an ice bath to adjust the pH to 7-8, and dichloromethane (10 mL) was added before liquid separation. The organic phase was dried with anhydrous sodium sulfate, filtered and concentrated under reduced pressure to give the target compound (75.1 mg, yield: 54.0%).
$^1$H NMR (400 MHz, DMSO-$d_6$) $\delta$(ppm): 7.25 (s, 1H), 6.87 (s, 1H), 6.82 (s, 1H), 6.74-6.75 (s, 1H), 4.34-4.39 (m, 2H), 4.11 (s, 3H), 3.97 (s, 3H), 2.92 (s, 1H), 2.66 (s, 2H), 2.32 (s, 3H), 1.85-1.88 (d, 1H), 1.59 (m, 3H), 1.54 (m, 1H), 1.29-1.32 (m, 3H).
Molecular formula: $C_{24}H_{29}N_5O_2S$, molecular weight: 451.59, LC-MS (Pos, m/z) = 452.4 $[M+H]^+$.

**Example 16: Synthesis of *N*-(3-aminocyclobutyl)-2-(6-ethyl-6*H*-thieno[2,3-*b*]pyrrol-5-yl)-7-methoxy-*N*,1-dimethyl-1*H*-benzo[*d*]imidazole-5-carboxamide (Compound 13)**

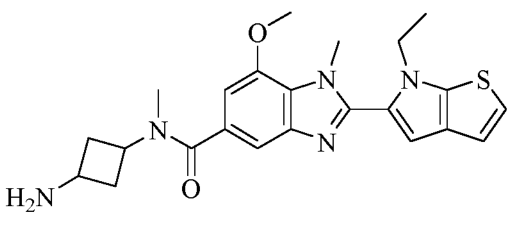

Step 1: Synthesis of *tert-butyl* (3-((4-methoxybenzyl)(methyl)amino) cyclobutyl)carbamate

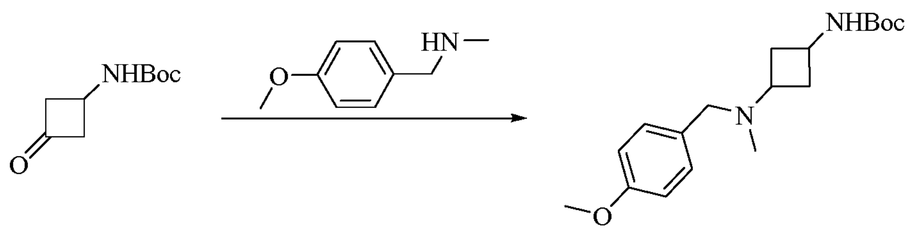

*Tert-butyl* (3-oxocyclobutyl)carbamate (2.69 g, 14.5 mmol, 1.1 eq.) was dissolved in dichloromethane (10.0 mL), and 1-(4-methoxyphenyl)-*N*-methylmethanamine (2.0 g, 13.2 mmol, 1.0 eq.) and glacial acetic acid (0.2 mL) were added. The mixture was stirred at room temperature for 2 h and added with sodium triacetoxyborohydride (5.6 g, 26.4 mmol, 2.0 eq.) to react at room temperature overnight. After the end of reaction, as detected by TLC, saturated aqueous sodium bicarbonate solution (200 mL) was added dropwise under an ice bath to adjust the pH to 7-8, and dichloromethane (100 mL) was added before liquid separation. The organic phase was dried with anhydrous sodium sulfate, filtered and concentrated to give a crude product, which was then purified in a silica gel column chromatography (dichloromethane:

methanol = 140:3 v/v) to give the target compound (2.1 g, yield: 49.6%).

Step 2: Synthesis of *tert-butyl* (3-(methylamino)cyclobutyl)carbamate

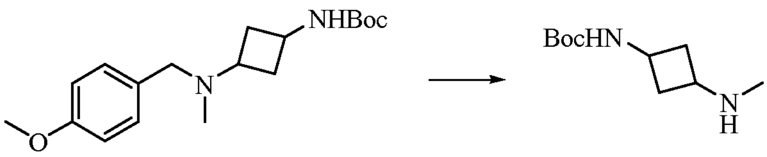

*Tert-butyl* (3-((4-methoxybenzyl)(methyl)amino)cyclobutyl)carbamate (2.1 g, 6.55 mmol, 1.0 eq.) was dissolved in methanol (20.0 mL), and Pd/C (525.0 mg) was added. Hydrogen was charged to replace three times by evacuation, and the mixture was stirred at room temperature overnight. After the end of reaction, as detected by TLC, the reaction solution was filtered, and the filtrate was concentrated under reduced pressure to give the target compound (1.83 g of crude product).

Step 3: Synthesis of *tert*-butyl (3-(3-methoxy-*N*-methyl-4-(methylamino)-5-nitrobenzamido)cyclobutyl)carbamate

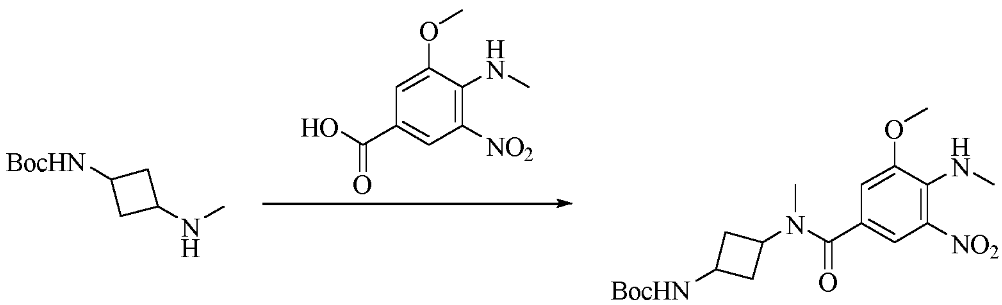

3-methoxy-4-(methylamino)-5-nitrobenzoic acid (1.72 g, 6.15 mmol, 1.0 eq.) was dissolved in DMF (10 mL). The solution was added with DIPEA (1.97 g, 12.31 mmol, 2.0 eq.) and HATU (3.19 g, 6.76 mmol, 1.1 eq.) under an ice bath and stirred for 2 h. Then a solution (10 mL) of *tert*-butyl (3-(methylamino)cyclobutyl)carbamate (1.53 g, 6.15 mmol, 1.0 eq.) in DMF was added, and the mixture was stirred at room temperature for 2 h. After the end of reaction, as detected by TLC, the reaction solution was concentrated under reduced pressure and added with ethyl acetate (100 mL) and saturated aqueous sodium chloride solution (150 mL) before liquid separation. The organic phase was dried with anhydrous sodium sulfate and concentrated to give a crude product, which was then purified in a silica gel column chromatography (dichloromethane:methanol = 200:1 v/v) to give the target compound (1.7 g, two-step yield: 67.7%).

Step 4: Synthesis of *tert*-butyl (3-(2-(6-ethyl-6*H*-thieno[2,3-*b*]pyrrol-5-yl)-7-methoxy-*N*,1-dimethyl-1*H*-benzo[*d*]imidazole-5-carbaxamindo)cyclobutyl) carbamate

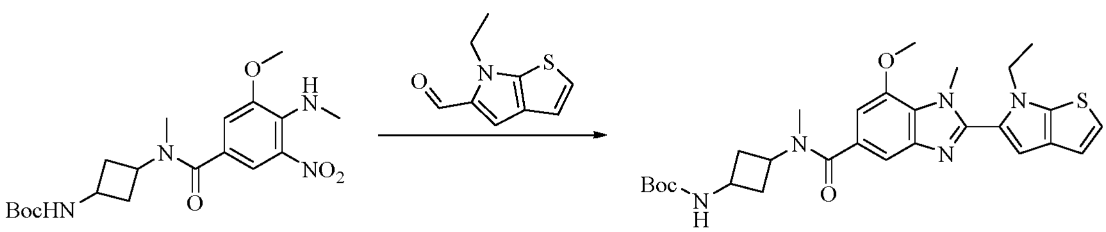

*Tert-butyl* (3-(3-methoxy-*N*-methyl-4-(methylamino)-5-nitrobenzamido) cyclobutyl)carbamate (1.17 g, 2.87 mmol, 1.0 eq.) was dissolved in ethanol (9.0 mL), and the solution was then added with an aqueous solution (4.5 mL)

of 6-ethyl-6*H*-thieno[2,3-*b*]pyrrole-5-carbaldehyde (515.1 mg, 2.87 mmol, 1.0 eq.) and sodium dithionite (1.50 g, 8.61 mmol, 3.0 eq.) to react at 100 °C overnight. After the end of reaction, as detected by TLC, the reaction solution was concentrated under reduced pressure to give a crude product, which was dissolved in dichloromethane (100 mL). The solution was filtered and the filtrate was dried with anhydrous sodium sulfate and concentrated to give a crude product, which was then purified in a silica gel column chromatography (dichloromethane:methanol = 100:1 v/v) to give the target compound (1.01 g, yield: 65.5%).

Step 5: Synthesis of *N*-(3-aminocyclobutyl)-2-(6-ethyl-6*H*-thieno[2,3-*b*]pyrrol-5-yl)-7-methoxy-*N*,1-dimethyl-1*H*-benzo[*d*]imidazole-5-carboxamide

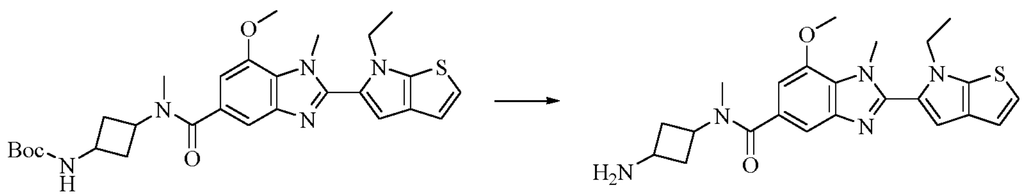

(Compound 13)

*Tert-butyl* (3-(2-(6-ethyl-6*H*-thieno[2,3-*b*]pyrrol-5-yl)-7-methoxy-*N*,1-dimethyl-1*H*-benzo[*d*]imidazole-5-carboxamido)cyclobutyl)carbamate (300.0 mg, 0.55 mmol, 1.0 eq.) was dissolved in dichloromethane (3.0 mL), trifluoroacetic acid (1.5 mL) was added dropwise under an ice bath, and the mixture was stirred at room temperature for 2 h. After the end of reaction, as detected by TLC, saturated aqueous sodium bicarbonate solution (10 mL) was added dropwise under an ice bath to adjust the pH to 7-8, and dichloromethane (15 mL) was added for extraction. The organic phase was dried with anhydrous sodium sulfate and concentrated under reduced pressure to give the target compound (109.3 mg, yield: 44.8%).

$^1$HNMR (400MHz, DMSO-$d_6$) δ(ppm):7.21 (s, 1H), 7.16-7.17 (m, 1H), 7.11-7.13 (m, 1H), 6.90 (s, 1H), 6.76 (s, 1H), 4.76 (s, 1H), 4.37-4.42 (m, 2H), 4.10 (s, 3H), 3.97 (s, 3H), 3.42-3.45 (m, 1H), 2.97-2.98 (s, 3H), 2.43-2.46 (m, 2H), 2.41 (m, 1H),1.90-1.95 (m, 1H),1.81 (m, 2H), 1.31-1.39 (m, 3H).

Molecular formula: $C_{23}H_{27}N_5O_2S$, molecular weight: 437.56, LC-MS (Pos,*m*/*z*) = 338.6 [M+H]$^+$.

**Example 17: Synthesis of (4-amino-6-azaspiro[2.5]octan-6-yl)(2-(6-ethyl-6*H*-thieno[2,3-*b*]pyrrol-5-yl)-7-methoxy-7-methyl-1*H*-benzo[*d*]imidazol-5-yl)methanone (Compound 14)**

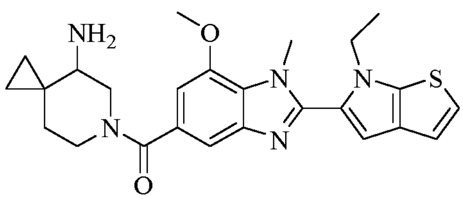

Step 1: Synthesis of 3-methoxy-4-(methylamino)-5-nitrobenzoic acid

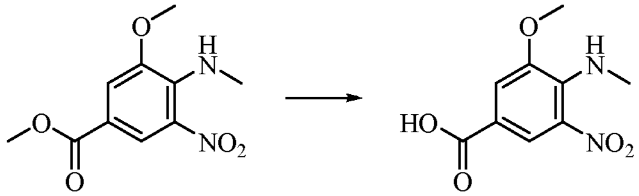

Methyl 3-methoxy-4-(methylamino)-5-nitrobenzoate (3.0 g, 12.4 mmol, 1.0 eq.) was dissolved in tetrahydrofuran

(30 mL), and an aqueous solution (6 mL) of lithium hydroxide monohydrate (2.0 g, 49.9 mmol, 4.0 eq.) was added dropwise. The resulting mixture was stirred at 45 °C overnight. After the end of reaction, as detected by TLC, the reaction solution was concentrated under reduced pressure, added with water (60 mL), added with 2 mol/L hydrochloric acid under an ice bath to adjust the pH to 2-3, and extracted with dichloromethane (50 mL). The organic phase was dried with anhydrous sodium sulfate and concentrated under reduced pressure to give the target compound (2.6 g, yield: 92.8%).

Step 2: Synthesis of *tert-butyl* (6-(3-methoxy-4-(methylamino)-5-nitrobenzoyl) - 6-azaspiro[2.5]octan-4-yl)carbamate

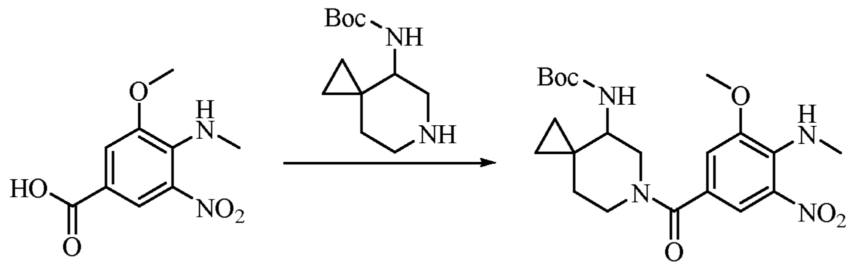

3-methoxy-4-(methylamino)-5-nitrobenzoic acid (150.0 mg, 0.66 mmol, 1.0 eq.) was added to a mixed solution of DMF (1.5 mL) and tetrahydrofuran (12 mL). The resulting solution was added with DIPEA (171.2 mg, 1.32 mmol, 2.0 eq.) and HATU (277.1 mg, 0.72 mmol, 1.1 eq.) under an ice bath to react for 2 h. *Tert*-butyl (6-azaspiro[2.5]octan-4-yl)carbamate (165.0 mg, 0.72 mmol, 1.1 eq.) was added. The solution was then stirred at room temperature overnight. After the end of reaction, as detected by TLC, the reaction solution was concentrated under reduced pressure and added with ethyl acetate (15 mL) and saturated aqueous sodium chloride solution (20 mL) before liquid separation. The organic phase was dried with anhydrous sodium sulfate, filtered and concentrated to give a crude product, which was then purified in a silica gel column chromatography (dichloromethane:methanol = 160:1 v/v) to give the target compound (200.6 mg, yield: 69.4%).

Step 3: Synthesis of *tert*-butyl (6-(2-(6-ethyl-6*H*-thieno[2,3-*b*]pyrrol-5-yl)-7-methoxy-1-methyl-1*H*-benzo[*d*]imidazole-5-carbonyl)-6-azaspiro[2.5]octan-4-yl)carbamate

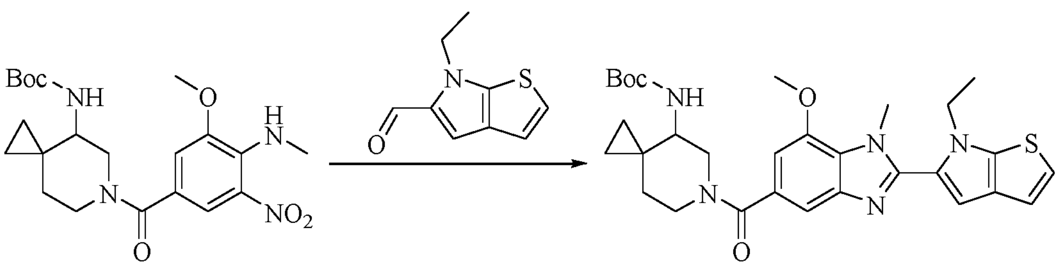

*Tert-butyl* (6-(3-methoxy-4-(methylamino)-5-nitrobenzoyl)-6-azaspiro [2.5]octan-4-yl)carbamate (200.0 mg, 0.46 mmol, 1.0 eq.) was dissolved in ethanol (8.0 mL), and the solution was then added with an aqueous solution (1.5 mL) of 6-ethyl-6*H*-thieno[2,3-*b*]pyrrole-5-carbaldehyde (82.5 mg, 0.46 mmol, 1.0 eq.) and sodium dithionite (240.2 mg, 1.38 mmol, 3.0 eq.) to react at 100 °C for 5 h under microwaves. After the end of reaction, as detected by TLC, the reaction solution was concentrated under reduced pressure and added with dichloromethane (20 mL) and water (15 mL) before liquid separation. The organic phase was dried with anhydrous sodium sulfate, filtered and concentrated to give a crude product, which was then purified in a silica gel column chromatography (dichloromethane:methanol = 80:1 v/v) to give the target compound (130.1 mg, yield: 50.1%).

Step 4: Synthesis of (4-amino-6-azaspiro[2.5]octan-6-yl)(2-(6-ethyl-6*H*-thieno [2,3-*b*]pyrrol-5-yl)-7-methoxy-1-methyl-1*H*-benzo[*d*]imidazol-5-yl)methanone

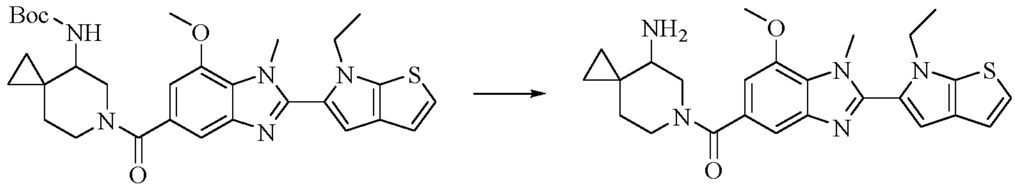

(Compound 14)

*Tert*-butyl (6-(2-(6-ethyl-6*H*-thieno[2,3-*b*]pyrrol-5-yl)-7-methoxy-1-methyl-1*H*-benzo[*d*]imidazole-5-carbonyl)-6-azaspiro[2.5]octan-4-yl)carbamate (130.1 mg, 0.23 mmol, 1.0 eq.) was added to ethanol (4 mL), a solution of hydrogen chloride in ethanol (30% w/w, 4 mL) was added dropwise under an ice bath, and the mixture was stirred at room temperature for 2 h. After the end of reaction, as detected by TLC, the reaction solution was concentrated under reduced pressure to remove ethanol, added with saturated aqueous sodium bicarbonate solution (10 mL) dropwise under an ice bath to adjust the pH to 7-8, and added with dichloromethane (15 mL) before liquid separation. The organic phase was dried with anhydrous sodium sulfate, filtered and concentrated to give a crude product, which was then purified in a preparative thin-layer chromatography (dichloromethane:methanol = 15:1 v/v) to give the target compound (20.1 mg, yield: 18.8%).

$^1$HNMR (400MHz, DMSO-$d_6$) δ(ppm):7.35 (s, 1H), 7.16-7.18 (m, 1H), 7.11-7.13 (m, 1H), 6.90 (s, 2H), 4.37-4.42 (m, 2H), 4.10 (s, 3H), 3.97 (s, 3H), 3.54 (m, 2H), 3.51 (m,1H), 1.74 (m, 2H), 1.32-1.35 (m, 3H), 1.19-1.24 (m, 2H), 0.57 (s, 2H), 0.22-0.30 (m, 2H).

Molecular formula: $C_{25}H_{29}N_5O_2S$, molecular weight: 463.60, LC-MS (Pos,*m*/*z*) = 464.2 $[M+H]^+$.

**Example 18: Synthesis of (3-amino-3,4-dihydroquinolin-1(2*H*)-yl)(2-(1-ethyl-1*H*-pyrrolo[2,3-*b*]pyridin-2-yl)-7-methoxy-1-methyl-1*H*-benzo[*d*]imidazol-5-yl)methanone (Compound** 15)

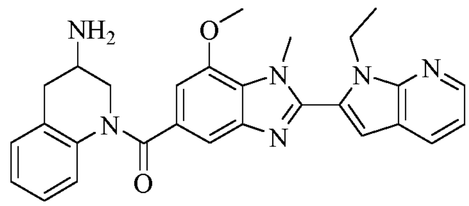

Step 1: Synthesis of 3-methoxy-4-(methylamino)-5-nitrobenzoyl chloride

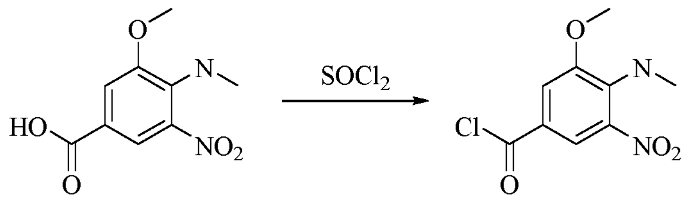


3-methoxy-4-(methylamino)-5-nitrobenzoic acid (600.0 mg, 2.65 mmol, 1.0 eq.) was dissolved in thionyl chloride (10 mL), and the solution was stirred at room temperature for 0.5 h. The reaction solution was concentrated under reduced pressure to give a yellow solid product (540 mg of crude product), which was directly used in the next step.

Step 2: Synthesis of *tert-butyl* (1-(3-methoxy-4-(methylamino)-5-nitrobenzoyl)-1,2,3,4-tetrahydroquinolin-3-yl)carbamate

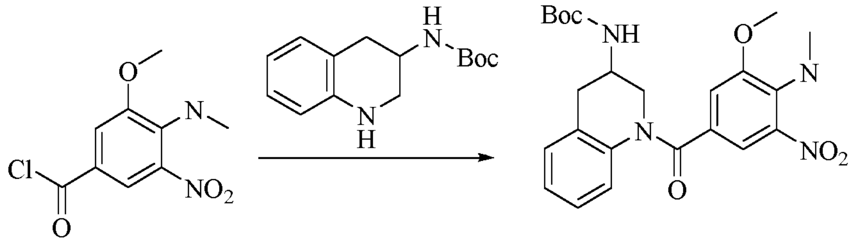

3-methoxy-4-(methylamino)-5-nitrobenzoyl chloride (300 mg, 1.23 mmol, 1.0 eq.), *tert-butyl* (1,2,3,4-tetrahydroquinoline-3-yl)carbamate (305 mg, 1.23 mmol, 1.0 eq.), triethylamine (269 mg, 2.69 mmol, 3.0 eq.) and DMAP (15 mg, 0.12 mmol, 0.1 eq.) were dissolved in dichloromethane (10 mL), and the solution was stirred at room temperature for 1 h. Water (5 mL) was added to quench the reaction, and dichloromethane (20 mL × 2) was added for extraction. The organic phases were combined and concentrated to give a crude product, which was then purified in a silica gel column chromatography (dichloromethane:methanol = 30:1 v/v) to give a red solid product (150 mg, yield: 26.7%).

Step 3: Synthesis of *tert*-butyl (1-(2-(1-ethyl-1*H*-pyrrolo[2,3-*b*]pyridin-2-yl)-7-methoxy-1-methyl-1*H*-benzo[*d*]imidazole-5-carbonyl)-1,2,3,4-tetrahydroquinolin-3-yl)carbamate

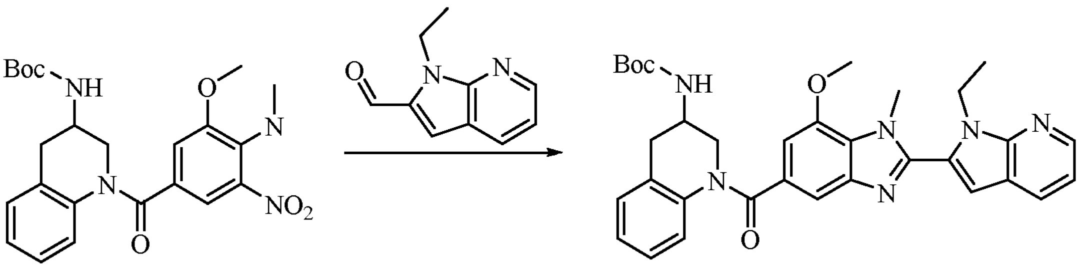

*Tert-butyl* (1-(3-methoxy-4-(methylamino)-5-nitrobenzoyl)-1,2,3,4-tetrahydroquinolin-3-yl)carbamate (50.0 mg, 0.1 mmol, 1.0 eq.) and 1-ethyl-1*H*-pyrrolo[2,3-*b*]pyridine-2-carbaldehyde (17.4 mg, 0.1 mmol, 1.0 eq.) were dissolved in a mixed solution of ethanol (2 mL) and water (1 mL), and the resulting solution was then added with sodium dithionite (53 mg, 0.3 mmol, 1.0 eq.) to react at 100 °C for 5 h under microwaves. The reaction solution was concentrated to give a crude product, which was then purified in a silica gel column chromatography (petroleum ether:ethyl acetate = 1:1 v/v) to give a colorless oily product (35 mg, yield: 60.3%).

Step 4: Synthesis of (3-amino-3,4-dihydroquinolin-1(2*H*)-yl)(2-(1-ethyl-1*H*-pyrrolo[2,3-*b*]pyridin-2-yl)-7-methoxy-1-methyl-1*H*-benzo[*d*]imidazol-5-yl)methanone

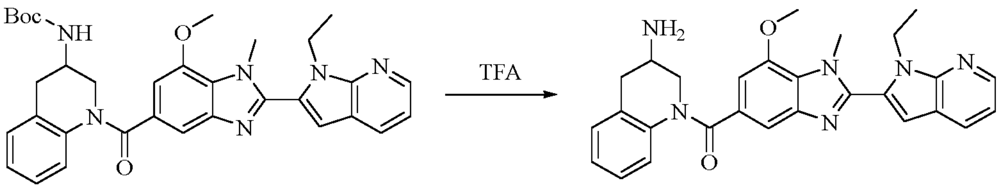


(Compound 15)

*Tert-butyl* (1-(2-(1-ethyl-1*H*-pyrrolo[2,3-*b*]pyridin-2-yl)-7-methoxy-1-methyl-1*H*-benzo[*d*]imidazole-5-carbonyl)-1,2,3,4-tetrahydroquinolin-3-yl)carbamate (35 mg) was dissolved in trifluoroacetic acid (1 mL), and the solution was

stirred at room temperature for 1 h. The reaction solution was concentrated to give a crude product, which was then purified in a reversed-phase column chromatography (acetonitrile: water: ammonia solution = 30:100:0.05 v/v) to give a white solid product (4.4 mg, yield: 26.6%).

$^1$HNMR (400MHz, DMSO-*d*$_6$) δ(ppm): 8.46 (m, 1H), 8.04 (m, 1H), 7.50 (s, 1H), 7.31 (s, 2H), 7.13-7.26 (m, 1H), 6.91-7.09 (m, 3H), 6.77 (s, 1H), 4.62-4.73 (m, 2H), 4.22 (m, 1H), 4.13 (s, 3H), 3.93 (s, 3H), 3.68 (m, 1H), 3.57 (m, 1H), 3.21 (m, 1H), 2.73 (m, 1H), 1.20-1.41 (m, 3H).

Molecular formula: $C_{28}H_{28}N_6O_2$, molecular weight: 480.23, LC-MS (Pos, m/z) = 481.21 [M+H]$^+$.

**Example 19: Synthesis of (4-amino-6-azaspiro[2.5]octan-6-yl)(2-(1-ethyl-1*H*-pyrrolo[2,3-*b*]pyridin-2-yl)-7-methoxy-1-methyl-1*H*-benzo[*d*]imidazol-5-yl)methanone hydrochloride (Hydrochloride of Compound 16)**

Step 1: Synthesis of *tert-butyl* (6-(3-methoxy-4-(methylamino)-5-nitrobenzoyl)-6-azaspiro[2.5]octan-4-yl)carbamate

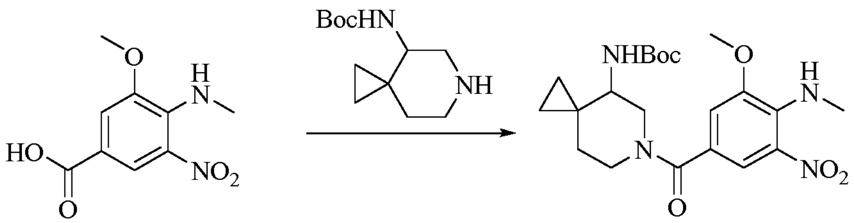

3-methoxy-4-(methylamino)-5-nitrobenzoic acid (150.0 mg, 0.663 mmol, 1.0 eq.) was dissolved in a mixed solvent of tetrahydrofuran (4 mL) and *N,N*-dimethylformamide (2 mL), and *N,N*-diisopropylethylamine (171.2 mg, 1.326 mmol, 2.0 eq.) and 2-(7-azabenzotriazol-1-yl)-*N,N,N',N'*- tetramethyluronium hexafluorophosphate (277.3 mg, 0.729 mmol, 1.1 eq.) were added under an ice bath. The mixture was stirred for 40 min under an ice bath, and then added with a solution of *tert-butyl* (6-azaspiro[2.5]octan -4-yl)carbamate (165.0 mg, 0.729 mmol, 1.1 eq.) in tetrahydrofuran (2 mL) dropwise. The resulting solution was then stirred at room temperature for 10 h. After the end of reaction, as detected by TLC, the reaction solution was concentrated and added with water (15 mL) and dichloromethane (30 mL) before liquid separation. The organic phase was dried with anhydrous sodium sulfate and concentrated to give a crude product, which was then separated in a silica gel column chromatography (200-300 mesh silica gel, dichloromethane:methanol = 40:1 v/v) to give a yellow solid product (200.3 mg, yield: 69.4%).

Step 2: Synthesis of *tert*-butyl (6-(2-(1-ethyl-1*H*-pyrrolo[2,3-*b*]pyridin-2-yl)-7-methoxy-1-methyl-1*H*-benzo[*d*]imidazole-5-carbonyl)-6-azaspiro[2.5]octan-4-yl)carbamate

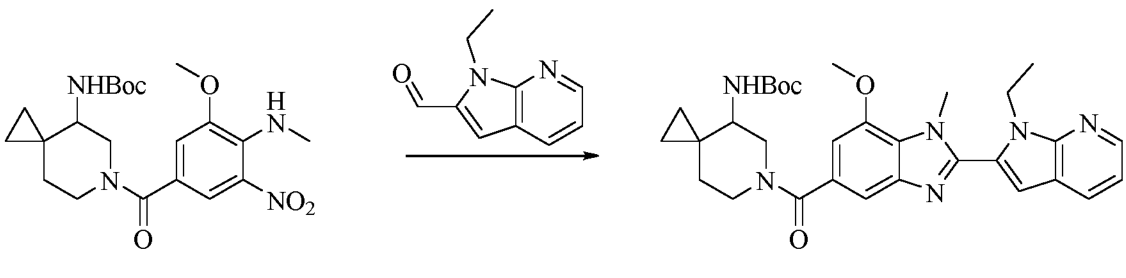

*Tert-butyl* (6-(3-methoxy-4-(methylamino)-5-nitrobenzoyl)-6-azaspiro [2.5]octan-4-yl)carbamate (200.0 mg, 0.46 mmol, 1.0 eq.) was dissolved in ethanol (8 mL), and the solution was then added with 1-ethyl-1*H*-pyrrolo[2,3-*b*] pyridine-2-carbaldehyde (80.1 mg, 0.46 mmol, 1.0 eq.), sodium dithionite (240.2 mg, 1.38 mmol, 3.0 eq.) and water (1 mL) to react at 100 °C for 5 h under microwaves. After the end of reaction, as detected by TLC, the reaction solution was concentrated under reduced pressure and added with water (10 mL) and dichloromethane (20 mL) before liquid separation. The organic phase was dried with anhydrous sodium sulfate, filtered and concentrated to give a crude product, which was then separated in a silica gel column chromatography (200-300 mesh silica gel, dichloromethane:methanol = 80:1 v/v) to give the target compound (135.1 mg, yield: 52.7%).

Step 3: Synthesis of (4-amino-6-azaspiro[2.5]octan-6-yl)(2-(1-ethyl-1*H*-pyrrolo[2,3-*b*]pyridin-2-yl)-7-methoxy-1-methyl-1*H*-benzo[*d*]imidazol-5-yl)methanone hydrochloride

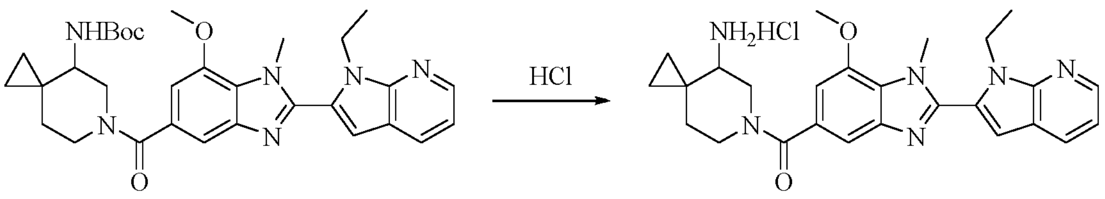


(Hydrochloride of Compound 16)

*Tert-butyl* (6-(2-(1-ethyl-1*H*-pyrrolo[2,3-*b*]pyridin-2-yl)-7-methoxy-1-methyl-1*H*-benzo[*d*]imidazole-5-carbonyl)-6-azaspiro[2.5]octan-4-yl)carbamate (121.7 mg, 0.217 mmol, 1.0 eq.) was dissolved in ethanol (4 mL), and a solution of hydrogen chloride in ethanol (30% w/w, 4 mL) was added dropwise. The mixture reacted at room temperature for 4 h. After the end of reaction, as detected by TLC, the reaction solution was concentrated to give a crude product, which was slurried with petroleum ether (4 mL) and methyl *tert*-butyl ether (2 mL) to give (4-amino-6-azaspiro[2.5]octan-6-yl)(2-(1-ethyl-1H-pyrrolo[2,3-*b*]pyridin-2-yl)-7-methoxy-1-methyl-1*H*-benzo[*d*]imidazol-5-yl)methanone hydrochloride (124.4 mg, yield: 100%).
$^1$HNMR (400MHz, DMSO-$d_6$) δ(ppm): 8.48 (d, *J* = 4.4 Hz, 1H), 8.37 (brs, 2H), 8.21 (d, *J* = 7.8 Hz, 1H), 7.59 (brs, 1H), 7.27-7.29 (m, 1H), 7.18 (s, 2H), 4.58-4.63 (m, 2H), 4.16 (s, 3H), 4.03 (s, 3H), 3.16-3.25 (m, 1H), 3.04-3.06 (m, 1H), 2.67-2.73 (m, 1H), 2.25-2.41 (m, 1H), 1.23-1.26 (m, 3H), 0.82-0.84 (m, 2H), 0.65-0.68 (m, 1H), 0.42-0.52 (m, 2H).
Molecular formula: $C_{26}H_{30}N_6O_2$, molecular weight: 458.57, LC-MS (Pos, *m/z)* = 459.3 $[M+H]^+$.

**Example 20: Synthesis of (*R*)-(3-aminopyrrolidin-1-yl)(2-(6-ethyl-6*H*-thieno[2,3-*b*]pyrrol-5-yl)-7-methoxy-1-methyl-1*H*-benzo[*d*]imidazol-5-yl)methanone (Compound 17)**

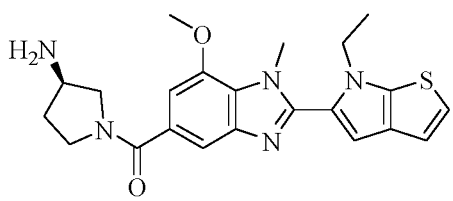

Step 1: Synthesis of *tert*-butyl (*R*)-(1-(3-methoxy-4-methylamino-5-nitrobenzoyl)pyrrolidin-3-yl)carbamate

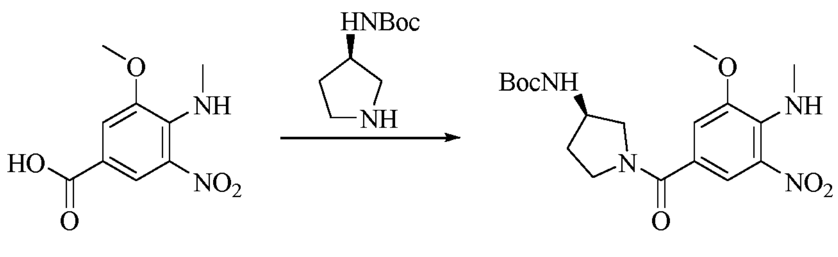

3-methoxy-4-(methylamino)-5-nitrobenzoic acid (500.0 mg, 2.21 mmol, 1.0 eq.) was added to DMF (5 mL), and the solution was cooled to 0 °C under an ice bath and added with HATU (1260.8 mg, 3.32 mmol, 1.5 eq.) and DIPEA (857.1 mg, 6.63 mmol, 3.0 eq.). The mixture was stirred at 0 °C for 60 min, and then added with *tert*-butyl (*R*)-pyrrolidin-3-ylcarbamate (370.5 mg, 1.99 mmol, 0.9 eq.). The resulting mixture was stirred at room temperature overnight. After the end of reaction, as detected by TLC, the reaction solution was concentrated under reduced pressure and added with ethyl acetate (10 mL). The organic phase was successively washed with saturated aqueous sodium bicarbonate

solution (10 mL), saturated aqueous ammonium chloride solution (10 mL), water (10 mL × 4) and saturated brine (10 mL), dried with anhydrous sodium sulfate, filtered under vacuum and concentrated under reduced pressure to give a crude product, which was then purified in a preparative thin-layer chromatography (PE:EA = 1:1, 0:1 v/v) to give the target compound (508.0 mg, yield: 64.7 %).

Step 2: Synthesis of *tert*-butyl (*R*)-(1-(2-(6-ethyl-6*H*-thieno[2,3-*b*]pyrrol-5-yl)-7-methoxy-1-methyl-1*H*-benzo[*d*]imidazole-5-carbonyl)pyrrolidin-3-yl) carbamate

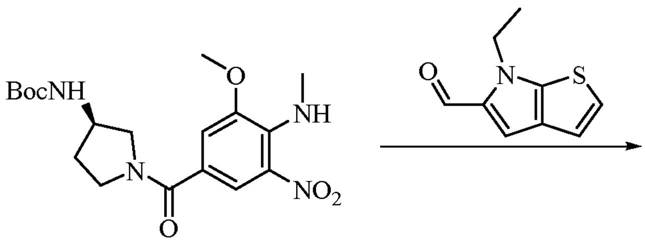

*Tert*-butyl (*R*)-(1-(3-methoxy-4-methylamino-5-nitrobenzoyl)pyrrolidin-3-yl)carbamate (306.0 mg, 0.78 mmol, 1.5 eq.) was dissolved in a mixed solution of water (2 mL) and ethanol (10 mL), and the resulting solution was then added with 6-ethyl-6*H*-thieno[2,3-*b*]pyrrole-5-carbaldehyde (92.7 mg, 0.52 mmol, 1.0 eq.) and $Na_2S_2O_4$ (270.2 mg, 1.55 mmol, 3.0 eq.) to react at 100 °C for 5 h under microwaves. After the end of reaction, as detected by TLC, the reaction solution was concentrated under reduced pressure and filtered. The filtrate was extracted with dichloromethane (5 mL × 3). The organic phases were combined, dried with anhydrous sodium sulfate and concentrated to give a crude product, which was then purified in a silica gel column chromatography (DCM:MeOH = 50:1-30:1 v/v) to give the target compound (250 mg, yield: 92.3 %).

Step 3: Synthesis of (*R*)-(3-aminopyrrolidin-1-yl)(2-(6-ethyl-6*H*-thieno [2,3-*b*]pyrrol-5-yl)-7-methoxy-1-methyl- 1*H*-benzo[*d*]imidazol-5-yl)methanone

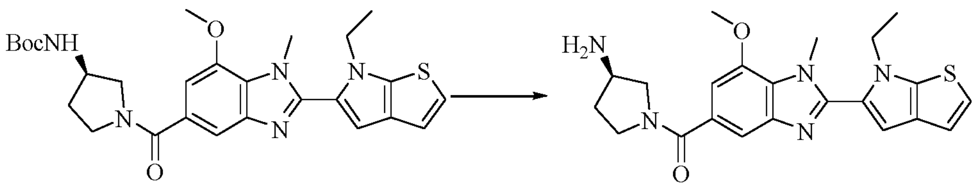

(Compound 17)

*Tert-butyl* (*R*)-(1-(2-(6-ethyl-6*H*-thieno[2,3-*b*]pyrrol-5-yl)-7-methoxy-1-methyl-1*H*-benzo[*d*]imidazole-5-carbonyl)pyrrolidin-3-yl)carbamate (245.0 mg, 0.47 mmol) was dissolved in a solution of hydrogen chloride in ethanol (30% w/w, 17 mL) under an ice bath, and the solution was stirred at room temperature for 2 h. After the end of reaction, as detected by TLC, the reaction solution was concentrated under reduced pressure. The solids were dissolved in water (10 mL), added with an aqueous solution (10% w/w) of $NaHCO_3$ to adjust the pH to 7-8, and extracted with dichloromethane (5 mL × 3). The organic phases were combined, dried with anhydrous sodium sulfate and filtered under vacuum. The filtrate was concentrated under reduced pressure to give a crude product, which was then purified in a preparative thin-layer chromatography (DCM:MeOH = 20:1, 10:1 v/v) to give the target compound (108 mg, yield: 54.2 %).
$^1$H NMR (400 MHz, DMSO-$d_6$) δ(ppm): 7.46-7.42 (d, 1H), 7.18-7.12 (q, 2H), 6.96-6.91 (d, 2H), 4.41-4.39 (d, 2H), 4.11 (s, 3H), 3.98 (s, 3H), 3.66-3.64 (d, 3H), 3.51 (s, 2H), 2.06-2.03 (d, 1H), 1.73-1.72 (d, 1H), 1.36-1.32 (t, 3H). Molecular formula: $C_{22}H_{25}N_5O_2S$, molecular weight: 423.54, LC-MS (Pos, *m/z)* = 424.38 $[M+H]^+$.

**Example 21: Synthesis of (*R*)-(3-aminopiperidin-1-yl)(2-(6-(cyclopropylmethyl)-6*H*-thieno[2,3-*b*]pyrrol-5-yl)-7-methoxy-1-methyl-1*H*-benzo[*d*]imidazol-5-yl)methanone (Compound 18)**

Step 1: Synthesis of ethyl 6*H*-thieno[2,3-*b*]pyrrole-5-carboxylate

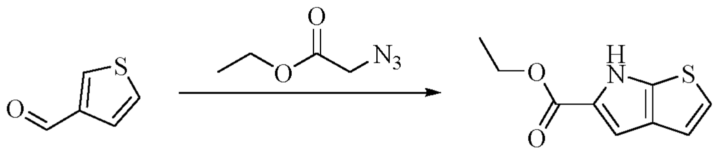

Metallic sodium (3.0 g, 130.0 mmol, 4.86 eq.) was added to absolute ethanol (60 mL) and stirred until dissolved, and a solution of thiophene-3-formaldehyde (3.0 g, 26.7 mmol, 1.0 eq.) and ethyl 2-azidoacetate (15.0 g, 116.0 mmol, 4.3 eq.) in ethanol (40 mL) was then added dropwise. The mixture was stirred at room temperature overnight. After the end of reaction, as detected by TLC, the reaction solution was added with saturated aqueous ammonium chloride solution (200 mL) under an ice bath, and extracted with methyl *tert*-butyl ether (200 mL × 3). The organic phase was dried with anhydrous sodium sulfate, concentrated under reduced pressure, added with dimethylbenzene (50 mL), and stirred at reflux overnight. After the end of reaction, as detected by TLC, the reaction solution was concentrated under reduced pressure to give a crude product, which was then purified in a silica gel column chromatography (petroleum ether:ethyl acetate = 5:1 v/v) to give the target compound (435.0 mg, yield: 8.3%).

Step 2: Synthesis of ethyl 6-(cyclopropylmethyl)-6*H*-thieno[2,3-*b*]pyrrole-5-carboxylate

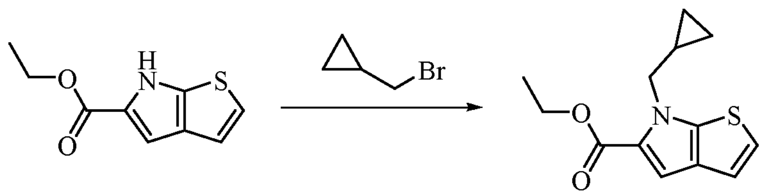

Ethyl 6*H*-thieno[2,3-*b*]pyrrole-5-carboxylate (435.0 mg, 2.22 mmol, 1.0 eq.) was dissolved in DMF (5 mL), and the solution was then added with (bromomethyl)cyclopropane (449.5 mg, 3.33 mmol, 1.5 eq.) and anhydrous potassium carbonate (613.6 mg, 4.44 mmol, 2.0 eq.) to react at 50 °C for 3 h. After the end of reaction, as detected by TLC, the reaction solution was concentrated under reduced pressure and added with ethyl acetate (15 mL) and water (40 mL) before liquid separation. The organic phase was dried with anhydrous sodium sulfate and concentrated to give a crude product, which was then purified in a silica gel column chromatography (petroleum ether:ethyl acetate = 100:1 v/v) to give the target compound (507.6 mg, yield: 91.7%).

Step 3: Synthesis of (6-(cyclopropylmethyl)-6*H*-thieno[2,3-*b*]pyrrol-5-yl) methanol

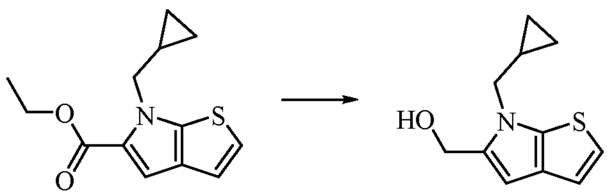

Ethyl 6-(cyclopropylmethyl)-6*H*-thieno[2,3-*b*]pyrrole-5-carboxylate (507.6 mg, 2.03 mmol, 1.0 eq.) was dissolved in THF (2.5 mL), and the solution was then added with a solution of diisobutyl aluminum hydride in toluene (1.5 mol/L, 4.66 mL) under an ice bath to react under an ice bath for 2 h. After the end of reaction, as detected by TLC, the

reaction solution was added with saturated aqueous sodium sulfate solution (20 mL), stirred for half an hour, and filtered under vacuum. The filtrate was dried with anhydrous sodium sulfate and concentrated under reduced pressure to give the target compound (430.9 mg of crude product).

Step 4: Synthesis of 6-(cyclopropylmethyl)-6*H*-thieno[2,3-*b*]pyrrole-5-carbaldehyde

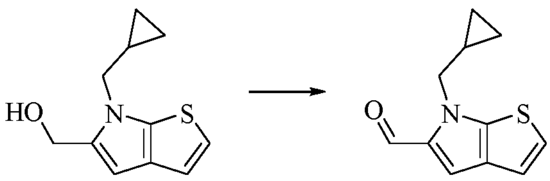

(6-(cyclopropylmethyl)-6*H*-thieno[2,3-*b*]pyrrol-5-yl)methanol (430.9 mg of crude product) was added to dichloromethane (12.5 mL), and the solution was then added with manganese dioxide (1.8 g, 20.3 mmol, 10.0 eq.) to react at 40 °C for 1 h. After the end of reaction, as detected by TLC, the reaction solution was filtered, and the filtrate was concentrated under reduced pressure to give the target compound (371.7 mg, two-step yield: 89.2%).

Step 5: Synthesis of *tert*-butyl (*R*)-(1-(2-(6-(cyclopropylmethyl)-6*H*-thieno [2,3-*b*]pyrrol-5-yl)-7-methoxy-1-methyl-1*H*-benzo[*d*]imidazole-5-carbonyl) piperidin-3-yl)carbamate

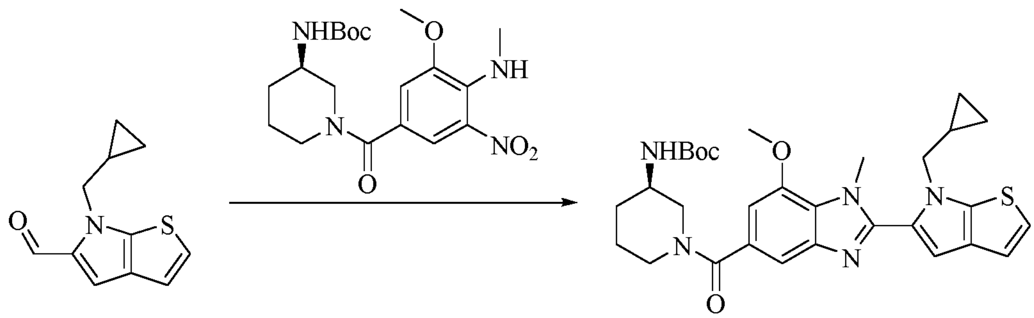

*Tert-butyl* (*R*)-(1-(3-methoxy-4-(methylamino)-5-nitrobenzoyl)piperidin-3-yl)carbamate (701.0 mg, 1.71 mmol, 1.0 eq.) was added to ethanol (10.0 mL), and the solution was then added with an aqueous solution (5 mL) of 6-(cyclopropylmethyl)-6*H*-thieno[2,3-*b*]pyrrole-5-carbaldehyde (371.7 mg, 1.81 mmol, 1.0 eq.) and sodium dithionite (945.3 mg, 5.43 mmol, 3.0 eq.) to react at 100 °C for 5 h under microwaves. After the end of reaction, as detected by TLC, the reaction solution was concentrated under reduced pressure and added with water (15 mL) and dichloromethane (20 mL) before liquid separation. The organic phase was dried with anhydrous sodium sulfate, filtered and concentrated to give a crude product, which was then purified in a silica gel column chromatography (dichloromethane:methanol = 100:1 to 80:1 v/v) to give the target compound (440.0 mg, yield: 44.0%).

Step 6: Synthesis of (*R*)-(3-aminopiperidin-1-yl)(2-(6-(cyclopropylmethyl)-6*H*-thieno[2,3-*b*]pyrrol-5-yl)-7-methoxy-1-methyl-1*H*-benzo[*d*]imidazol-5-yl)methanone

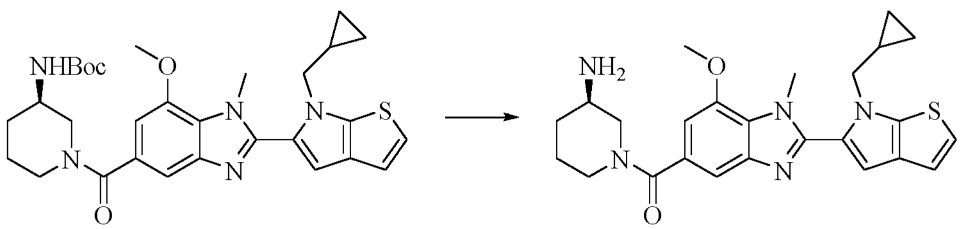

(Compound 18)

*Tert-butyl* (*R*)-(1-(2-(6-(cyclopropylmethyl)-6*H*-thieno[2,3-*b*]pyrrol-5-yl) -7-methoxy-1-methyl-1*H*-benzo[*d*]imidazole-5-carbonyl)piperidin-3-yl)carbamate (440.0 mg, 0.78 mmol, 1.0 eq.) was added to dichloromethane (2.0 mL) and stirred until dissolved. Trifluoroacetic acid (2.0 mL) was added dropwise under an ice bath, and the mixture was then stirred at room temperature for 2 h. After the end of reaction, as detected by TLC, saturated aqueous sodium bicarbonate solution (10 mL) was added dropwise under an ice bath to adjust the pH to 7-8, and dichloromethane (15 mL) was added for extraction. The organic phase was dried with anhydrous sodium sulfate and purified by a column (dichloromethane:methanol = 40:1-30:1-20:1 v/v) to give (*R*)-(3-aminopiperidin-1-yl)(2-(6-(cyclopropylmethyl)-6*H*-thieno[2,3-*b*]pyrrol-5-yl)-7-methoxy-1-methyl-1*H*-benzo[*d*]imidazol-5-yl)methanone, which was concentrated under reduced pressure to give the target compound (208.2 mg, yield: 57.6%).
$^1$HNMR (400MHz, DMSO-$d_6$) δ(ppm):7.25-7.26 (s, 1H), 7.15-7.16 (m, 1H), 7.10-7.11 (m, 1H), 6.90 (s, 1H), 6.82 (s, 1H), 4.24-4.26 (m, 2H), 4.09 (s, 3H), 3.97 (s, 3H), 2.92 (s, 1H), 2.67 (s, 2H), 1.85-1.88 (m, 1H), 1.58-1.67 (m, 3H), 1.39-1.46 (m, 1H), 1.19-1.25 (m, 2H), 0.45-0.48 (m, 2H), 0.26 (m, 2H).
Molecular formula: $C_{25}H_{29}N_5O_2S$, molecular weight: 463.60, LC-MS (Pos,*m*/*z*) = 464.38 $[M+H]^+$.

**Example 22: Synthesis of (*R*)-(3-aminoazepan-1-yl)(2-(6-ethyl-6*H*-thieno [2,3-*b*]pyrrol-5-yl)-7-methoxy-1-methyl-1*H*-benzo[*d*]imidazol-5-yl)methanone (Compound 19)**

Step 1: Synthesis of *tert*-butyl (*R*)-3-(((benzyloxy)carbonyl)amino)azepane-1-carboxylate

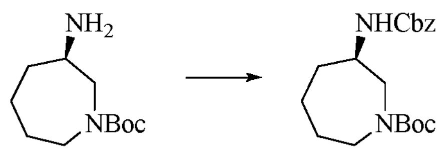

*Tert-butyl* (*R*)-3-aminoazepane-1-carboxylate (500.0 mg, 2.33 mmol, 1.0 eq.) and triethylamine (472.0 mg, 4.67 mmol, 2.0 eq.) were dissolved in dichloromethane (5.0 mL), and the solution was then added with benzyl chloroformate (517.0 mg, 3.03 mmol, 1.3 eq.) at 0 °C to react at room temperature in nitrogen atmosphere for 2 h. After the end of reaction, as detected by TLC, the reaction solution was concentrated under reduced pressure to give the target compound (1.0 g of crude product).

Step 2: Synthesis of benzyl (*R*)-azepan-3-yl carbamate

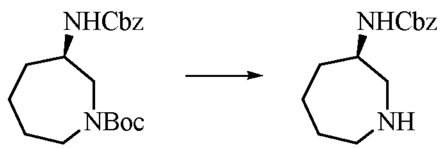

*Tert-butyl* (*R*)-3-(((benzyloxy)carbonyl)amino)azepane-1-carboxylate (1.0 g) was dissolved in ethanol (2.5 mL), and the solution was then added with a solution of hydrogen chloride in ethanol (30% w/w, 2.5 mL) to react at room temperature overnight. After the end of reaction, as detected by TLC, the reaction solution was concentrated under reduced pressure to give a crude product, which was then purified in a silica gel column chromatography (DCM: MeOH = 100: 1-20: 1 v/v) to give the target compound (200.0 mg, two-step yield: 34.54%).

Step 3: Synthesis of benzyl (*R*)-(1-(3-methoxy-4-(methylamino)-5-nitrobenzoyl) azeane-3-yl)carbamate

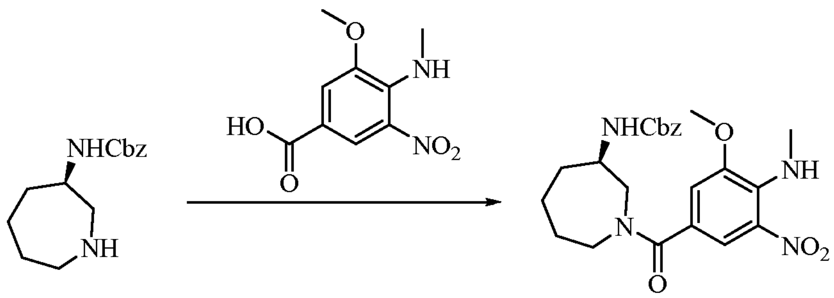

3-methoxy-4-(methylamino)-5-nitrobenzoic acid (200.0 mg, 0.805 mmol, 1.0 eq.) and DIPEA (312.0 mg, 2.42 mmol, 3.0 eq.) were dissolved in DMF (2.0 mL), and the solution was then added with HATU (459.0 mg, 1.21 mmol, 1.5 eq.) at 0 °C in nitrogen atmosphere, and stirred for 0.5 h. The mixture was added with benzyl (*R*)-azepan-3-ylcarbamate (200.0 mg, 0.805 mmol) to react at room temperature for 2 h. After the end of reaction, as detected by TLC, the reaction solution was added with water (40.0 mL) and extracted with ethyl acetate (15.0 mL × 2). The organic phases were combined, washed successively with saturated aqueous sodium carbonate solution (2.0 mL), saturated aqueous ammonium chloride solution (2.0 mL) and saturated sodium chloride solution (2.0 mL), dried and concentrated under reduced pressure to give a crude product, which was then purified in a silica gel column chromatography (DCM: MeOH = 200:1-100:1) to give the target compound (323.0 mg, yield: 87.9%).

Step 4: Synthesis of benzyl (*R*)-(1-(2-(6-ethyl-6*H*-thieno[2,3-*b*]pyrrol-5-yl)-7-methoxy-1-methyl-1*H*-benzo[*d*]imidazole-5-carbonyl)azepan-3-yl)carbamate

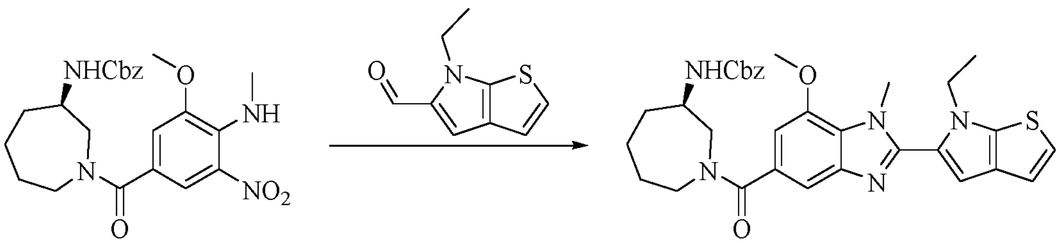

Benzyl (*R*)-(1-(3-methoxy-4-(methylamino)-5-nitrobenzoyl)azepan-3-yl) carbamate (300.0 mg, 0.657 mmol, 1.0 eq.), 6-ethyl-6*H*-thieno[2,3-*b*]pyrrole-5-carbaldehyde (118.0 mg, 0.657 mmol, 1.0 eq.) and sodium dithionite (344.0 mg, 1.98 mmol, 3.0 eq.) were dissolved in a mixed solution of ethanol (6.0 mL) and water (3.0 mL), and in nitrogen atmosphere, the resulting solution reacted at 100 °C for 5 h under microwaves. After the end of reaction, as detected by TLC, the reaction solution was concentrated under reduced pressure, added with ethyl acetate and filtered under vacuum. The filtrate was concentrated under reduced pressure to give a crude product, which was then purified in a silica gel column chromatography (DCM:MeOH = 100:1-60:1 v/v) to give the target compound (260.0 mg, yield: 67.5%).

Step 5: Synthesis of (*R*)-(3-aminoazepan-1-yl)(2-(6-ethyl-6*H*-thieno[2,3-*b*]pyrrol-5-yl)-7-methoxy-1-methyl-1*H*-benzo[*d*]imidazol-5-yl)methanone

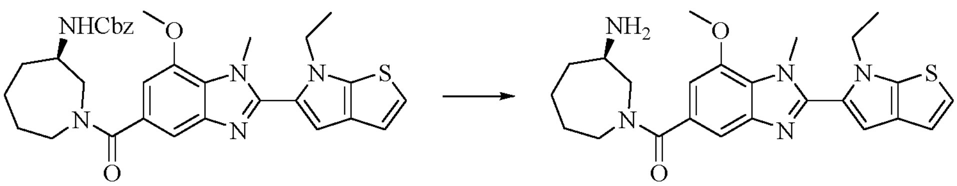

(Compound 19)

Benzyl (*R*)-(1-(2-(6-ethyl-6*H*-thieno[2,3-*b*]pyrrol-5-yl)-7-methoxy-1-methyl-1*H*-benzo[*d*]imidazole-5-carbonyl)azepan-3-yl)carbamate (340.0 mg, 0.580 mmol, 1.0 eq.) was dissolved in acetonitrile (8.0 mL), and in nitrogen atmosphere, the solution was added with trimethyliodosilane (290.0 mg, 1.45 mmol, 2.5 eq.) to react for 3 h. After the end of reaction, as detected by TLC, triethylamine (2.0 mL) was added to quench the reaction. The reaction solution was added with water (30.0 mL) and extracted with ethyl acetate (10.0 mL × 3). The organic phases were combined, washed successively with saturated aqueous sodium bicarbonate solution (2.0 mL) and saturated sodium chloride solution (2.0 mL), dried with anhydrous sodium sulfate and concentrated under reduced pressure to give a crude product, which was then purified in a silica gel column chromatography (DCM:MeOH = 100:1-11:1 v/v) to give the target compound (230.0 mg, yield: 87.8%).

$^1$H NMR (400 MHz, DMSO-$d_6$) δ(ppm): 7.24 (s, 1H), 7.18-7.16 (m, 1H), 7.13-7.11 (m, 1H), 6.90 (s, 1H), 6.80 (s, 1H), 4.43-4.37 (m, 2H), 4.10 (s, 3H), 3.97 (s, 3H), 3.41-3.17 (m, 4H), 2.98-2.81 (m, 2H), 1.85-1.72 (m, 4H), 1.36-1.32 (m, 4H). Molecular formula: $C_{24}H_{29}N_5O_2S$, molecular weight: 451.59, LC-MS (Pos, *m/z)* = 452.37 [M+H]$^+$.

**Example 23: Synthesis of (*R*)-(3-aminopiperidin-1-yl)(2-(6-isobutyl-6*H*-thieno[2,3-*b*]pyrrol-5-yl)-7-methoxy-1-methyl-1*H*-benzo[*d*]imidazol-5-yl)methanone (Compound 20)**

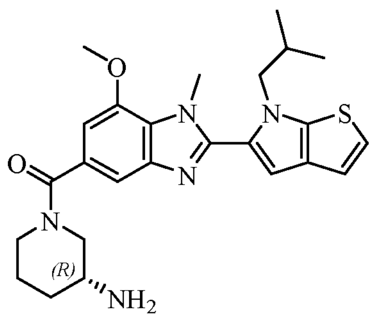

Step 1: Synthesis of intermediate ethyl 6-isobutyl-6*H*-thieno[2,3-*b*]pyrrole-5-carboxylate

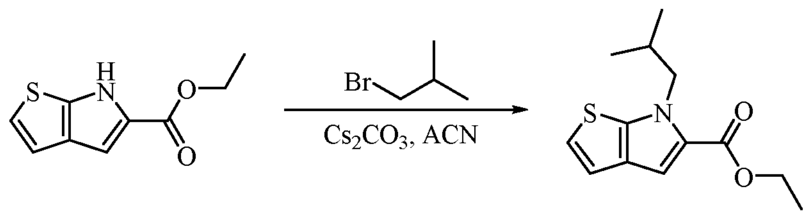


The intermediate ethyl 6*H*-thieno[2,3-*b*]pyrrole-5-carboxylate (1.50 g,7.68 mmol, 1.0 eq.), cesium carbonate (3.75 g, 11.52 mmol, 1.5 eq.) and 1-bromo-2-methylpropane (1.58 g, 11.52 mmol, 1.5 eq.) were successively added to acetonitrile (25 mL) and the mixture was then heated to 90 °C to react for 1.5 h. After the end of reaction, as detected by TLC, the reaction solution was cooled to room temperature and filtered. The filtrate was concentrated to give a crude product, which was then purified in a silica gel column chromatography (ethyl acetate:petroleum ether = 3:100 v/v) to give the target compound (1.52 g, yield: 78.7%).

Step 2: Synthesis of intermediate (6-isobutyl-6*H*-thieno[2,3-*b*]pyrrol-5-yl) methanol

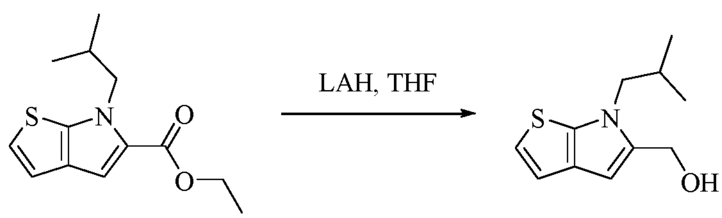

Ethyl 6-isobutyl-6*H*-thieno[2,3-*b*]pyrrole-5-carboxylate (1.50 g, 5.97 mmol, 1.0 eq.) was dissolved in THF (25 mL), and the solution was cooled to about 0 °C under an ice-water bath. Lithium aluminum hydride (227 mg, 5.97 mmol, 1.0 eq.) was added to the aforementioned solution and stirred for 0.5 h, and the resulting mixture reacted at room temperature for 2 h. After the end of reaction, as detected by TLC, the reaction solution was added successively with water (0.23 mL), 15% w/w sodium hydroxide solution (0.23 mL) and water (0.69 mL) to precipitate a large number of white flocculent solids, dried with anhydrous magnesium sulfate, and filtered. The filtrate was concentrated to give the target compound (1.15 g, yield: 92.1%).

Step 3: Synthesis of intermediate 6-isobutyl-6*H*-thieno[2,3-*b*]pyrrole-5-carbaldehyde

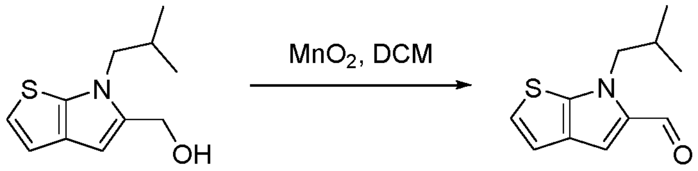


(6-isobutyl-6*H*-thieno[2,3-*b*]pyrrol-5-yl)methanol (500 mg, 2.39 mmol, 1.0 eq.) was added to dichloromethane (20 mL), and the solution was then added with manganese dioxide (2.08 g, 23.89 mmol, 10 eq.) and stirred at room temperature for 16 h. After the end of reaction, as detected by TLC, the reaction solution was filtered with celite. The filtrate was concentrated to give a brown oily product (390 mg, yield: 78.8%).

Step 4: Synthesis of intermediate *tert*-butyl (*R*)-(1-(2-(6-isobutyl-6*H*-thieno[2, 3-*b*]pyrrol-5-yl)-7-methoxy-1-methyl-1*H*-benzo[*d*]imidazole-5-carbonyl)piperidin-3-yl)carbamate

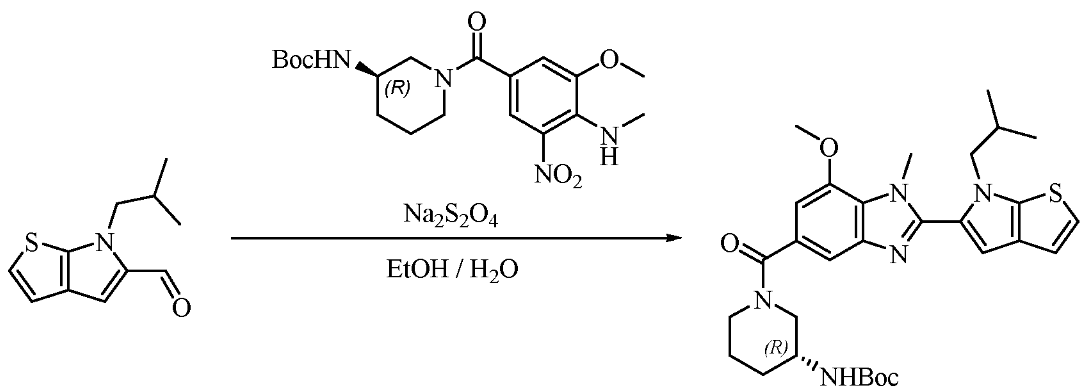


6-isobutyl-6*H*-thieno[2,3-*b*]pyrrole-5-carbaldehyde (365 mg, 1.76 mmol, 1.2 eq.), *tert-butyl* (*R*)-(1-(3-methoxy-4-(methylamino)-5-nitrobenzoyl) piperidin-3-yl)carbamate (600 mg, 1.47 mmol, 1.0 eq.) and sodium dithionite (1.28 g, 7.34 mmol, 5.0 eq.) were successively added to a mixed solvent of ethanol (15 mL) and water (7.5 mL), and the solution reacted at 90 °C for 10 h. After the end of reaction, as detected by TLC, the reaction solution was poured into water (50 mL) and extracted with ethyl acetate (30 mL). The organic phase was successively washed with water (20 mL) and saturated brine (20 mL), dried with $Na_2SO_4$ and filtered. The filtrate was concentrated to give a crude product, which was then purified in a silica gel column chromatography (dichloromethane:methanol = 50:1 v/v) to give the target compound (550 mg, yield: 66.2%).

Step 5: Synthesis of (*R*)-(3-aminopiperldin-1-yl)(2-(6-isobutyl-6*H*-thieno[2,3-*b*]pyrrol-5-yl)-7-methoxy-1-methyl-1*H*-benzo[*d*]imidazol-5-yl)methanone

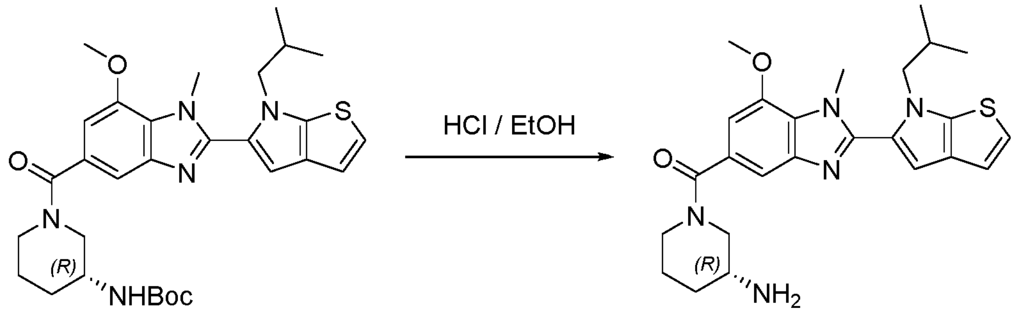


The intermediate *tert*-butyl (*R*)-(1-(2-(6-isobutyl-6*H*-thieno[2,3-*b*]pyrrol - 5-yl)-7-methoxy-1-methyl-1*H*-benzo[*d*]imidazole-5-carbonyl)piperidin-3-yl)carbamate (300 mg, 0.53 mmol, 1.0 eq.) and phenol (149.72 mg, 1.59 mmol, 3.0 eq.) were dissolved in methanol (5 mL), and the solution was then added with a solution of hydrogen chloride in ethanol (5 mL) and stirred at room temperature for 2 h. After the end of reaction, as detected by TLC, the reaction solution was concentrated and added with dichloromethane (20 mL). The organic phase was successively washed with saturated aqueous sodium bicarbonate solution (15 mL), water (15 mL) and saturated brine (15 mL), dried with $Na_2SO_4$, and filtered. The filtrate was concentrated to give a crude product, which was then purified in a silica gel column chromatography (dichloromethane:methanol = 50:1 to 10:1 v/v) to give the target compound (130 mg, yield: 52.7%).
$^1$H NMR (400 MHz, DMSO-$d_6$) δ(ppm): 7.27 (m, 1H), 7.16 (d, 1H), 7.11 (d, 1H), 6.91 (s, 1H), 6.82 (s, 1H), 4.10-4.40 (m, 3H), 4.09 (s, 3H), 3.97 (s, 3H), 3.60-3.80 (m, 1H), 2.90-3.00 (m, 1H), 2.60-2.75 (m, 2H), 2.00-2.30 (m, 3H), 1.80-1.95 (m, 1H), 1.60-1.75 (m, 1H), 1.40-1.50 (m, 1H), 1.20-1.35 (m, 1H), 0.71 (d, 6H).
Molecular formula: $C_{25}H_{31}N_5O_2S$, molecular weight: 465.62, LC-MS (*m*/*z*) = 465.94 $[M+H]^+$.

**Example 24: Synthesis of (*R*)-(3-aminopiperidin-1-yl)(7-methoxy-1-methyl-2-(6-(2,2,2-trifluoroethyl)-6*H*-thieno[2,3-*b*]pyrrol-5-yl)-1*H*-benzo[*d*]imidazol-5-yl)methanone (Compound 21)**

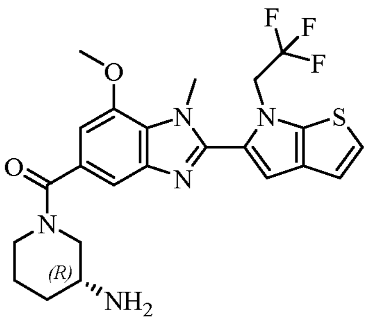

Step 1: Synthesis of 2,2,2-trifluoroethyl 4-methylbenzenesulfonate

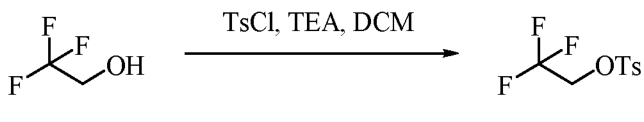


2,2,2-trifluoroethan-1-ol (5.77 g, 57.70 mmol, 1.1 eq.), *p*-toluenesulfonyl chloride (10.0 g, 52.45 mmol, 1.0 eq.) and triethylamine (10.62 g, 104.91 mmol, 2.0 eq.) were dissolved in dichloromethane (150 mL), and the solution was stirred at room temperature for 18 h. The reaction was completed. After washing with water (40 mL), the organic phase was dried and concentrated to give a crude product, which was then purified in a silica gel column chromatography (petroleum ether:ethyl acetate = 30:1 v/v) to give a colorless oily product (4.7 g, yield: 34.8%).

Step 2: Synthesis of ethyl 6-(2,2,2-trifluoroethyl)-6*H*-thieno[2,3-*b*]pyrrole-5-carboxylate

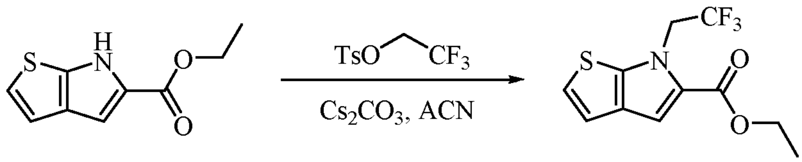


Ethyl 6*H*-thieno[2,3-*b*]pyrrole-5-carboxylate (500 mg, 2.56 mmol, 1.0 eq.), 2,2,2-trifluoroethyl 4-methylbenzenesulfonate (846 mg, 3.33 mmol, 1.3 eq.) and cesium carbonate (1.25 g, 3.84 mmol, 1.5 eq.) were added to acetonitrile (15 mL), and the mixture was heated to reflux for 35 h. After the end of reaction, as detected by TLC, the reaction solution was filtered. The filtrate was concentrated to give a crude product, which was then purified in a silica gel column chromatography (petroleum ether:ethyl acetate = 50:1 v/v) to give the target compound (580 mg, yield: 81.8%).

Step 3: Synthesis of (6-(2,2,2-trifluoroethyl)-6*H*-thieno[2,3-*b*]pyrrol-5-yl) methanol

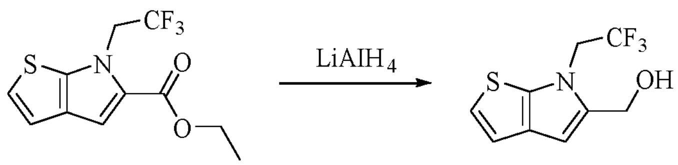


Lithium aluminum hydride (79.39 mg, 2.09 mmol, 1.0 eq.) was added to tetrahydrofuran (20 mL), and the mixture was cooled to 0 °C. Ethyl 6-(2,2,2-trifluoroethyl)-6*H*-thieno[2,3-*b*]pyrrole-5-carboxylate (580 mg, 2.09 mmol, 1.0 eq.) was dissolved in tetrahydrofuran (4 mL), and then the solution was slowly added to the suspension of lithium aluminum hydride in tetrahydrofuran, with the temperature of the reaction system maintained at about 0 °C. The mixture was then stirred for 2 h. After the end of reaction, as detected by TLC, the reaction solution was successively and slowly added with water (80 mg), 15% w/w aqueous sodium hydroxide solution (80 mg) and water (240 mg), dried with anhydrous sodium sulfate overnight, and filtered. The filtrate was concentrated to give the target compound (490 mg, yield: 99.6%).

Step 4: Synthesis of 6-(2,2,2-trifluoroethyl)-6*H*-thieno[2,3-*b*]pyrrole-5-carbaldehyde

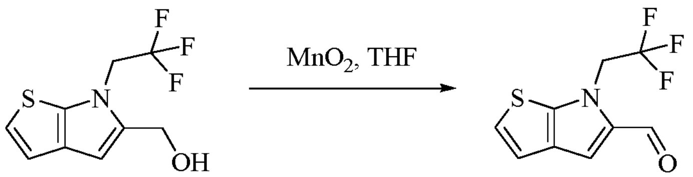


(6-(2,2,2-trifluoroethyl)-6*H*-thieno[2,3-*b*]pyrrol-5-yl)methanol (250 mg, 1.06 mmol, 1.0 eq.) was dissolved in tetrahydrofuran (4 mL), and the solution was added with manganese dioxide (1.0 g, 10.6 mmol, 10.0 eq.) and stirred at room temperature for 18 h. The reaction solution was filtered. The filtrate was concentrated to give the target compound (240 mg of crude product), which was directly used in the next step.

Step 5: Synthesis of *tert*-butyl (*R*)-(1-(7-methoxy-1-methyl-2-(6-(2,2,2-trifluoroethyl)-6*H*-thieno[2,3-*b*]pyrrol-5-yl)-1*H*-benzo[*d*]imidazole-5-carbonyl)piperidin-3-yl)carbamate

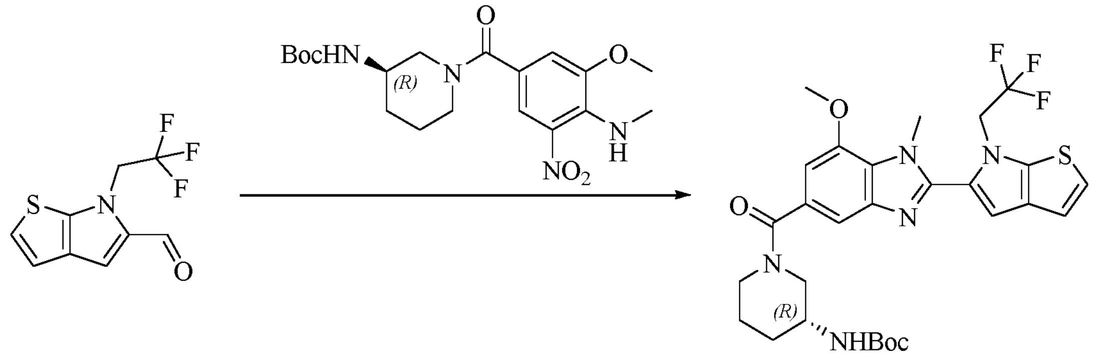

6-(2,2,2-trifluoroethyl)-6*H*-thieno[2,3-*b*]pyrrole-5-carbaldehyde (240 mg of crude product) and *tert*-butyl (*R*)-(1-(3-methoxy-4-(methylamino)-5-nitrobenzoyl)piperidin-3-yl)carbamate (324 mg, 0.79 mmol) were dissolved in ethanol (4 mL), and sodium dithionite (748 mg, 4.3 mmol) was dissolved in water (2 mL). After the two solutions were mixed, the mixture was stirred at 90 °C for 4 h. The reaction was completed. The reaction solution was concentrated and added with ethyl acetate (50 mL) and water (10 mL). The organic phase was washed with water (10 mL × 2), dried with anhydrous sodium sulfate, filtered and concentrated to give a crude product, which was then purified in a silica gel column chromatography (dichloromethane: methanol = 60:1 v/v) to give the target compound (380 mg, yield: 80.9 %).

Step 6: Synthesis of (*R*)-(3-aminopiperidin-1-yl)(7-methoxy-1-methyl-2-(6-(2,2,2-trifluoroethyl)-6*H*-thieno[2,3-*b*]pyrrol-5-yl)-1*H*-benzo[*d*]imidazol-5-yl)methanone

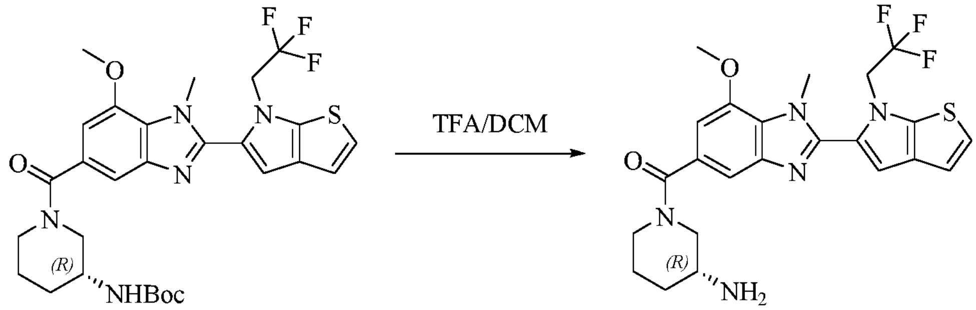


*Tert-butyl* (*R*)-(1-(7-methoxy-1-methyl-2-(6-(2,2,2-trifluoroethyl)-6*H*-thieno[2,3-*b*]pyrrol-5-yl)-1*H*-benzo[*d*]imidazole-5-carbonyl)piperidin-3-yl)carbamate (380 mg, 0.64 mmol, 1.0 eq.) was dissolved in a mixed solvent of dichloromethane (2 mL) and trifluoroacetic acid (2 mL), and the solution was added with phenol (302 mg, 3.2 mmol, 5.0 eq.) and stirred at room temperature for 10 min. After the end of reaction, as detected by TLC, the reaction solution was concentrated at low temperature, added with dichloromethane (100 mL), and washed with 15% w/w aqueous sodium hydroxide solution (20 mL × 2). The organic phase was dried, concentrated, added with ethyl acetate (0.5 mL) and petroleum ether (1 mL) to precipitate solids, and filtered to give the target compound (230 mg, yield: 73.2%).
$^1$H NMR (400 MHz, $CDCl_3$) δ(ppm): 7.41 (s, 1H), 7.08 (d, *J* = 5.28 Hz, 1H), 7.01 (d, *J* = 5.28 Hz, 1H), 6.86 (s, 1H), 6.83 (s, 1H), 5.23-5.39 (m, 2H), 4.18 (s, 3H), 4.02 (s, 3H), 2.72-3.15 (m, 4H), 2.07 (s, 1H), 1.42-1.89 (m, 4H). Molecular formula: $C_{23}H_{24}F_3N_5O_2S$, molecular weight: 491.16, LC-MS (Pos, m/z) = 492.23 $[M+H]^+$.

**Example 25: Synthesis of (*R*)-(3-aminopiperidin-1-yl)(2-(6-(cyclobutylmethyl)-6*H*-thieno[2,3-*b*]pyrrol-5-yl)-7-methoxy-1-methyl-1*H*-benzo[*d*]imidazol-5-yl)methanone (Compound 22)**

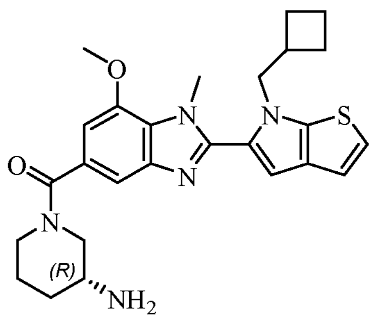

Step 1: Synthesis of intermediate ethyl 6-(cyclobutylmethyl)-6*H*-thieno[2,3-*b*] pyrrole-5-carboxylate

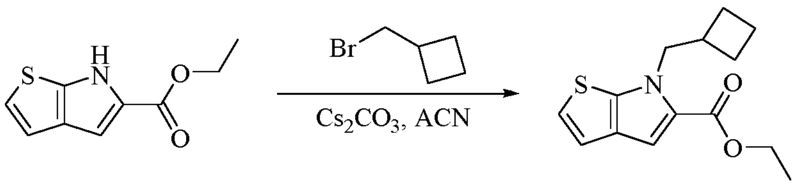


Ethyl 6*H*-thieno[2,3-*b*]pyrrole-5-carboxylate (1.50 g, 7.68 mmol, 1.0 eq.), cesium carbonate (3.75 g, 11.52 mmol, 1.5 eq.) and (bromomethyl) cyclobutane (1.72 g, 11.52 mmol, 1.5 eq.) were successively added to acetonitrile (25 mL) and the mixture reacted at 90 °C for 1.5 h. After the end of reaction, as detected by TLC, the reaction solution was cooled to room temperature and filtered. The filtrate was concentrated to give a crude product, which was then purified in a silica gel column chromatography (ethyl acetate:petroleum ether = 3:100 v/v) to give the target compound (1.45 g, yield: 71.7%).

Step 2: Synthesis of intermediate (6-(cyclobutylmethyl)-6*H*-thieno[2,3-*b*] pyrrol-5-yl)methanol

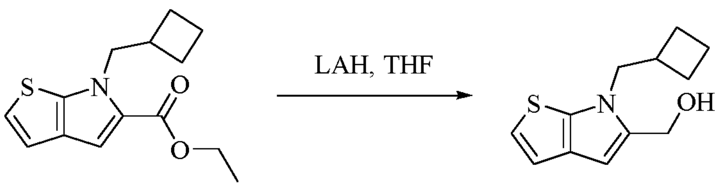


Ethyl 6-(cyclobutylmethyl)-6*H*-thieno[2,3-*b*]pyrrole-5-carboxylate (1.45 g, 5.51 mmol, 1.0 eq.) was dissolved in THF (25 mL), and the solution was cooled to about 0 °C under an ice-water bath. Lithium aluminum hydride (209 mg, 5.51 mmol, 1.0 eq.) was added into the aforementioned solution and stirred for 0.5 h, and the resulting mixture reacted at room temperature for 2 h. After the end of reaction, as detected by TLC, the reaction solution was successively added with water (0.21 mL), 15% w/w sodium hydroxide solution (0.21 mL) and water (0.63 mL) to precipitate a large number of white flocculent solids, dried with anhydrous magnesium sulfate, and filtered. The filtrate was concentrated to give the target compound (1.2 g, yield: 98.5%).

Step 3: Synthesis of intermediate 6-(cyclobutylmethyl)-6*H*-thieno[2,3-*b*] pyrrole-5-carbaldehyde

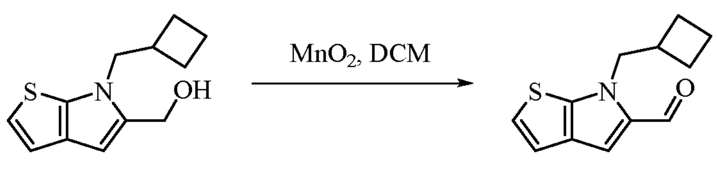

(6-(cyclobutylmethyl)-6*H*-thieno[2,3-*b*]pyrrol-5-yl)methanol (500 mg, 2.26 mmol, 1.0 eq.) was added to dichloromethane (20 mL), and the solution was then added with manganese dioxide (1.96 g, 22.59 mmol, 10 eq.) and stirred at room temperature for 15 h. After the end of reaction, as detected by TLC, the reaction solution was filtered. The filtrate was concentrated to give the target compound (420 mg, yield: 84.8%).

Step 4: Synthesis of intermediate *tert*-butyl (*R*)-(1-(2-(6-(cyclobutylmethyl)-6*H*-thieno[2,3-*b*]pyrrol-5-yl)-7-methoxy-1-methyl-1*H*-benzo[*d*]imidazole-5-carbonyl)piperidin-3-yl)carbamate

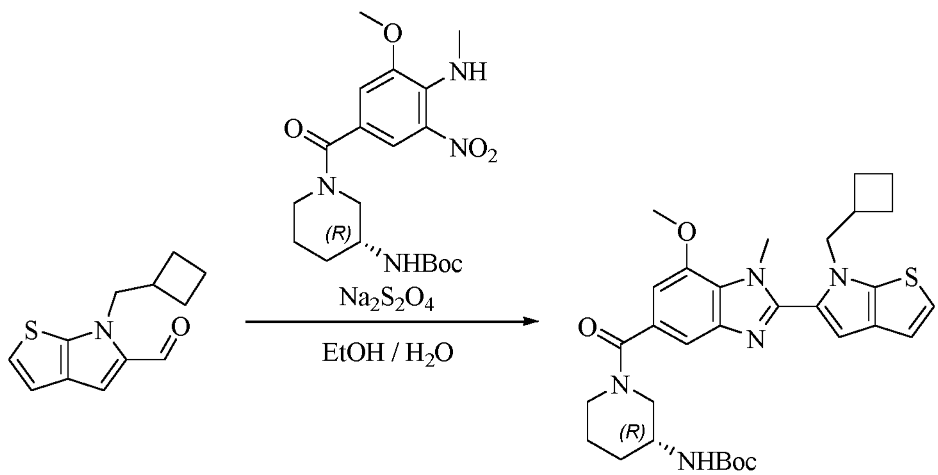


6-(cyclobutylmethyl)-6*H*-thieno[2,3-*b*]pyrrole-5-carbaldehyde (387 mg, 1.76 mmol, 1.2 eq.), *tert*-butyl (*R*)-(1-(3-methoxy-4-(methylamino)-5-nitrobenzoyl)piperidin-3-yl)carbamate (600 mg, 1.47 mmol, 1.0 eq.) and sodium dithionite (1.28 g, 7.34 mmol, 5.0 eq.) were successively added to a mixed solvent of ethanol (15 mL) and water (7.5 mL), and the solution reacted at 90 °C for 8 h. After the end of reaction, as detected by TLC, the reaction solution was poured into water (30 mL) and extracted with ethyl acetate (30 mL). The organic phase was successively washed with water (20 mL) and saturated brine (20 mL), dried with $Na_2SO_4$, and filtered. The filtrate was concentrated to give a crude product, which was then purified in a silica gel column chromatography (dichloromethane: methanol = 50:1 v/v) to give the target compound (540 mg, yield: 63.6%).

Step 5: Synthesis of (*R*)-(3-aminopiperidin-1-yl)(2-(6-(cyclobutylmethyl)-6*H*-thieno[2,3-*b*]pyrrol-5-yl)-7-methoxy-1-methyl-1*H*-benzo[*d*]imidazol-5-yl) methanone

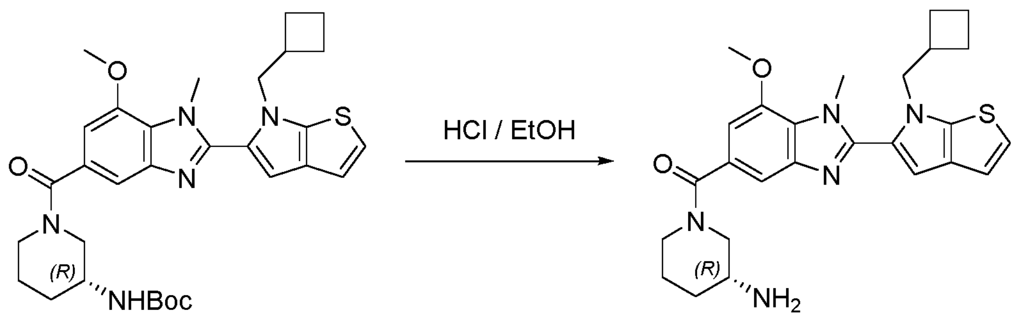


*Tert-butyl* (*R*)-(1-(2-(6-(cyclobutylmethyl)-6*H*-thieno[2,3-*b*]pyrrol-5-yl)-7-methoxy-1-methyl-1*H*-benzo[*d*]imidazole-5-carbonyl)piperidin-3-yl)carbamate (300 mg, 0.52 mmol, 1.0 eq.) and phenol (146.61 mg, 1.56 mmol, 3.0 eq.) were dissolved in methanol (5 mL), and the solution was added with a solution of hydrogen chloride in ethanol (30% w/w, 5 mL) and stirred at room temperature for 2 h. After the end of reaction, as detected by TLC, the reaction solution was concentrated and added with dichloromethane (20 mL). The organic phase was successively washed with saturated aqueous sodium bicarbonate solution (15 mL), water (15 mL) and saturated brine (15 mL), dried with $Na_2SO_4$, and filtered. The filtrate was concentrated to give a crude product, which was then purified in a silica gel column chromatography (dichloromethane:methanol = 50:1 to 10:1 v/v) to give the target compound (135 mg, yield: 54.4%).
$^1$H NMR (400 MHz, DMSO-$d_6$) δ(ppm): 7.28 (m, 1H), 7.16 (d, 1H), 7.10 (d, 1H), 6.89 (s, 1H), 6.82 (s, 1H), 4.10-4.40 (m, 3H), 4.09 (s, 3H), 3.97 (s, 3H), 3.60-3.80 (m, 1H), 2.90-3.00 (m, 1H), 2.60-2.75 (m, 2H), 2.00-2.30 (m, 2H), 1.60-1.95 (m, 6H), 1.40-1.55 (m, 3H), 1.20-1.30 (m, 2H).
Molecular formula: $C_{26}H_{31}N_5O_2S$, molecular weight: 477.63, LC-MS (*m*/*z*) = 477.93 [M+H]$^+$.

**Example 26: Synthesis of (*R*)-(3-aminopiperidin-1-yl)(2-(6-(cyclopropylmethyl)-2-methyl-6*H*-thieno[2,3-*b*]pyrrol-5-yl)-7-methoxy-1-methyl-1*H*-benzo[*d*]imidazol-5-yl)methanone (Compound 23)**

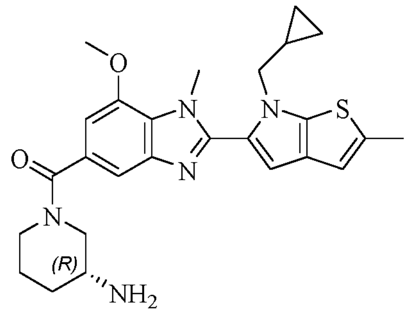

Step 1: Synthesis of 5-methylthiophene-3-carbaldehyde

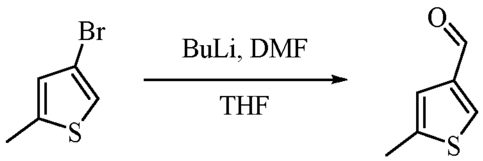


4-bromo-2-methylthiophene (20.0 g, 0.11 mol, 1.0 eq.) was dissolved in tetrahydrofuran (200 mL), and *n*-butyllithium (1.6 mol/L, 76.0 mL, 0.12 mol, 1.1 eq.) was slowly added dropwise at -80 °C in nitrogen atmosphere. The mixture was then stirred for 10 min. DMF (12.4 g, 0.17 mol, 1.5 eq.) was dissolved in tetrahydrofuran (10 mL), and the solution was added dropwise to the reaction solution at -75 °C. The reaction solution was then slowly warmed to room temperature, added with saturated aqueous ammonium chloride solution (30 mL) under an ice bath, and extracted with ethyl acetate (200 mL × 2). The organic phases were combined, dried, filtered and concentrated to give a crude product, which was then purified in a silica gel column chromatography (petroleum ether:ethyl acetate = 100:1 v/v) to give the target compound (3.5 g, yield: 24.7%).
$^1$HNMR (400 MHz, $CDCl_3$) δ(ppm): 9.81 (s, 1H), 7.89 (s, 1H), 7.20 (s, 1H), 2.51 (s, 3H).
Molecular formula: $C_6H_6OS$, molecular weight: 126.01, LC-MS (Pos, *m*/*z)* = 127.23 [M+H]$^+$.

Step 2: Synthesis of ethyl (Z) 2-azido-3-(5-methylthiophen-3-yl)acrylate

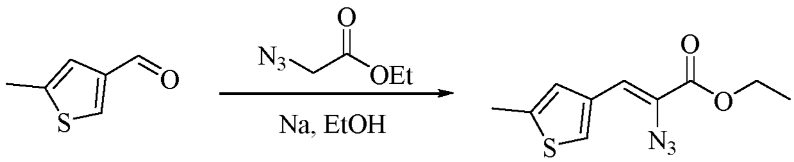


Sodium (2.5 g, 110 mmol, 4.0 eq.) was dissolved in ethanol (100 mL) to prepare a sodium ethoxide solution, which was cooled to -5 °C. 5-methylthiophen-3-carbaldehyde (3.5 g, 28 mmol, 1.0 eq.) and ethyl 2-azidoacetate (14.3 g, 110 mmol, 4.0 eq.) were dissolved in ethanol (20 mL), and the solution was slowly added dropwise to the newly

prepared sodium ethoxide solution with the temperature maintained at about -3 °C. The mixture was then stirred for 1 h at below 0 °C. Saturated aqueous ammonium chloride solution (30 mL) was added, and the reaction solution was poured into ice water, stirred for 10 min, and filtered. The crude product was dissolved in ethyl acetate, and the solution was dried and concentrated to give the target compound (4.0 g, yield: 60.7%).

Step 3: Synthesis of ethyl 2-methyl-6*H*-thieno[2,3-*b*]pyrrole-5-carboxylate

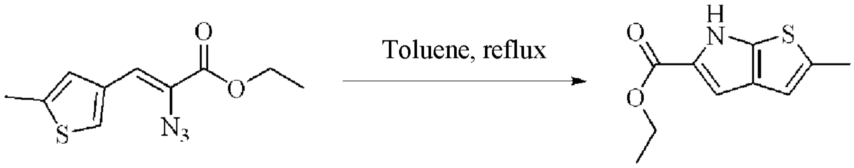


Ethyl (Z) 2-azido-3-(5-methylthiophen-3-yl)acrylate (4.0 g, 17 mmol, 1.0 eq.) was dissolved in toluene (110 mL), and the solution was refluxed for 1 h. After the end of reaction, as detected by TLC, the reaction solution was concentrated to give a crude product, which was then purified in a silica gel column chromatography (PE:EA = 30:1 v/v) to give the target compound (3.5 g, yield: 99.2%).
$^1$HNMR (400 MHz, $CDCl_3$) δ(ppm): 9.22 (s, 1H), 6.95 (s, 1H), 6.67 (s, 1H), 4.30-4.41 (m, 2H), 2.51 (s, 3H), 1.42-1.37 (m, 3H).

Step 4: Synthesis of ethyl 6-(cyclopropylmethyl)-2-methyl-6*H*-thieno[2,3-*b*] pyrrole-5-carboxylate

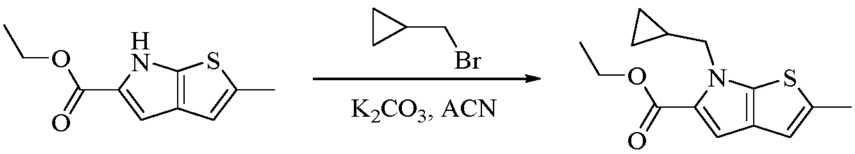


Ethyl 2-methyl-6*H*-thieno[2,3-*b*]pyrrole-5-carboxylate (3.0 g, 14.35 mmol, 1.0 eq.), cesium carbonate (11.4 g, 35 mmol, 2.5 eq.) and cyclopropylmethyl bromide (2.5 g, 19 mmol, 1.3 eq.) were dissolved in acetonitrile (100 mL), and the solution was refluxed for 1 h. After the end of reaction, as detected by TLC, the reaction solution was concentrated and added with water (30 mL) and ethyl acetate (200 mL). The organic phase was dried and concentrated to give a crude product, which was then purified in a silica gel column chromatography (petroleum ether: ethyl acetate = 100:1 v/v) to give the target compound (3.5 g, yield: 92.6%).
$^1$H NMR (400 MHz, $CDCl_3$) δ(ppm): 7.06 (s, 1H), 6.67 (s, 1H), 4.29-4.36 (m, 4H), 2.51 (s, 3H), 1.42-1.37 (m, 4H), 0.55-0.58 (m, 2H), 0.45-0.48 (m, 2H). Molecular formula: $C_{14}H_{17}NO_2S$, molecular weight: 263.10, LC-MS (Pos, *m*/*z*) = 264.23 $[M+H]^+$.

Step 5: Synthesis of (6-(cyclopropylmethyl)-2-methyl-6*H*-thieno[2,3-*b*]pyrrol-5-yl)methanol

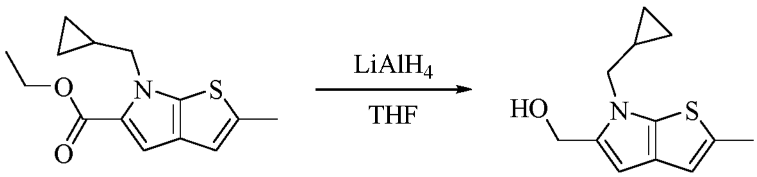


Lithium aluminum hydride (0.78 g, 20.5 mmol, 1.5 eq.) was suspended in tetrahydrofuran (40 mL), and the suspension was cooled to 0 °C. Ethyl 6-(cyclopropylmethyl)-2-methyl-6*H*-thieno[2,3-*b*]pyrrole-5-carboxylate (3.6 g, 13.67 mmol, 1.0 eq.) was dissolved in tetrahydrofuran (20 mL), and then the solution was slowly added to the suspension, with the temperature of the reaction system maintained at about 0 °C. The mixture was then stirred for 2 h. After the

end of reaction, as detected by TLC, the reaction solution was successively and slowly added with water (1.3 g), 15% w/w aqueous sodium hydroxide solution (1.3 g) and water (3.9 g), dried with anhydrous sodium sulfate, and filtered. The filtrate was concentrated to give the target compound (3 g, yield: 99.1%).

Step 6: Synthesis of 6-(cyclopropylmethyl)-2-methyl-6*H*-thieno[2,3-*b*] pyrrol-5-carbaldehyde

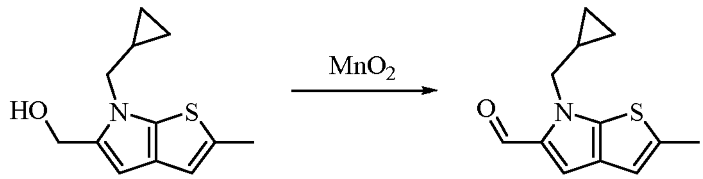


(6-(cyclopropylmethyl)-2-methyl-6*H*-thieno[2,3-*b*]pyrrol-5-yl)methanol (200 mg, 0.9 mmol, 1.0 eq.) was dissolved in tetrahydrofuran (3 mL), and the solution was added with manganese dioxide (787 mg, 9.0 mmol, 10.0 eq.) and stirred at room temperature for 18 h. After the end of reaction, as detected by TLC, the reaction solution was filtered. The filtrate was concentrated to give the target compound (280 mg of crude product), which was directly used in the next step.

Step 7: Synthesis of *tert*-butyl (*R*)-(1-(2-(6-(cyclopropylmethyl)-2-methyl-6*H*-thieno[2,3-*b*]pyrrol-5-yl)-7-methoxy-1-methyl-1*H*-benzo[*d*]imidazole-5-carbonyl)piperidin-3-yl)carbamate

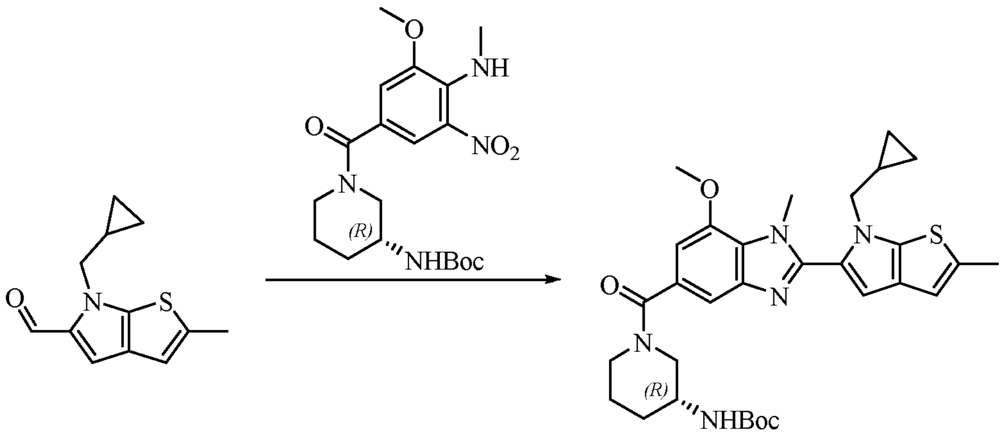

6-(cyclopropylmethyl)-2-methyl-6*H*-thieno[2,3-*b*]pyrrole-5-carbaldehyde (280 mg of crude product) and *tert*-butyl (*R*)-(1-(3-methoxy-4-(methylamino)-5-nitrobenzoyl)piperidin-3-yl)carbamate (401 mg, 0.98 mmol) were dissolved in ethanol (4 mL), and sodium dithionite (870 mg, 5.0 mmol) was dissolved in water (2 mL). After the two solutions were mixed, the mixture reacted at 90 °C for 4 h. After the end of reaction, as detected by TLC, the reaction solution was concentrated and added with ethyl acetate (50 mL) and water (10 mL). The organic phase was dried and concentrated to give a crude product, which was then purified in a silica gel column chromatography (dichloromethane: methanol = 50:1 v/v) to give the target compound (450 mg, yield: 79.6%).

Step 8: Synthesis of (*R*)-(3-aminopiperidin-1-yl)(2-(6-(cyclopropylmethyl)-2-methyl-6*H*-thieno[2,3-*b*]pyrrol-5-yl)-7-methoxy-1-methyl-1*H*-benzo[*d*]imidazol-5-yl)methanone

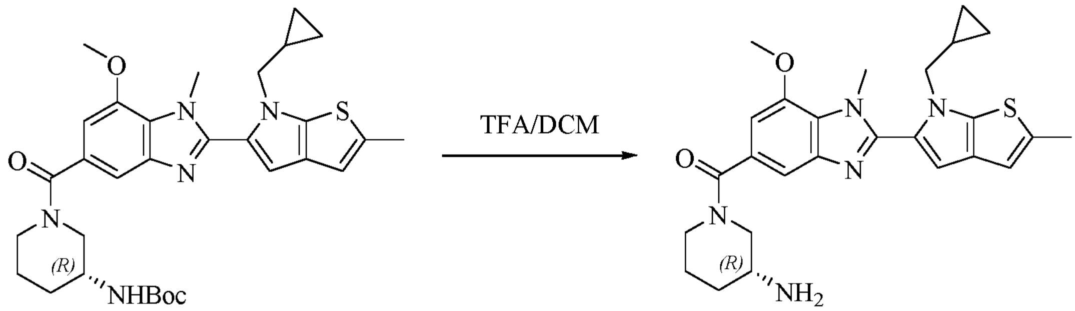


*Tert*-butyl (*R*)-(1-(2-(6-(cyclopropylmethyl)-2-methyl-6*H*-thieno[2,3-*b*] pyrrol-5-yl)-7-methoxy-1-methyl-1*H*-benzo[*d*]imidazol-5-carbonyl)piperidin-3-yl)carbamate (420 mg, 0.73 mmol, 1.0 eq.) was dissolved in a mixed solvent of dichloromethane (4 mL) and trifluoroacetic acid (4 mL), and the solution was added with phenol (342 mg, 3.64 mmol, 5.0 eq.) and stirred at room temperature for 10 min. After the end of reaction, as detected by TLC, the reaction solution was concentrated, added with dichloromethane (100 mL), and washed with 15% w/w sodium hydroxide (20 mL × 2). The organic phase was dried, concentrated, added with ethyl acetate (0.5 mL) and petroleum ether (1 mL) to precipitate solids, and filtered to give the target compound (250 mg, yield: 71.8%).

$^1$H NMR (400 MHz, $CDCl_3$) δ(ppm): 7.40 (s, 1H), 6.85 (s, 1H), 6.72 (s, 1H), 6.60 (s, 1H), 4.22 (d, *J* = 7.0 Hz, 2H), 4.14 (s, 3H), 4.00 (s, 3H), 2.61-3.19 (m, 4H), 2.57 (s, 3H), 1.98-2.09 (m, 1H), 1.25-1.83 (m, 5H), 0.42-0.52 (m, 2H), 0.15-0.22 (m, 2H).

Molecular formula: $C_{26}H_{31}N_5O_2S$, molecular weight: 477.22, LC-MS (Pos, *m/z*) = 478.32[M+H]$^+$.

**Example 27: Synthesis of (*R*)-(3-aminopiperidin-1-yl)(2-(6-(cyclopropylmethyl)-6*H*-furo[2,3-*b*]pyrrol-5-yl)-7-methoxy-1-methyl-1*H*-benzo[*d*]imidazol-5-yl)methanone (Compound 24)**

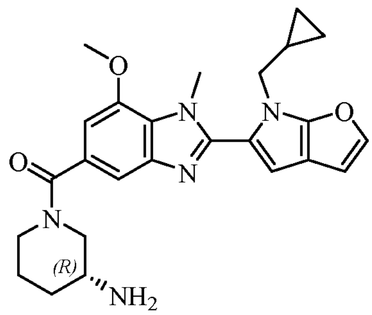

Step 1: Synthesis of furan-3-ylmethanol

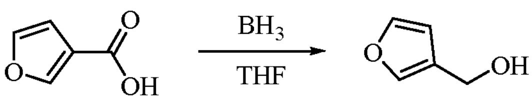


Furan-3-carboxylic acid (25 g, 0.22 mmol, 1.0 eq.) was dissolved in tetrahydrofuran (330 mL), and borane-tetrahydrofuran solution (1 mol/L, 330 mL) was slowly added dropwise under an ice bath. The mixture was then stirred at room temperature for 2 h. After the end of reaction, as detected by TLC, methanol (40 mL) was slowly added dropwise under an ice bath to quench the reaction. The reaction solution was concentrated under reduced pressure, added with ethyl acetate (500 mL), and washed with 2 mol/L diluted hydrochloric acid (40 mL × 2). The organic phase was dried and concentrated to give a crude product, which was then purified in a silica gel column chromatography (petroleum ether:ethyl acetate = 4:1 v/v) to give the target compound (12 g, yield: 55.7%).

Step 2: Synthesis of furan-3-carbaldehyde

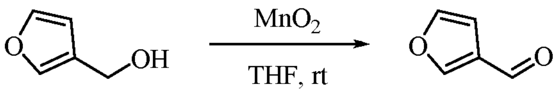


Furan-3-ylmethanol (12.0 g, 0.12 mol, 1.0 eq.) was dissolved in tetrahydrofuran (500 mL), and the solution was added with manganese dioxide (156.4 g, 1.2 mol, 10.0 eq.) and stirred at room temperature for 18 h. After the end of reaction, as detected by TLC, the reaction solution was filtered. The filtrate was concentrated to give the target compound (9.0 g of crude product), which was directly used in the next step.

Step 3: Synthesis of ethyl (Z) 2-azido-3-(furan-3-yl)acrylate

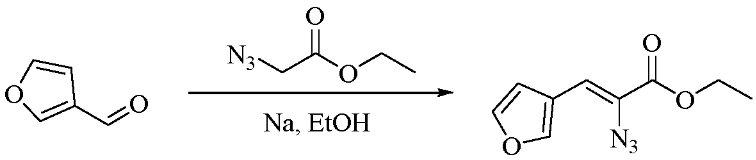


Sodium (8.6 g, 0.38 mol, 4.0 eq.) was dissolved in ethanol (300 mL), and the solution was cooled to -5°C. Furan-3-carbaldehyde (9.0 g, 0.1 mol, 1.0 eq.) and ethyl 2-azidoacetate (48.3 g, 0.38 mol, 4.0 eq.) were dissolved in ethanol (60 mL), and the solution was slowly added dropwise to the newly prepared sodium ethoxide solution with the temperature maintained at about -3 °C. The mixture was then stirred for 1 h at below 0 °C. Saturated aqueous ammonium chloride solution (30 mL) was added, and the reaction solution was poured into ice water, stirred for 10 min, and filtered. The filter cake was dissolved in ethyl acetate, and the solution was dried with anhydrous sodium sulfate and concentrated to give the target compound (8.4 g, yield: 40.6%).

Step 4: Synthesis of ethyl 6*H*-furo[2,3-*b*]pyrrole-5-carboxylate

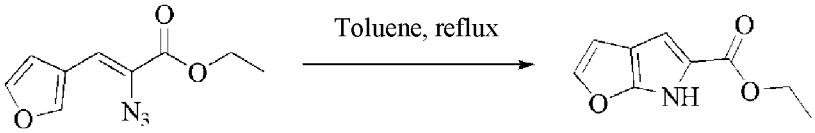


Ethyl (Z) 2-azido-3-(furan-3-yl)acrylate (8.4 g, 0.04 mol, 1.0 eq.) was dissolved in toluene (200 mL), and the solution was refluxed for 1 h. After the end of reaction, as detected by TLC, the reaction solution was concentrated to give a crude product, which was then purified in a silica gel column chromatography (PE:EA = 30:1 v/v) to give the target compound (4.24 g, yield: 59.2%).
Molecular formula: $C_9H_9NO_3$, molecular weight: 179.06, LC-MS (Pos, *m*/*z)* = 180.23 $[M+H]^+$.
$^1H$ NMR (400 MHz, $CDCl_3$) $\delta$(ppm): 9.22 (s, 1H), 6.85 (s, 1H), 6.55 (s, 1H), 4.30-4.41 (m, 2H), 1.36-1.42 (m, 3H).

Step 5: Synthesis of ethyl 6-(cyclopropylmethyl)-6*H*-furo[2,3-*b*]pyrrole-5-carboxylate

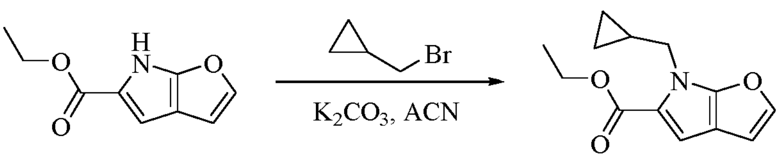

Ethyl 6*H*-furo[2,3-*b*]pyrrole-5-carboxylate (4.24 g, 0.024 mol, 1.0 eq.), cesium carbonate (20.0 g, 0.06 mmol, 2.5 eq.) and cyclopropylmethyl bromide (4.1 g, 0.03 mmol, 1.3 eq.) were dissolved in acetonitrile (120 mL), and the solution was refluxed at 80 °C for 1 h. After the end of reaction, as detected by TLC, the reaction solution was concentrated and added with water (30 mL) and ethyl acetate (200 mL). The organic phase was dried and concentrated to give a crude product, which was then purified in a silica gel column chromatography (petroleum ether:ethyl acetate = 100:1 v/v) to give the target compound (5.3 g of crude product).

$^1$H NMR (400 MHz, $CDCl_3$) δ(ppm): 7.30 (s, 1H), 6.92 (s, 1H), 6.53 (s, 1H), 4.21-4.36 (m, 4H), 1.35-1.42 (m, 4H), 0.51-0.58 (m, 2H), 0.41-0.52 (m, 2H). Step 6: Synthesis of (6-(cyclopropylmethyl)-6*H*-furo[2,3-*b*]pyrrol-5-yl) methanol

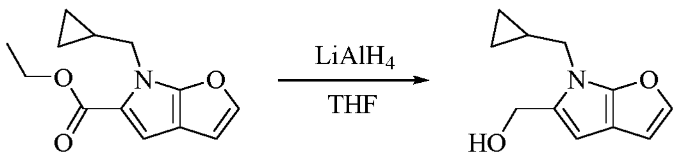


Lithium aluminum hydride (1.3 g, 34.1 mmol, 1.5 eq.) was suspended in tetrahydrofuran (100 mL), and the suspension was cooled to 0 °C. Ethyl 6-(cyclopropylmethyl)-6*H*-furo[2,3-*b*]pyrrole-5-carboxylate (5.3 g of crude product) was dissolved in tetrahydrofuran (20 mL), and then the solution was slowly added to the suspension, with the temperature of the reaction system maintained at below -5°C. The mixture was then stirred for 2 h. After the end of reaction, as detected by TLC, the reaction solution was successively and slowly added with water (1.3 g), 15% w/w aqueous sodium hydroxide solution (1.3 g) and water (3.9 g), dried with anhydrous sodium sulfate, and filtered. The filtrate was concentrated to give the target compound (4.1 g, two-step yield: 90.6%).

Step 7: Synthesis of 6-(cyclopropylmethyl)-6*H*-furo[2,3-*b*]pyrrole-5-carbaldehyde

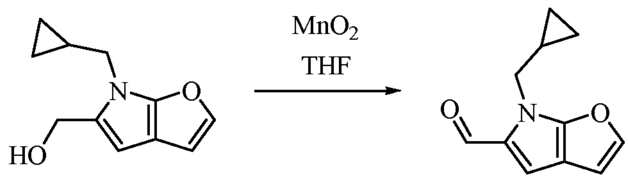


(6-(cyclopropylmethyl)-6*H*-furo[2,3-*b*]pyrrol-5-yl)methanol (3 g, 0.016 mol, 1.0 eq.) was dissolved in tetrahydrofuran (100 mL), and the solution was added with manganese dioxide (13.6 g, 0.16 mol, 10.0 eq.) and stirred at room temperature for 18 h. After the end of reaction, as detected by TLC, the reaction solution was filtered. The filtrate was concentrated to give a crude product, which was then purified in a silica gel column chromatography (elution by dichloromethane) to give the target compound (330 mg, yield: 11.1%).

Step 8: Synthesis of *tert*-butyl (*R*)-(1-(2-(6-(cyclopropylmethyl)-6*H*-furo [2,3-*b*]pyrrol-5-yl)-7-methoxy-1-methyl-1*H*-benzo[*d*]imidazole-5-carbonyl) piperidin-3-yl)carbamate

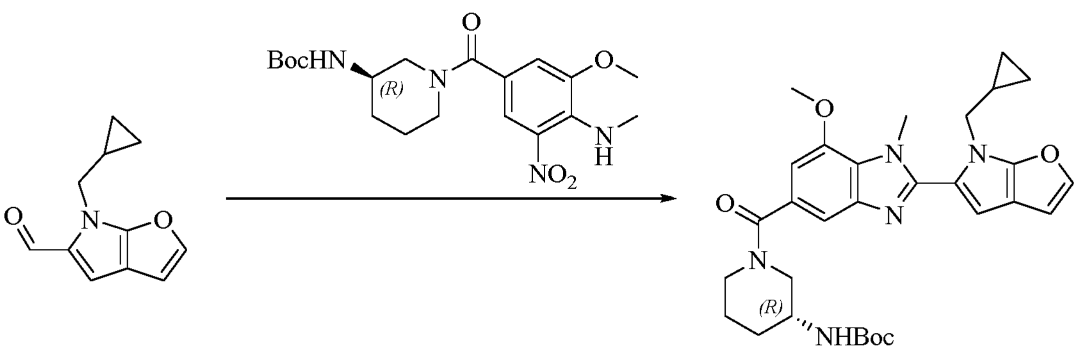

6-(cyclopropylmethyl)-6*H*-furo[2,3-*b*]pyrrole-5-carbaldehyde (310 mg, 1.64 mmol, 1.0 eq.) and *tert*-butyl

(*R*)-(1-(3-methoxy-4-(methylamino)-5-nitrobenzoyl)piperidin-3-yl)carbamate (578 mg, 1.4 mmol, 1.0 eq.) were dissolved in ethanol (2 mL), and sodium dithionite (1.2 g, 7.0 mmol, 5.0 eq.) was dissolved in water (2 mL). After the two solutions were mixed, the mixture reacted at 90 °C for 4 h. After the end of reaction, as detected by TLC, the reaction solution was concentrated and added with ethyl acetate (50 mL) and water (10 mL). The organic phase was dried and concentrated to give a crude product, which was then purified in a silica gel column chromatography (dichloromethane:methanol = 50:1 v/v) to give the target compound (130 mg, yield: 14.5 %).

Step 9: Synthesis of (*R*)-(3-aminopiperidin-1-yl)(2-(6-(cyclopropylmethyl)-6*H*-furo[2,3-*b*]pyrrol-5-yl)-7-methoxy-1-methyl-1*H*-benzo[*d*]imidazol-5-yl) methanone

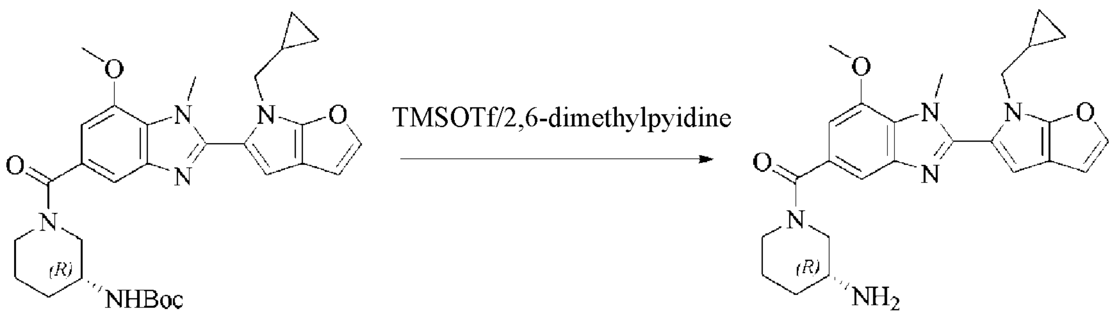


*Tert-butyl* (*R*)-(1-(2-(6-(cyclopropylmethyl)-6*H*-furo[2,3-*b*]pyrrol-5-yl)-7-methoxy-1-methyl-1*H*-benzo[*d*]imidazole-5-carbonyl)piperidin-3-yl)carbamate (110 mg, 0.2 mmol) was dissolved in dichloromethane (2 mL), and the solution was added with 2,6-dimethylpyridine (214.0 mg, 2.0 mmol, 10 eq.) and trimethylsilyl trifluoromethanesulfonate (222 mg, 1.0 mmol, 5.0 eq.) under an ice bath and stirred at room temperature for 6 h. After the end of reaction, as detected by TLC, the reaction solution was concentrated at low temperature to give a crude product, which was then purified in a silica gel column chromatography (dichloromethane:methanol = 40:1 v/v) to give the target compound (39.0 mg, yield: 43.6%).

$^1$H NMR (400 MHz, DMSO-$d_6$) δ(ppm): 7.61 (d, *J* = 1.96, 1H), 7.28 (s, 1H), 6.83 (s, 1H), 6.72 (d, *J* = 1.96, 1H), 6.64 (s, 1H), 4.25 (d, *J* = 7.0 Hz, 2H), 4.11 (s, 3H), 3.97 (s, 3H), 3.35 (s, 4H), 1.88-1.97 (m, 1H), 1.60-1.79 (m, 1H), 1.30-1.49 (m, 2H), 1.15-1.29 (m, 2H), 0.38-0.49 (m, 2H), 0.19-0.28 (m, 2H). Molecular formula: $C_{25}H_{29}N_5O_3$, molecular weight: 447.23, LC-MS (Pos, *m*/*z*) = 448.32 $[M+H]^+$.

The present invention can be better understood according to the following experimental examples. However, it is easily understood by those skilled in the art that the contents described in experimental examples are only used to illustrate the present invention, and should not and will not limit the present invention described in detail in the claims.

**Experimental example 1: Assay of enzyme-inhibiting activity of compound**

Sample: The compound disclosed herein prepared according to exemplary methods.

(1) Reagents and consumables

Peptidylarginine deiminase 4 (PAD4): Cayman, Cat. No. 10500, Lot. No. 0519694;
Carboxyfluorescein-labeled peptide (Peptide FAM-AcH4 (1-5)): GL Biochem, Cat. No. 0200046, Lot. No. P161110-TL545976;
Dimethyl sulfoxide (DMSO): Sigma, Cat. No. D2650, Lot. No. 474382;
Ethylenediaminetetraacetic acid (EDTA): Sigma, Cat. No. E5134, CAS No. 60-00-4;
96-well plate: Corning, Cat. No. 3365, Lot. No. 22008026;
384-well plate: Corning, Cat. No. 3573, Lot. No. 12608008.

(2) Preparation of 5× concentrations of compounds

An appropriate amount of the compound to be assayed was dissolved in DMSO to 10 mM. An appropriate amount of 10 mM mother liquor of the compound to be assayed was diluted to 1.5 mM with DMSO, and the solution was then serially diluted 3-fold with DMSO to give 50× concentrations of gradient compound solutions. Finally, 10-fold dilution was carried out with reaction buffer (an aqueous solution of 100 mM HEPES (2-[4-(2-hydroxyethyl)piperazin-1-yl]ethanesulfonic acid) containing 0.01% v/v Triton, pH 8.0) to give 5× concentrations of gradient compound solutions (150 μM, 50 μM, 16.667 μM, 5.556 μM, 1.852 μM, 0.617 μM, 0.206 μM, 0.069 μM, 0.023 μM, and 0.008 μM).

(3) Preparation of solutions

1) Reaction buffer: aqueous solution of 100 mM HEPES (2-[4-(2-hydroxyethyl)piperazin-1-yl]ethanesulfonic acid) containing 0.01% v/v Triton, pH 8.0;
2) Stop solution: aqueous solution of 100 mM HEPES (2-[4-(2-hydroxyethyl)piperazin-1-yl]ethanesulfonic acid) containing 0.015% w/v Brij-35, 0.2% v/v Coating Reagent (coating solution) #3 (PE, part no. 760050) and 50 mM EDTA, pH 8.0;
3) 2.5× enzyme solution: An appropriate amount of PAD4 was diluted to 2.5× (final concentration: 10 nM) with reaction buffer;
4) 2.5× polypeptide solution: An appropriate amount of carboxyfluorescein - labeled peptide (FAM-AcH4(1-5) peptide) and $CaCl_2$ solution was diluted to 2.5× (the final concentration of polypeptide: 5 μM, the final concentration of $Ca^{2+}$: 0.1 mM) with reaction buffer.

(4) Method

5 μL of 5× compound solutions or solvents with different concentrations were added to a 384-well plate, 10 μL of 2.5× enzyme solution was then added, and the plate was incubated at room temperature for 10 min. 10 μL of 2.5 × polypeptide solution was then added to each well, and the plate was incubated at 28 °C for a certain period of time. 25 μL of stop solution was added to stop reaction. Caliper was used for detection. The inhibition rate was calculated according to the following formula:

$$\text{Inhibition percentage} = (\max - \text{transformation})/(\max - \min) * 100.$$

"max" denotes DMSO control; and "min" denotes low control. Results

Table 1.

| Samples | PAD4 $IC_{50}$ (μM) |
|---|---|
| Compound GSK199 | 0.14 |
| Compound 3 | 0.20 |
| Compound 4 | 0.35 |
| Compound 9 | 0.11 |
| Compound 18 | 0.036 |
| Compound 20 | 0.058 |
| Compound 21 | 0.055 |
| Compound 22 | 0.04 |
| Compound 23 | 0.025 |
| Compound 24 | 0.054 |

It can be seen from the results in Table 1 that the compound disclosed herein has good inhibitory activity on PAD4, and a good clinical application potential in treating diseases mediated by PAD4 abnormality.

**Experimental example 2: Evaluation on liver microsomal stability of the compound disclosed herein in human/canine**

**Composition of the incubation system:**

| Substances to be added | Initial concentration | Proportion (% v/v) | Final concentration |
|---|---|---|---|
| Phosphate buffer | 100 mM | 50 | 50 mM |
| $MgCl_2$ | 20 mM | 5 | 1 mM |
| Liver microsome | 20 mg protein/mL | 2.5 | 0.5 mg protein/mL |
| Water to be supplemented | | 22.5 | - |
| Compound | 10 μM | 10 | 1 μM |
| β-NADPH | 10 mM | 10 | 1 mM |

**Preparation of tested sample:**

An appropriate amount of the compound was precisely weighed and dissolved in DMSO to prepare a 5.0 mM stock solution. 5.0 mM stock solution was diluted to 1.0 mM with DMSO, and then diluted with water to 10 μM, serving as a compound working solution for later use (DMSO content in the reaction system: 0.1% v/v).

**Steps:**

(1) The liver microsomes (20 mg protein/mL) were taken from a -80 °C refrigerator, pre-incubated on a 37 °C water bath thermostatic oscillator for 3 min, and thawed for use.

(2) A mixed incubation system solution (without compounds or β-NADPH) was prepared according to the proportion of "composition of the incubation system" described above and pre-incubated on a 37 °C water bath thermostatic oscillator for 2 min.

(3) Control group (without β-NADPH): 30 μL of water and 30 μL of compound working solution (10 μM) were added to 240 μL of the mixed incubation system solution in step (2), and the mixture was vortexed for 30 s and mixed. The total volume of the mixture was 300 μL. The sample was duplicated. The reaction solution was placed on a 37 °C water bath thermostatic oscillator for incubation, and timing was started. Samples were collected at 0 min and 60 min.

(4) Sample group: 70 μL of β-NADPH solution (10 mM) and 70 μL of compound working solution (10 μM) were added to 560 μL of the mixed incubation system solution in step (2), and the total volume of the mixture was 700 μL. The mixture was vortexed for 30 s and mixed. The sample was duplicated. The reaction solution was placed on a 37 °C water bath thermostatic oscillator for incubation, and timing was started. Samples were collected at 0 min, 5 min, 10 min, 20 min, 30 min and 60 min after timing.

(5) The reaction solution was vortexed for 3 min, and centrifuged at 12,000 rpm for 5 min.

(6) 50 μL of the supernatant was added to 150 μL of water, and the resulting solution was vortexed and mixed before LC/MS/MS analysis.

**Data analysis:**

The half-life ($t_{1/2}$) and clearance (Cl) were calculated using the following first-order kinetic formula:

$$C_t = C_0 * e^{-kt}$$

$$t_{1/2} = \ln 2/k = 0.693/k$$

$$Cl_{int} = V_d * k$$

$V_d$ = 1/protein content in liver microsomes

Note: k denotes the slope of the logarithm of the remaining amount of a compound vs. time, $V_d$ denotes apparent volume of distribution, Co denotes drug concentration at 0 h, Ct denotes drug concentration at time t, t denotes time, e denotes natural number, and $Cl_{int}$ denotes intrinsic clearance.

**Results:**

**Table 2. Evaluation results of liver microsomal stability of the compound disclosed herein in human**

| Compound | Human | |
|---|---|---|
| | $CL_{int}$ (mL/min/mg) | $t_{1/2}$ (min) |
| Compound GSK199 | 0.0052 | 267 |
| Compound 3 | 0.0050 | 277 |
| Compound 4 | 0.0020 | 693 |
| Compound 18 | 0.0066 | 210 |

**Table 3. Evaluation results of liver microsomal stability of the compound disclosed herein in canine**

| Compound | Canine | |
|---|---|---|
| | $CL_{int}$ (mL/min/mg) | $t_{1/2}$ (min) |
| Compound GSK199 | 0.0018 | 770 |
| Compound 4 | 0.0042 | 330 |
| Compound 9 | 0.0010 | 1386 |

It can be seen from Tables 2-3 that the compound disclosed herein has good stability and low clearance in human and canine liver microsomes.

**Experimental example 3: Pharmacokinetics (PK) of the compound in rats**

**Administration and sample collection:**

The test compound was dissolved in a mixture of 5% v/v DMSO + 20% v/v (30% v/v solutol (polyethylene glycol-15 hydroxystearate purchased from BASF, batch no. 84773247G0)) + 75% v/v brine to prepare a solution, which was then administered to SD rats by oral gavage at a dose of 5.0 mg/kg. Blood samples were collected at 15 min, 30 min, 1 h, 2 h, 4 h, 6 h, 8 h and 24 h.

The test compound was dissolved in a mixture of 5% v/v DMSO + 20% v/v (30% v/v solutol) + 75% v/v brine to prepare a solution, which was administered to SD rats by intravenous bolus at a dose of 1 mg/kg. Blood samples were collected at 5 min, 15 min, 30 min, 1 h, 2 h, 4 h, 6 h, 8 h and 24 h.

The animals were fixed, with tails warmed in a water bath 10 min before each sampling time point. About 100 μL of blood was collected via the tail vein, and the blood samples were collected in anticoagulation tubes containing EDTA-$K_2$. The blood samples were centrifuged at 8,000 rpm for 6 min at 4 °C to give plasma samples within 30 min after collection. The plasma samples were stored in a refrigerator at -80 °C before test.

**Sample analysis:**

The sample was taken out from the -80 °C refrigerator, and vortexed for 5 min after naturally thawed at room temperature. 20 μL of plasma sample was pipetted into a 1.5 mL centrifuge tube, and 200 μL of 100 ng/mL internal standard working solution (a solution of tolbutamide in methanol) was added. The resulting mixture was mixed, vortexed for 5 min, and then centrifuged at 12,000 rpm for 5 min. 50 μL of the supernatant was pipetted into a 96-well plate with 150 μL of water per well, and the plate was vortexed for 5 min before LC-MS/MS analysis.

**Data processing:**

The concentration was calculated by Analyst 1.6.3 from AB Sciex. Parameters such as mean, standard deviation and coefficient of variation (excluding those output directly by Analyst 1.6.3) were calculated by Microsoft Excel. PK parameters were calculated by Pharsight Phoenix 6.1 software NCA ($T_{max}$ was in median).

**Results:**

Table 4. PK parameters of compounds in SD rats (IV: 1 mg/kg, PO: 5 mg/kg, n = 3)

| Compound | $t_{z1/2}$ iv/po (h) | $V_{z_obs}$ iv (L/kg) | $Cl_{_obs}$ iv (L/h/kg) | Tmax po (h) | $AUC_{last}$ iv/po (h*ng/mL) | F% |
|---|---|---|---|---|---|---|
| Compound GSK199 | 1.63/2.23 | 6.75 | 2.89 | 4.00 | 330/496 | 33.1 |
| Compound 3 | 9.97/- | 11.2 | 0.79 | 6.00 | 1196/934 | 15.6 |
| Compound 9 | 5.96/6.33 | 22.0 | 2.62 | 6.00 | 303/633 | 41.8 |
| Compound 18 | 3.92/3.96 | 5.64 | 1.00 | 6.00 | 1013/2359 | 46.6 |
| Compound 20 | 6.94/13.2 | 2.86 | 0.30 | 8.00 | 3158/11240 | 71.2 |
| Compound 21 | 6.93/4.70 | 8.73 | 0.92 | 8.00 | 772/1286 | 33.3 |
| Compound 22 | 14.6/12.0 | 1.76 | 0.10 | 6.00 | 7677/13452 | 35.0 |
| Compound 23 | 7.90/7.52 | 11.6 | 1.08 | 6.00 | 887/1383 | 31.2 |

**Note:** $t_{z1/2}$: terminal half-life; $Cl_{_obs}$: clearance; $V_{z_obs}$: apparent volume of distribution; $T_{max}$: time to maximum plasma concentration; $AUC_{last}$: area under concentration-time curve (0-24 h); F%: absolute bioavailability; -: not detected.

It can be seen from Table 4 that the compound disclosed herein has higher exposure and lower clearance in comparison with the reference GSK199.

**Experimental example 4:** Evaluation on liver microsomal stability of the compound disclosed herein in mouse/rat

**Objective:** To evaluate the liver microsomal stability of the compound disclosed herein in mouse/rat

**Composition of the incubation system:**

| Substances to be added | Initial concentration | Proportion (% v/v) | Final concentration |
|---|---|---|---|
| Phosphate buffer | 100 mM | 50 | 50 mM |
| $MgCl_2$ | 20 mM | 5 | 1 mM |
| Liver microsome | 20 mg protein/mL | 2.5 | 0.5 mg protein/mL |
| Water to be supplemented | - | 22.5 | - |
| Compound | 10 μM | 10 | 1 μM |
| β-NADPH | 10 mM | 10 | 1 mM |

**Preparation of Compound 3, Compound 9 and Compound 18:**

An appropriate amount of the compound was precisely weighed and dissolved in DMSO to prepare a 5.0 mM stock solution. 5.0 mM stock solution was diluted to 1.0 mM with DMSO, and then diluted with water to 10 μM, serving as a compound working solution for later use (DMSO content in the reaction system: 0.1% v/v).

**Preparation of Compound 20, Compound 21, Compound 22 and Compound 23:**

An appropriate amount of the compound was precisely weighed and dissolved in methanol to prepare a 5.0 mM stock solution. 5.0 mM stock solution was diluted to 1.0 mM with methanol, and then diluted with 10% methanol-water (v/v) solution to 10 μM, serving as a compound working solution for later use (methanol content in the reaction system: 1.1% v/v).

**Steps:**

(1) The liver microsomes (20 mg protein/mL) were taken from a -80 °C refrigerator, pre-incubated on a 37 °C water bath thermostatic oscillator for 3 min, and thawed for use.
(2) A mixed incubation system solution (without compounds or β-NADPH) was prepared according to the proportion of "composition of the incubation system" described above and pre-incubated on a 37 °C water bath thermostatic oscillator for 2 min.
(3) Control group (without β-NADPH): 30 μL of water and 30 μL of compound working solution (10 μM) were added to 240 μL of the mixed incubation system solution in step (2), and the mixture was vortexed for 30 s and mixed. The total volume of the mixture was 300 μL. The sample was duplicated. The reaction solution was placed on a 37 °C water bath thermostatic oscillator for incubation, and timing was started. Samples were collected at 0 min and 60 min.
(4) Sample group: 70 μL of β-NADPH solution (10 mM) and 70 μL of compound working solution (10 μM) were added to 560 μL of the mixed incubation system solution in step (2), and the total volume of the mixture was 700 μL. The mixture was vortexed for 30 s and mixed. The sample was duplicated. The reaction solution was placed on a 37 °C water bath thermostatic oscillator for incubation, and timing was started. Samples were collected at 0 min, 5 min, 10 min, 20 min, 30 min and 60 min after timing.
(5) The reaction solution was vortexed for 3 min, and centrifuged at 12,000 rpm for 5 min.
(6) 50 μL of the supernatant was added to 150 μL of water, and the resulting solution was vortexed and mixed before LC/MS/MS analysis.

**Data analysis:**

The half-life ($t_{1/2}$) and clearance (Cl) were calculated using the following first-order kinetic formula:

$$C_t = C_0 * e^{-kt}$$

$$t_{1/2} = \ln 2/k = 0.693/k$$

$$Cl_{int} = V_d * k$$

$V_d$ = 1/protein content in liver microsomes
Note: k denotes the slope of the logarithm of the remaining amount of a compound vs. time, $V_d$ denotes apparent volume of distribution, Co denotes drug concentration at 0 h, Ct denotes drug concentration at time t, t denotes time, e denotes natural number, and $Cl_{int}$ denotes intrinsic clearance.

**Results:**

**Table 5. Assay results of the liver microsomal stability of the compound disclosed herein in mouse and rat**

| Compound | Mouse | | Rat | |
|---|---|---|---|---|
| | $CL_{int}$ (mL/min/mg) | $t_{1/2}$ (min) | $CL_{int}$ (mL/min/mg) | $t_{1/2}$ (min) |
| Compound 3 | - | - | 0.0058 | 239 |
| Compound 9 | - | - | $\rightarrow 0$ | $\rightarrow \infty$ |
| Compound 18 | 0.0056 | 248 | - | - |
| Compound 20 | 0.0020 | 693 | 0.0044 | 315 |
| Compound 21 | - | - | 0.0078 | 178 |
| Compound 22 | 0.0086 | 161 | - | - |
| Compound 23 | 0.0058 | 239 | - | - |
| "-" represents not detected. | | | | |

It can be seen from Table 5 that the compound disclosed herein has good stability and low clearance in mouse and rat liver microsomes.

**Claims**

1. A compound of general formula (I) or a pharmaceutically acceptable salt, stereoisomer or tautomer thereof:

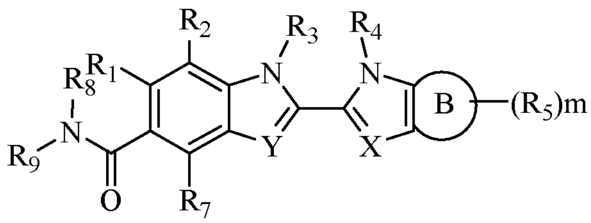

(I)

wherein X and Y are each independently selected from $CR_6$ or N;
$R_1$ is hydrogen or $C_{1-6}$ alkyl;
$R_2$ is hydrogen, $C_{1-6}$ alkyl, 3-6 membered cycloalkyl, $C_{1-6}$ alkoxy, $C_{1-6}$ haloalkyl or $C_{1-6}$ haloalkoxy;
$R_3$ is hydrogen, cyano, $C_{1-6}$ alkyl, $C_{1-6}$ alkoxy, $C_{1-6}$ alkoxy $C_{1-6}$ alkyl, $-L_1-Cy_1$, or $C_{1-6}$ alkyl, $C_{1-6}$ alkylcarbonyl, $C_{1-6}$ alkoxycarbonyl, $C_{1-6}$ alkylsulfonyl, $C_{1-6}$ alkoxy $C_{1-6}$ alkyl, $C_{1-6}$ alkylamino or $(C_{1-6}$ alkyl$)_2$ amino unsubstituted or substituted by halogen, cyano, amino or hydroxyl, $L_1$ is absent or is $C_{1-6}$ alkylene, $Cy_1$ is 3-12 membered cycloalkyl, 3-12 membered cycloalkenyl, 3-12 membered heterocyclyl, aryl or 5-10 membered heteroaryl, and $Cy_1$ may be optionally substituted by hydrogen, halogen, cyano, amino, hydroxyl, $C_{1-6}$ alkyl or $C_{1-6}$ alkoxy;
$R_4$ is hydrogen, cyano, 3-6 membered cycloalkyl, or $C_{1-6}$ alkyl, $C_{1-6}$ alkylcarbonyl, $C_{1-6}$ alkoxycarbonyl, $C_{1-6}$ alkylsulfonyl, $C_{1-6}$ alkoxy $C_{1-6}$ alkyl, $C_{1-6}$ alkylamino or $(C_{1-6}$ alkyl$)_2$ amino unsubstituted or substituted by halogen, cyano, amino, hydroxyl or 3-6 membered cycloalkyl;
$R_5$ is hydrogen, halogen, cyano, amino, hydroxyl, $-L_2-Cy_2$, or $C_{1-6}$ alkyl, $C_{2-8}$ alkenyl, $C_{1-6}$ alkylcarbonyl, $C_{1-6}$ alkylcarbonylamino, $C_{1-6}$ alkoxy, $C_{1-6}$ alkoxycarbonyl, $C_{1-6}$ alkylsulfonyl, $C_{1-6}$ alkylthio, $C_{1-6}$ alkylsulfonylamino, $C_{1-6}$ alkylamino or $(C_{1-6}$ alkyl$)_2$ amino unsubstituted or substituted by halogen, cyano, amino or hydroxyl, $L_2$ is absent or is $C_{1-6}$ alkylene, $C_{1-6}$ alkyleneoxy, $C_{2-6}$ alkenylene or $C_{1-6}$ alkyleneamino, $Cy_2$ is 3-12 membered cycloalkyl, 3-12 membered cycloalkenyl, 3-12 membered heterocyclyl, aryl or 5-10 membered heteroaryl, and $Cy_2$ may be optionally substituted by hydrogen, halogen, cyano, amino, hydroxyl, $C_{1-6}$ alkyl or $C_{1-6}$ alkoxy;
$R_6$ is hydrogen, $C_{1-6}$ alkyl or $C_{3-6}$ cycloalkyl;
$R_7$ is hydrogen or $C_{1-6}$ alkyl;
$R_8$ is hydrogen, halogen, cyano, amino, hydroxyl, $-L_3-Cy_3$, or $C_{1-6}$ alkyl, $C_{2-8}$ alkenyl, $C_{1-6}$ alkylcarbonyl, $C_{1-6}$ alkylcarbonylamino, $C_{1-6}$ alkoxy or $C_{1-6}$ alkoxycarbonyl unsubstituted or substituted by halogen, cyano, amino or hydroxyl, $L_3$ is absent or is $C_{1-6}$ alkylene or $C_{2-6}$ alkenylene, $Cy_3$ is 3-12 membered cycloalkyl, 3-12 membered

cycloalkenyl, 3-12 membered heterocyclyl, aryl or 5-10 membered heteroaryl, and $Cy_3$ may be optionally substituted by hydrogen, halogen, cyano, amino, hydroxyl, $C_{1-6}$ alkyl or $C_{1-6}$ alkoxy;
$R_9$ is hydrogen or $C_{1-6}$ alkyl;
alternatively, $R_8$ and $R_9$, along with N connected thereto, form 4-7 membered heterocyclyl, 6-11 membered ortho-heterocyclyl, 7-12 membered spiro-heterocyclyl or 6-10 membered bridged heterocyclyl unsubstituted or substituted by a substituent selected from hydrogen, halogen, cyano, amino, hydroxyl, $C_{1-6}$ alkyl, $C_{1-6}$ alkoxy, $C_{1-6}$ alkylamino, $(C_{1-6}$ alkyl$)_2$ amino and $-L_4-Cy_4$, $L_4$ is absent or is $C_{1-6}$ alkylene, and $Cy_4$ is 3-12 membered cycloalkyl, 3-12 membered cycloalkenyl, 3-12 membered heterocyclyl, aryl or 5-10 membered heteroaryl;
ring B is

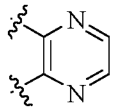,
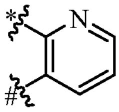,
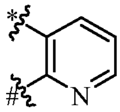,
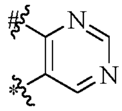

or 5 membered heteroaryl;
wherein ring B is fused to

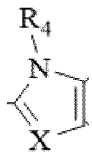

with the * terminus upward and the # terminus downward, and if no * terminus or # terminus is specified, ring B can be fused in any direction;
with the *proviso* that
when ring B is

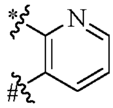

and $R_8$ and $R_9$ along with N connected thereto form

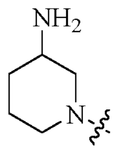,

X is N;
when ring B is

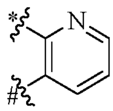

and X is $CR_6$, $R_8$ and $R_9$ along with N connected thereto form

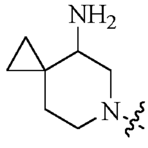;

and
m is an integer of 0 to 4.

2. The compound or the pharmaceutically acceptable salt, stereoisomer or tautomer thereof according to claim 1, wherein X and Y are each independently selected from $CR_6$ or N;
$R_1$ is hydrogen or $C_{1-6}$ alkyl;
$R_2$ is hydrogen, $C_{1-6}$ alkyl, $C_{1-6}$ alkoxy, $C_{1-6}$ haloalkyl or $C_{1-6}$ haloalkoxy;
$R_3$ is hydrogen, cyano, $C_{1-6}$ alkyl, $C_{1-6}$ alkoxy, $C_{1-6}$ alkoxy $C_{1-6}$ alkyl, or $C_{1-6}$ alkyl unsubstituted or substituted by halogen, cyano, amino or hydroxyl;
$R_4$ is hydrogen, cyano, 3-6 membered cycloalkyl, or $C_{1-6}$ alkyl unsubstituted or substituted by halogen, cyano, amino, hydroxyl or 3-6 membered cycloalkyl;
$R_5$ is hydrogen, halogen, cyano, amino, hydroxyl, or $C_{1-6}$ alkyl or $C_{1-6}$ alkoxy unsubstituted or substituted by halogen, cyano, amino or hydroxyl;
$R_6$ is hydrogen or $C_{1-6}$ alkyl;
$R_7$ is hydrogen or $C_{1-6}$ alkyl;
$R_8$ is hydrogen, halogen, cyano, amino, hydroxyl, $-L_3-Cy_3$, or $C_{1-6}$ alkyl unsubstituted or substituted by halogen, cyano, amino or hydroxyl, $L_3$ is absent or is $C_{1-6}$ alkylene, $Cy_3$ is 3-6 membered cycloalkyl, 3-12 membered heterocyclyl, aryl or 5-10 membered heteroaryl, and $Cy_3$ may be optionally substituted by hydrogen, halogen, cyano, amino, hydroxyl, $C_{1-6}$ alkyl or $C_{1-6}$ alkoxy;
$R_9$ is hydrogen or $C_{1-6}$ alkyl;
alternatively, $R_8$ and $R_9$ along with N connected thereto form 5-6 membered heterocyclyl, 7-10 membered ortho-heterocyclyl, 7-11 membered spiro-heterocyclyl or 7-10 membered bridged heterocyclyl unsubstituted or substituted by a substituent selected from hydrogen, halogen, cyano, amino, hydroxyl, $C_{1-6}$ alkyl and $C_{1-6}$ alkoxy;
ring B is 5 membered heteroaryl, and preferably, ring B is

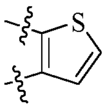, 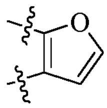

or

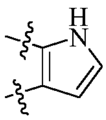;

and m is an integer of 0 to 4.

3. The compound or the pharmaceutically acceptable salt, stereoisomer or tautomer thereof according to claim 2, wherein X is $CR_6$, and Y is N;
$R_1$ is hydrogen or $C_{1-6}$ alkyl;
$R_2$ is hydrogen, $C_{1-6}$ alkyl or $C_{1-6}$ alkoxy;
$R_3$ is hydrogen or $C_{1-6}$ alkyl;
$R_4$ is hydrogen, or $C_{1-6}$ alkyl unsubstituted or substituted by halogen or 3-6 membered cycloalkyl;
$R_5$ is hydrogen, halogen, $C_{1-6}$ alkyl or $C_{1-6}$ alkoxy;
$R_6$ is hydrogen or $C_{1-6}$ alkyl;
$R_7$ is hydrogen or $C_{1-6}$ alkyl;
$R_8$ and $R_9$ along with N connected thereto form 5-6 membered heterocyclyl unsubstituted or substituted by a substituent selected from amino;
ring B is 5 membered heteroaryl, preferably

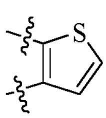, 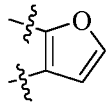 or 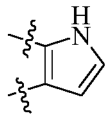;

and
m is an integer of 0 to 4.

**4.** The compound or the pharmaceutically acceptable salt, stereoisomer or tautomer thereof according to claim 3, wherein $R_8$ and $R_9$ along with N connected thereto form pyrrolidinyl, piperidinyl, piperazinyl or morpholinyl unsubstituted or substituted by a substituent selected from amino.

**5.** The compound or the pharmaceutically acceptable salt, stereoisomer or tautomer thereof according to claim 2, wherein X is $CR_6$, and Y is N;
$R_8$ and $R_9$ along with N connected thereto form

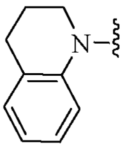, 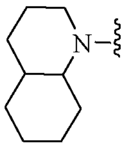,

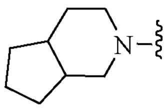, 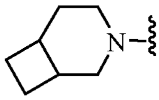, 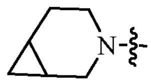, 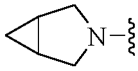, 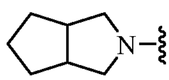, 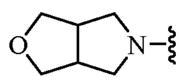,

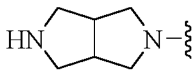, 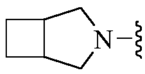 or 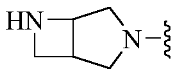

unsubstituted or substituted by a substituent selected from hydrogen, halogen, cyano, amino, hydroxyl, $C_{1-6}$ alkyl and $C_{1-6}$ alkoxy.

**6.** The compound or the pharmaceutically acceptable salt, stereoisomer or tautomer thereof according to claim 2, wherein X is $CR_6$, and Y is N;
$R_8$ and $R_9$ along with N connected thereto form

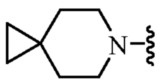, 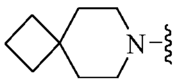,

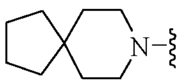, 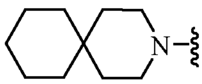, 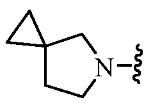, 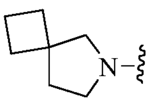, 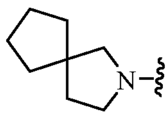, 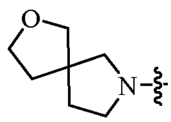,

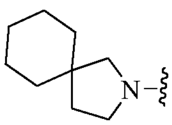, 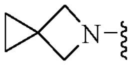, 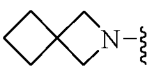, 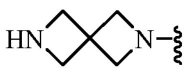, 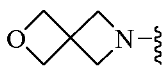, 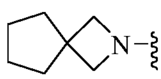,

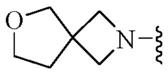, 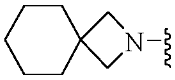 or 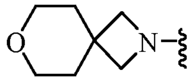

unsubstituted or substituted by a substituent selected from hydrogen, halogen, cyano, amino, hydroxyl, $C_{1-6}$ alkyl and $C_{1-6}$ alkoxy.

**7.** The compound or the pharmaceutically acceptable salt, stereoisomer or tautomer thereof according to claim 2, wherein X is $CR_6$, and Y is N;
$R_8$ and $R_9$ along with N connected thereto form

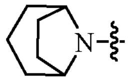, 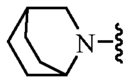,

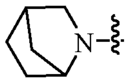 or 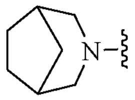

unsubstituted or substituted by a substituent selected from hydrogen, halogen, cyano, amino, hydroxyl, $C_{1-6}$ alkyl and $C_{1-6}$ alkoxy.

**8.** The compound or the pharmaceutically acceptable salt, stereoisomer or tautomer thereof according to claim 2, wherein X is $CR_6$, and Y is N;
$R_8$ is hydrogen, halogen, cyano, amino, hydroxyl, $-L_3-Cy_3$, or $C_{1-6}$ alkyl unsubstituted or substituted by halogen, cyano, amino or hydroxyl, $L_3$ is absent or is $C_{1-6}$ alkylene, $Cy_3$ is 3-6 membered cycloalkyl or 3-8 membered heterocyclyl, and $Cy_3$ may be optionally substituted by halogen, cyano, amino, hydroxyl, $C_{1-6}$ alkyl or $C_{1-6}$ alkoxy; and
$R_9$ is hydrogen or $C_{1-6}$ alkyl.

**9.** The compound or the pharmaceutically acceptable salt, stereoisomer or tautomer thereof according to claim 3, wherein X is $CR_6$, and Y is N;
$R_1$ is hydrogen or $C_{1-6}$ alkyl;
$R_2$ is hydrogen, $C_{1-6}$ alkyl or $C_{1-6}$ alkoxy;
$R_3$ is hydrogen or $C_{1-6}$ alkyl;
$R_4$ is hydrogen, or $C_{1-6}$ alkyl unsubstituted or substituted by halogen or 3-6 membered cycloalkyl;
$R_5$ is hydrogen, halogen, $C_{1-6}$ alkyl or $C_{1-6}$ alkoxy;
$R_6$ is hydrogen or $C_{1-6}$ alkyl;
$R_7$ is hydrogen or $C_{1-6}$ alkyl;
$R_8$ is hydrogen, or $C_{1-6}$ alkyl unsubstituted or substituted by amino;
$R_9$ is hydrogen or $C_{1-6}$ alkyl;
alternatively, $R_8$ and $R_9$ along with N connected thereto form 5-6 membered saturated nitrogen containing heterocyclyl unsubstituted or substituted by a substituent
selected from amino;
ring B is

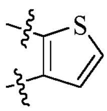;

and
m is 0 or 1.

**10.** The compound or the pharmaceutically acceptable salt, stereoisomer or tautomer thereof according to claim 1, wherein X and Y are each independently selected from $CR_6$ or N;

$R_1$ is hydrogen or $C_{1-6}$ alkyl;
$R_2$ is hydrogen, $C_{1-6}$ alkyl or $C_{1-6}$ alkoxy;
$R_3$ is hydrogen or $C_{1-6}$ alkyl;
$R_4$ is hydrogen, or $C_{1-6}$ alkyl unsubstituted or substituted by halogen or 3-6 membered cycloalkyl;
$R_5$ is hydrogen, halogen, $C_{1-6}$ alkyl or $C_{1-6}$ alkoxy;
$R_6$ is hydrogen or $C_{1-6}$ alkyl;
$R_7$ is hydrogen or $C_{1-6}$ alkyl;
$R_8$ is hydrogen, $-L_3-Cy_3$, or $C_{1-6}$ alkyl unsubstituted or substituted by amino, $L_3$ is absent, and $Cy_3$ is 3-6 membered cycloalkyl optionally substituted by amino;
$R_9$ is hydrogen or $C_{1-6}$ alkyl;
alternatively, $R_8$ and $R_9$ along with N connected thereto form 4-7 membered heterocyclyl or 7-12 membered spiro-heterocyclyl unsubstituted or substituted by a substituent selected from amino;
ring B is

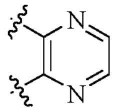,
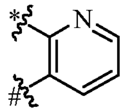,
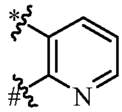

or 5 membered heteroaryl;
wherein ring B is fused to

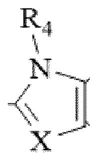

with the * terminus upward and the # terminus downward, and if no * terminus or # terminus is specified, ring B can be fused in any direction;
with the *proviso* that
when ring B is

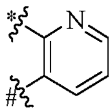

and $R_8$ and $R_9$ along with N connected thereto form

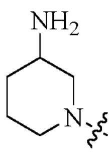,

X is N;
when ring B is

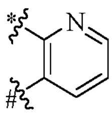

and X is $CR_6$, $R_8$ and $R_9$ along with N connected thereto form

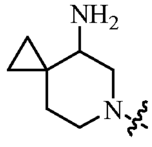;

and
m is 0 or 1.

**11.** A compound of the following formulas, or a pharmaceutically acceptable salt, stereoisomer or tautomer thereof:

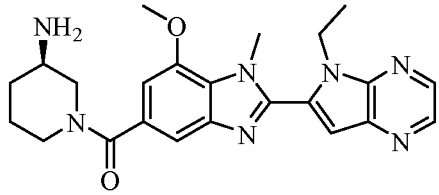, 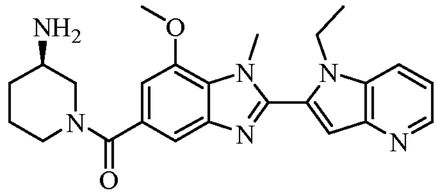,

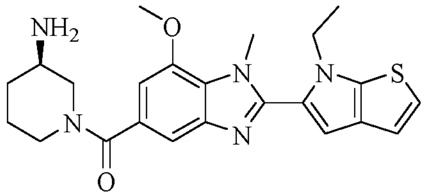, 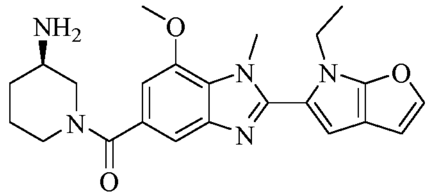,

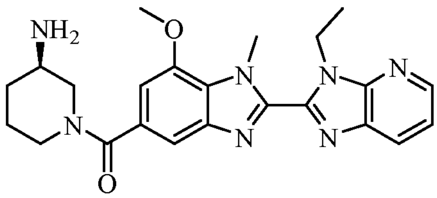, 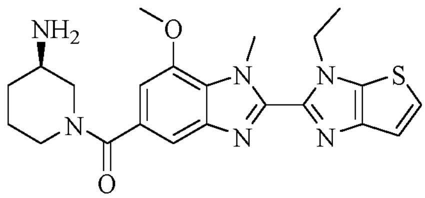,

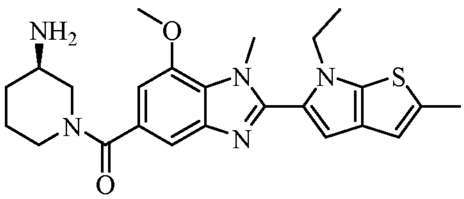, 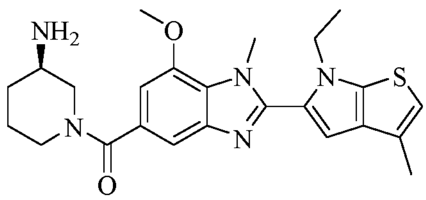,

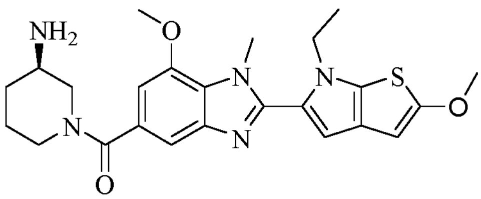, 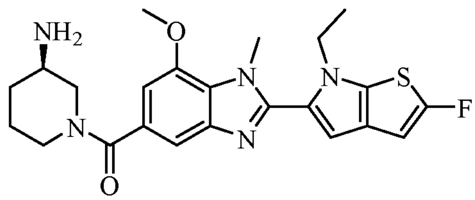,

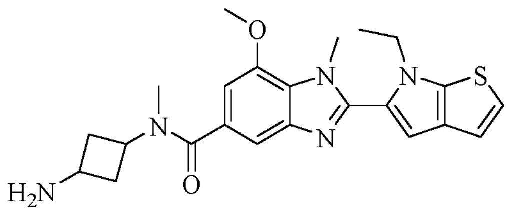,
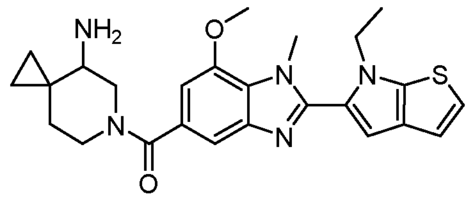,
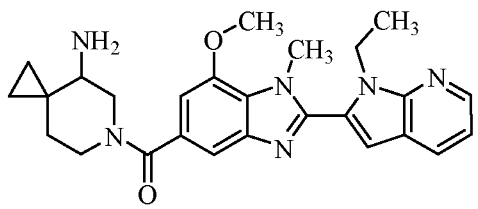,
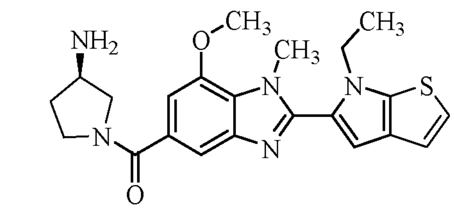,
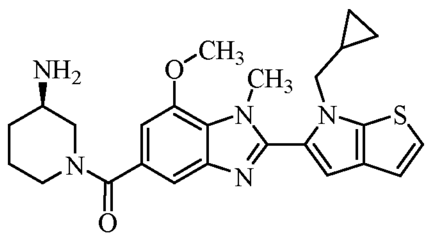,
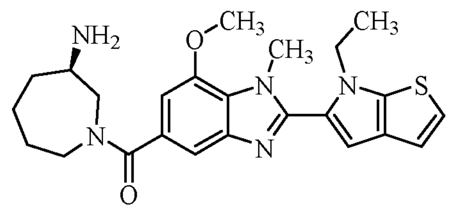,
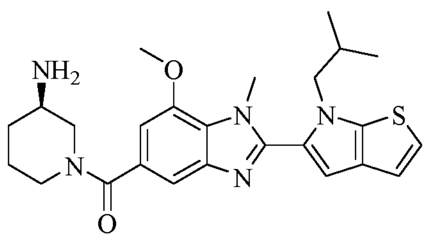,
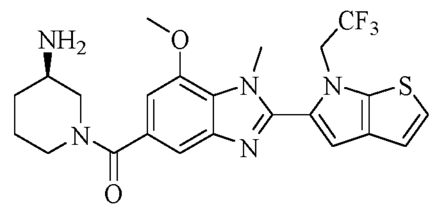,
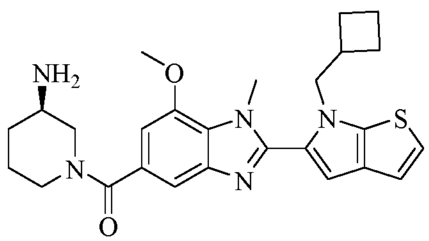,
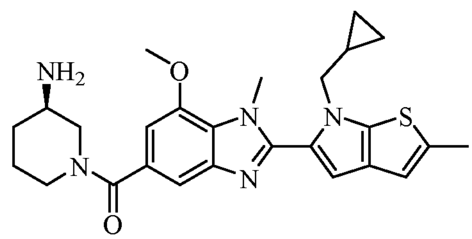,
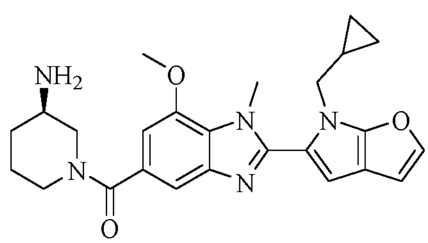,
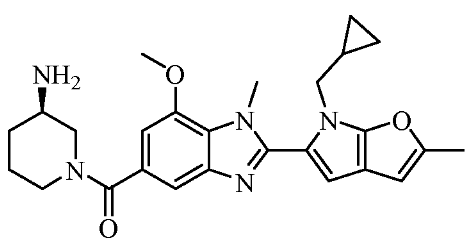.

**12.** A pharmaceutical composition comprising one or more of the compounds or the pharmaceutically acceptable salts, stereoisomers and tautomers thereof according to any one of claims 1-11, and optionally containing one or more pharmaceutical carriers, wherein, the pharmaceutical composition is optionally formulated into any pharmaceutically acceptable dosage form.

**13.** Use of the compound or the pharmaceutically acceptable salt, stereoisomer and tautomer thereof according to any one of claims 1-11 or the pharmaceutical composition according to claim 12 in preparing a medicament for treating or preventing a disease mediated by peptidylarginine deiminase (PAD4).

**14.** The use according to claim 13, wherein the disease mediated by PAD4 is selected from rheumatoid arthritis, vasculitis, systemic lupus erythematosus, ulcerative colitis, multiple sclerosis, cystic fibrosis, cancer, cutaneous lupus erythematosus, asthma and psoriasis.

**INTERNATIONAL SEARCH REPORT**

International application No.
**PCT/CN2019/076129**

**A. CLASSIFICATION OF SUBJECT MATTER**

C07D 471/04(2006.01)i; C07D 491/048(2006.01)i; C07D 495/04(2006.01)i; C07D 487/04(2006.01)i; A61K 31/4184(2006.01)i; A61K 31/4545(2006.01)i; A61K 31/4985(2006.01)i; A61K 31/454(2006.01)i; A61K 31/4709(2006.01)i; A61K 31/438(2006.01)i; A61K 31/55(2006.01)i; A61P 35/00(2006.01)i; A61P 29/00(2006.01)i; A61P 27/00(2006.01)i; A61P 37/00(2006.01)i; A61P 17/06(2006.01)i; A61P 19/02(2006.01)i; A61P 25/28(2006.01)i; A61P 11/00(2006.01)i

According to International Patent Classification (IPC) or to both national classification and IPC

**B. FIELDS SEARCHED**

Minimum documentation searched (classification system followed by classification symbols)

C07D; A61K 31/-; A61P

Documentation searched other than minimum documentation to the extent that such documents are included in the fields searched

Electronic data base consulted during the international search (name of data base and, where practicable, search terms used)

CNKI, CNABS, VEN, CAPLUS, REGISTRY: 肽酰精氨酸脱亚胺酶, PAD4, 抑制剂, 苯并咪唑, 酰胺, peptidylarginine deiminase, inhibitor, benzoimidazol, benzodiazol, structure search

**C. DOCUMENTS CONSIDERED TO BE RELEVANT**

| Category* | Citation of document, with indication, where appropriate, of the relevant passages | Relevant to claim No. |
|---|---|---|
| X | WO 2017100594 A1 (PADLOCK THERAPEUTICS, INC.) 15 June 2017 (2017-06-15) see description, paragraph [0044], and claims 1-21 | 1, 12-14 |
| A | WO 2017100594 A1 (PADLOCK THERAPEUTICS, INC.) 15 June 2017 (2017-06-15) see description, paragraph [0044], and claims 1-21 | 2-11 |
| X | CN 104470919 A (GLAXOSMITHKLINE PLC) 25 March 2015 (2015-03-25) see claims 1 and 18-22, and description, paragraph [0001] | 1, 12-14 |
| A | CN 104470919 A (GLAXOSMITHKLINE PLC) 25 March 2015 (2015-03-25) see claims 1 and 18-22, and description, paragraph [0011] | 2-11 |
| X | AMERICAN CHEMICAL SOCIETY ACS. "STNext Registry Database" *http://next.stn.org,* 05 July 2017 (2017-07-05), following compounds in CAS RN: 2101329-03-5, 2101328-01-0, 2101324-36-9 and 2101324-17-6 | 1 |

☐ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **10 May 2019** | **27 May 2019** |

| Name and mailing address of the ISA/CN | Authorized officer |
|---|---|
| **State Intellectual Property Office of the P. R. China**<br>**No. 6, Xitucheng Road, Jimenqiao Haidian District, Beijing 100088**<br>**China** | |
| Facsimile No. **(86-10)62019451** | Telephone No. |

Form PCT/ISA/210 (second sheet) (January 2015)

**INTERNATIONAL SEARCH REPORT**
**Information on patent family members**

International application No.
**PCT/CN2019/076129**

| Patent document cited in search report | Publication date (day/month/year) | Patent family member(s) | Publication date (day/month/year) |
|---|---|---|---|
| WO 2017100594 A1 | 15 June 2017 | JP 2019505568 A | 28 February 2019 |
| | | KR 20180098574 A | 04 September 2018 |
| | | AU 2016366398 A1 | 19 July 2018 |
| | | US 2017166565 A1 | 15 June 2017 |
| | | SG 11201804668U A | 28 June 2018 |
| | | TW 201726674 A | 01 August 2017 |
| | | MX 2018006632 A | 09 November 2018 |
| | | IL 259794 D0 | 30 August 2018 |
| | | AR 107032 A1 | 14 March 2018 |
| | | EA 201891251 A1 | 28 December 2018 |
| | | US 9963448 B2 | 08 May 2018 |
| | | EP 3386590 A1 | 17 October 2018 |
| | | CN 109069869 A | 21 December 2018 |
| | | BR 112018011709 A2 | 04 December 2018 |
| | | UY 37018 A | 30 June 2017 |
| | | CA 3007639 A1 | 15 June 2017 |
| CN 104470919 A | 25 March 2015 | RS 55684 B1 | 31 July 2017 |
| | | EP 2877467 A1 | 03 June 2015 |
| | | BR 112015001545 A2 | 04 July 2017 |
| | | HR P20161530 T1 | 10 February 2017 |
| | | SM T201700052 B | 08 March 2017 |
| | | LT 2877467 T | 10 January 2017 |
| | | KR 20150038438 A | 08 April 2015 |
| | | AU 2012386257 A1 | 12 March 2015 |
| | | PL 2877467 T3 | 31 August 2017 |
| | | HU E033294 T2 | 28 November 2017 |
| | | RU 2611010 C2 | 17 February 2017 |
| | | SI EP2877467 T1 | 28 February 2017 |
| | | US 9833449 B2 | 05 December 2017 |
| | | US 9127003 B2 | 08 September 2015 |
| | | IN 2935KON2014 A | 08 May 2015 |
| | | US 2015175600 A1 | 25 June 2015 |
| | | PT 2877467 T | 02 January 2017 |
| | | DK 2877467 T3 | 13 February 2017 |
| | | EP 2877467 B1 | 02 November 2016 |
| | | US 2017119750 A1 | 04 May 2017 |
| | | US 10039755 B2 | 07 August 2018 |
| | | US 9518054 B2 | 13 December 2016 |
| | | JP 2015522628 A | 06 August 2015 |
| | | CA 2879341 A1 | 30 January 2014 |
| | | ES 2609126 T3 | 18 April 2017 |
| | | CN 104470919 B | 06 July 2016 |
| | | US 2016009716 A1 | 14 January 2016 |
| | | KR 101916443 B1 | 08 November 2018 |
| | | WO 2014015905 A1 | 30 January 2014 |
| | | CY 1118524 T1 | 12 July 2017 |
| | | AU 2012386257 B2 | 08 September 2016 |
| | | SI 2877467 T1 | 28 February 2017 |
| | | RU 2014152456 A | 20 September 2016 |
| | | US 2018161316 A1 | 14 June 2018 |

Form PCT/ISA/210 (patent family annex) (January 2015)

**INTERNATIONAL SEARCH REPORT**
**Information on patent family members**

International application No.
**PCT/CN2019/076129**

| Patent document cited in search report | Publication date (day/month/year) | Patent family member(s) | Publication date (day/month/year) |
|---|---|---|---|
| | | JP 6063567 B2 | 18 January 2017 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2014015905 A1